(19) Europäisches Patentamt / European Patent Office / Office européen des brevets

(11) **EP 4 679 986 A1**

(12) **EUROPEAN PATENT APPLICATION**
published in accordance with Art. 153(4) EPC

(43) Date of publication:
**14.01.2026 Bulletin 2026/03**

(21) Application number: **24767112.6**

(22) Date of filing: **04.03.2024**

(51) International Patent Classification (IPC):
***H10K 30/60*** *(2023.01)* ***C07D 495/14*** *(2006.01)*
***H10K 30/30*** *(2023.01)* ***H10K 39/32*** *(2026.01)*
***H10K 85/40*** *(2023.01)* ***H10K 85/60*** *(2023.01)*

(52) Cooperative Patent Classification (CPC):
**C07D 495/14; C07D 495/22; C07D 513/14; H10K 30/30; H10K 30/60; H10K 39/32; H10K 85/211; H10K 85/40; H10K 85/60; H10K 85/615; H10K 85/621; H10K 85/654; H10K 85/657; H10K 85/6572; H10K 85/6574;**
(Cont.)

(86) International application number:
**PCT/JP2024/008059**

(87) International publication number:
**WO 2024/185744 (12.09.2024 Gazette 2024/37)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **06.03.2023 JP 2023033825**

(71) Applicant: **FUJIFILM Corporation**
**Tokyo 106-8620 (JP)**

(72) Inventors:
- **KANEKO, Kazuhei**
  **Ashigarakami-gun, Kanagawa 258-8577 (JP)**
- **SUGIURA, Hiroki**
  **Ashigarakami-gun, Kanagawa 258-8577 (JP)**
- **YONEKUTA, Yasunori**
  **Ashigarakami-gun, Kanagawa 258-8577 (JP)**

(74) Representative: **HGF**
**HGF Limited**
**4th Floor, 1 City Square**
**Leeds LS1 2AL (GB)**

(54) **PHOTOELECTRIC CONVERSION ELEMENT, IMAGING ELEMENT, OPTICAL SENSOR, PRODUCTION METHOD FOR IMAGING ELEMENT, COMPOUND**

(57) An object of the present invention is to provide a photoelectric conversion element having excellent quantum efficiency in a case of receiving red and green light, and to provide an imaging element, an optical sensor, and a compound, which are related to the photoelectric conversion element. The photoelectric conversion element of the present invention includes, in the following order, a conductive film, a photoelectric conversion film, and a transparent conductive film, in which the photoelectric conversion film contains a compound represented by Formula (1).

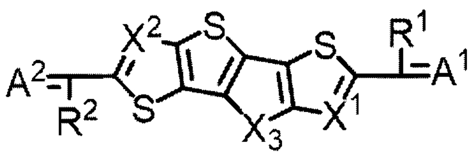

(1)

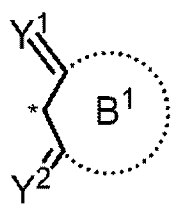

(A-1)

EP 4 679 986 A1

(52) Cooperative Patent Classification (CPC): (Cont.)
**H10K 85/6576;** H10K 30/353

## Description

### BACKGROUND OF THE INVENTION

1. Field of the Invention

The present invention relates to a photoelectric conversion element, an imaging element, an optical sensor, a method for manufacturing an imaging element, and a compound.

2. Description of the Related Art

In recent years, development of an element (for example, an imaging element) having a photoelectric conversion film has progressed.
For example, US2022/0135587A discloses, as a material used for a photoactive organic electronic component, a compound having a specific structure.

### SUMMARY OF THE INVENTION

In recent years, further improvements are also required for various characteristics required for a photoelectric conversion element used in an imaging element and an optical sensor, along with demands for improving performance of the imaging element, the optical sensor, and the like.
For example, it is required that quantum efficiency in a case where the photoelectric conversion element receives red and green light is high. The above-described red and green light refers to light in a wavelength range of 500 to 700 nm.
As a result of studying the photoelectric conversion element containing the compound, disclosed in US2022/0135587A, the present inventors have found that there is room for improvement in quantum efficiency in a case of receiving the red and green light.
An object of the present invention is to provide a photoelectric conversion element having excellent quantum efficiency in a case of receiving red and green light.
Another object of the present invention is to provide an imaging element, an optical sensor, a method for manufacturing an imaging element, and a compound, which are related to the above-described photoelectric conversion element.
As a result of conducting an extensive investigation to achieve the objects, the present inventors have found that the objects can be achieved by the following constitution.

[1] A photoelectric conversion element comprising, in the following order:

a conductive film;
a photoelectric conversion film; and
a transparent conductive film,
in which the photoelectric conversion film contains a compound represented by Formula (1) described later.

[2] The photoelectric conversion element according to [1],

in which $X^3$ represents $-C(R^{c1}R^{c2})-$,
where $R^{c1}$ and $R^{c2}$ may be bonded to each other to form a ring which may have a substituent, and in a case where the ring which may have a substituent has a plurality of the substituents, the substituents may be bonded to each other to form a ring which may have a substituent.

[3] The photoelectric conversion element according to [1] or [2],
in which the group represented by Formula (A-1) described later is a group represented by Formula (A-2) described later.
[4] The photoelectric conversion element according to [3],
in which, in Formula (A-2) described later, $R^{N2}$ and $R^{N3}$ are groups different from each other.
[5] The photoelectric conversion element according to any one of [1] to [4],
in which $X^1$ and $X^2$ each independently represent $-CR^{a1}=$.
[6] The photoelectric conversion element according to any one of [1] to [5],
in which any one of requirements 1 to 4 described later is satisfied.
[7] The photoelectric conversion element according to [6],

in which any one of requirements 5 to 7 described later or the requirement 4 described later is satisfied.
[8] The photoelectric conversion element according to any one of [1] to [7],

in which at least one of $X^1$ or $X^2$ represents -$CR^{a2}$=,
$R^{a2}$'s each independently represent an aliphatic hydrocarbon group which may have a substituent, an aromatic ring group which may have a substituent, a halogen atom, an alkoxy group which may have a substituent, an acyl group which may have a substituent, an aliphatic heterocyclic group which may have a substituent, a cyano group, a nitro group, an amino group, or -Si($R^{Si1}R^{Si2}R^{Si3}$), and $R^{Si1}$, $R^{Si2}$, and $R^{Si3}$ each independently represent an aliphatic hydrocarbon group which may have a substituent or an aromatic ring group which may have a substituent.

[9] The photoelectric conversion element according to any one of [1] to [8],

in which the photoelectric conversion film further contains an n-type organic semiconductor, and
the photoelectric conversion film has a bulk hetero structure formed in a state in which the compound represented by Formula (1) and the n-type organic semiconductor are mixed with each other.

[10] The photoelectric conversion element according to [9],
in which the n-type organic semiconductor includes fullerenes selected from the group consisting of a fullerene and derivatives of the fullerene.
[11] The photoelectric conversion element according to any one of [1] to [10],
in which the photoelectric conversion film further contains a p-type organic semiconductor.
[12] The photoelectric conversion element according to any one of [1] to [11],
in which the photoelectric conversion film further contains a coloring agent.
[13] The photoelectric conversion element according to any one of [1] to [12], further comprising:
one or more interlayers between the conductive film and the transparent conductive film, in addition to the photoelectric conversion film.
[14] An imaging element comprising:
the photoelectric conversion element according to any one of [1] to [13].
[15] An optical sensor comprising:
the photoelectric conversion element according to any one of [1] to [13].
[16] A method for manufacturing an imaging element, comprising:
a step of manufacturing the photoelectric conversion element according to any one of [1] to [13].
[17] A compound represented by Formula (1) described later.
[18] The compound according to [17],

in which $X^3$ represents -C($R^{c1}R^{c2}$)-,
where $R^{c1}$ and $R^{c2}$ may be bonded to each other to form a ring which may have a substituent, and in a case where the ring which may have a substituent has a plurality of the substituents, the substituents may be bonded to each other to form a ring which may have a substituent.

[19] The compound according to [17] or [18],
in which the group represented by Formula (A-1) described later is a group represented by Formula (A-2) described later.
[20] The compound according to [19],
in which, in Formula (A-2) described later, $R^{N2}$ and $R^{N3}$ are groups different from each other.
[21] The compound according to any one of [17] to [20],
in which $X^1$ and $X^2$ each independently represent -$CR^{a1}$=.
[22] The compound according to any one of [17] to [21],
in which any one of requirements 1 to 4 described later is satisfied.
[23] The compound according to [22],
in which any one of requirements 5 to 7 described later or the requirement 4 is satisfied.
[24] The compound according to any one of [17] to [23],

in which at least one of $X^1$ or $X^2$ represents -$CR^{a2}$=,
$R^{a2}$'s each independently represent an aliphatic hydrocarbon group which may have a substituent, an aromatic ring group which may have a substituent, a halogen atom, an alkoxy group which may have a substituent, an acyl group which may have a substituent, an aliphatic heterocyclic group which may have a substituent, a cyano group,

a nitro group, an amino group, or -Si($R^{Si1}R^{Si2}R^{Si3}$), and $R^{Si1}$, $R^{Si2}$, and $R^{Si3}$ each independently represent an aliphatic hydrocarbon group which may have a substituent or an aromatic ring group which may have a substituent.

According to the present invention, it is possible to provide a photoelectric conversion element having excellent quantum efficiency in a case of receiving red and green light.

In addition, according to the present invention, it is also possible to provide an imaging element, an optical sensor, a method for manufacturing an imaging element, and a compound, which are related to the above-described photoelectric conversion element.

## BRIEF DESCRIPTION OF THE DRAWINGS

Fig. 1 is a schematic cross-sectional view showing an example of a configuration of a photoelectric conversion element.
Fig. 2 is a schematic cross-sectional view showing an example of a configuration of a photoelectric conversion element.

## DESCRIPTION OF THE PREFERRED EMBODIMENTS

Hereinafter, the present invention will be described in detail.

The description of the configuration requirements described below is made on the basis of representative embodiments of the present invention, but it should not be construed that the present invention is limited to those embodiments.

In the present specification, numerical ranges represented by "to" include numerical values before and after "to" as lower limit values and upper limit values.

In the present specification, a hydrogen atom may be any of a light hydrogen atom (normal hydrogen atom) or a heavy hydrogen atom (for example, a deuterium atom or the like).

In the present specification, in a case of a plurality of substituents, linking groups, and the like (hereinafter, also referred to as "substituent and the like") represented by a specific reference numeral, or in a case of simultaneously defining a plurality of the substituent and the like, it means that each of the substituent and the like may be the same as or different from each other. This also applies to a case of specifying the number of substituents and the like.

In the present specification, the "substituent" includes a group exemplified as the following substituent W, unless otherwise specified.

(Substituent W)

The substituent W in the present specification will be described below.

Examples of the substituent W include a halogen atom (for example, a fluorine atom, a chlorine atom, a bromine atom, an iodine atom, and the like), an alkyl group (including a cycloalkyl group, a bicycloalkyl group, and a tricycloalkyl group), an alkenyl group (including a cycloalkenyl group and a bicycloalkenyl group), an alkynyl group, an aryl group, a heterocyclic group, a cyano group, a nitro group, an alkoxy group, an aryloxy group, a silyl group, a silyloxy group, a heterocyclic oxy group, an acyloxy group, a carbamoyloxy group, an alkoxycarbonyloxy group, an aryloxycarbonyloxy group, a secondary or tertiary amino group (including an anilino group), an alkylthio group, an arylthio group, a heterocyclic thio group, an alkyl or an arylsulfinyl group, an alkyl or an arylsulfonyl group, an acyl group, an aryloxycarbonyl group, an alkoxycarbonyl group, an aryl or a heterocyclic azo group, an imide group, a phosphino group, a phosphinyl group, a phosphinyloxy group, a phosphinylamino group, a phosphono group, a carboxy group, a phosphoric acid group, a sulfonic acid group, a hydroxy group, a thiol group, an acylamino group, a carbamoyl group, a ureido group, a boronic acid group, and a primary amino group. In addition, each of the above-described groups may further have a substituent (for example, one or more groups of each of the above-described groups), as possible. For example, an alkyl group which may have a substituent is also included as the form of the substituent W.

In addition, in a case where the substituent W has a carbon atom, the number of carbon atoms in the substituent W is, for example, 1 to 20.

The number of atoms other than a hydrogen atom in the substituent W is, for example, 1 to 30.

It is also preferable that a specific compound described later does not contain, as a substituent, a carboxy group, a salt of a carboxy group, a salt of a phosphoric acid group, a sulfonic acid group, a salt of a sulfonic acid group, a hydroxy group, a thiol group, an acylamino group, a carbamoyl group, a ureido group, or a boronic acid group ($-B(OH)_2$) and/or a

primary amino group.

In the present specification, examples of the halogen atom include a fluorine atom, a chlorine atom, a bromine atom, and an iodine atom.

In the present specification, the aliphatic hydrocarbon group may be linear, branched, or cyclic.

Examples of the above-described aliphatic hydrocarbon group include an alkyl group, an alkenyl group, and an alkynyl group.

In addition, in the present specification, unless otherwise specified, the number of carbon atoms in the alkyl group is preferably 1 to 20, more preferably 1 to 10, and still more preferably 1 to 6.

The alkyl group may be linear, branched, or cyclic.

Examples of the alkyl group include a methyl group, an ethyl group, an n-propyl group, an i-propyl group, an n-butyl group, a t-butyl group, an n-hexyl group, and a cyclopentyl group.

In addition, the alkyl group may be any of a cycloalkyl group, a bicycloalkyl group, or a tricycloalkyl group, and may have a ring structure thereof as a partial structure.

In the alkyl group which may have a substituent, examples of the substituent which may be included in the alkyl group include the groups exemplified as the substituent W. Among these, an aryl group (preferably having 6 to 18 carbon atoms and more preferably having 6 carbon atoms), a heteroaryl group (preferably having 5 to 18 carbon atoms and more preferably having 5 or 6 carbon atoms), or a halogen atom (preferably a fluorine atom or a chlorine atom) is preferable.

In the present specification, unless otherwise specified, an alkyl group moiety in the alkoxy group is preferably the above-described alkyl group. An alkyl group moiety in the alkylthio group is preferably the above-described alkyl group.

In the alkoxy group which may have a substituent, examples of the substituent which may be included in the alkoxy group include the same examples as the substituent in the alkyl group which may have a substituent.

In the alkylthio group which may have a substituent, examples of the substituent which may be included in the alkylthio group include the same examples as the substituent in the alkyl group which may have a substituent.

In the present specification, the alkenyl group may be any of linear, branched, or cyclic, unless otherwise specified. The number of carbon atoms in the above-described alkenyl group is preferably 2 to 20. In the alkenyl group which may have a substituent, examples of the substituent which may be included in the alkenyl group include the same examples as the substituent in the alkyl group which may have a substituent.

In the present specification, the alkynyl group may be any of linear, branched, or cyclic, unless otherwise specified. The number of carbon atoms in the above-described alkynyl group is preferably 2 to 20. In the alkynyl group which may have a substituent, examples of the substituent which may be included in the alkynyl group include the same examples as the substituent in the alkyl group which may have a substituent.

In the present specification, unless otherwise specified, an aromatic ring or an aromatic ring constituting the aromatic ring group may be any of a monocyclic ring or a polycyclic ring (for example, 2 to 6 rings). The monocyclic aromatic ring is an aromatic ring having only one aromatic ring structure as a ring structure. The polycyclic (for example, 2 to 6 rings or the like) aromatic ring is an aromatic ring formed by a plurality of (for example, 2 to 6 or the like) fused aromatic ring structures, as a ring structure.

The number of ring members in the above-described aromatic ring is preferably 5 to 15.

The above-described aromatic ring may be any of an aromatic hydrocarbon ring or an aromatic heterocyclic ring.

In a case where the above-described aromatic ring is an aromatic heterocyclic ring, the number of heteroatoms included as ring member atoms is, for example, 1 to 10. Examples of the above-described heteroatom include a nitrogen atom, a sulfur atom, an oxygen atom, a selenium atom, a tellurium atom, a phosphorus atom, a silicon atom, and a boron atom.

Examples of the above-described aromatic hydrocarbon ring include a benzene ring, a naphthalene ring, an anthracene ring, a phenanthrene ring, and a fluorene ring.

Examples of the above-described aromatic heterocyclic ring include a pyridine ring, a pyrimidine ring, a pyridazine ring, a pyrazine ring, a triazine ring (for example, 1,2,3-triazine ring, 1,2,4-triazine ring, 1,3,5-triazine ring, and the like), a tetrazine ring (for example, 1,2,4,5-tetrazine ring and the like), a quinoxaline ring, a pyrrole ring, a furan ring, a thiophene ring, an imidazole ring, an oxazole ring, a thiazole ring, a benzopyrrole ring, a benzofuran ring, a benzothiophene ring, a benzimidazole ring, a benzoxazole ring, a benzothiazole ring, a naphthopyrrole ring, a naphthofuran ring, a naphthothiophene ring, a naphthimidazole ring, a naphthoxazole ring, a pyrroloimidazole ring (for example, a 5H-pyrrolo[1,2-a]imidazole ring and the like), an imidazooxazole ring (for example, an imidazo[2,1-b]oxazole ring and the like), a thienothiazole ring (for example, a thieno[2,3-d]thiazole ring and the like), a benzothiadiazole ring, a benzodithiophene ring (for example, benzo[1,2-b:4,5-b']dithiophene ring and the like), a thienothiophene ring (for example, thieno[3,2-b]thiophene ring and the like), a thiazolothiazole ring (for example, thiazolo[5,4-d]thiazole ring and the like), a naphthodithiophene ring (for example, a naphtho[2,3-b:6,7-b']dithiophene ring, a naphtho[2,1-b:6,5-b']dithiophene ring, a naphtho[1,2-b:5,6-b']dithiophene ring, a 1,8-dithiadicyclopenta[b,g]naphthalene ring, and the like), a benzothienobenzothiophene ring, a dithieno[3,2-b:2',3'-d]thiophene ring, and a 3,4,7,8-tetrathiadicyclopenta[a,e]pentalene ring.

In the aromatic ring which may have a substituent, examples of the type of the substituent which may be included

in the aromatic ring include the groups exemplified as the substituent W. In a case where the above-described aromatic ring has a substituent, the number of substituents may be 1 or more (for example, 1 to 4, or the like).

In the present specification, examples of the "aromatic ring group" include a group obtained by removing one or more (for example, 1 to 5 or the like) hydrogen atoms from the above-described aromatic ring.

In the present specification, examples of the "aryl group" include a group obtained by removing one hydrogen atom from a ring corresponding to the aromatic hydrocarbon ring among the above-described aromatic rings.

In the present specification, examples of the "heteroaryl group" include a group obtained by removing one hydrogen atom from a ring corresponding to the aromatic heterocyclic ring among the above-described aromatic rings.

In the present specification, examples of the "arylene group" include a group obtained by removing two hydrogen atoms from a ring corresponding to the aromatic hydrocarbon ring among the above-described aromatic rings.

In the present specification, examples of the "heteroarylene group" include a group obtained by removing two hydrogen atoms from a ring corresponding to the aromatic heterocyclic ring among the above-described aromatic rings.

In the aromatic ring group which may have a substituent, the aryl group which may have a substituent, the heteroaryl group which may have a substituent, the arylene group which may have a substituent, and the heteroarylene group which may have a substituent, examples of the type of the substituent which may be included in these groups include the groups exemplified as the substituent W. In a case where these groups each of which may have a substituent have a substituent, the number of substituents may be 1 or more (for example, 1 to 4, or the like).

In the present specification, a non-aromatic ring represents a ring structure which does not correspond to an aromatic ring. Examples of the non-aromatic ring include an aliphatic hydrocarbon ring and an aliphatic heterocyclic ring.

Examples of the above-described aliphatic hydrocarbon ring include a cycloalkane, a cycloalkene, and a cycloalkyne.

Examples of the above-described aliphatic heterocyclic ring include a pyrrolidine ring, an oxolane ring, a thiolane ring, a piperidine ring, a tetrahydropyran ring, a thiacine ring, a piperazine ring, a morpholine ring, a quinocyclidine ring, an azetidine ring, an oxetane ring, an aziridine ring, a dioxane ring, and a γ-butyrolactone ring.

In the present specification, examples of the "aliphatic hydrocarbon ring group" include a group obtained by removing one or more (for example, 1 to 5 or the like) hydrogen atoms from a ring corresponding to the aliphatic hydrocarbon ring.

In the present specification, examples of the "aliphatic heterocyclic group" include a group obtained by removing one or more (for example, 1 to 5 or the like) hydrogen atoms from a ring corresponding to the aliphatic heterocyclic ring.

In the present specification, unless otherwise specified, a ring (for example, the aromatic ring and the non-aromatic ring) may be a monocyclic ring or a polycyclic ring (for example, 2 to 6 rings). The monocyclic ring is a structure having only one ring as a ring structure; and the polycyclic ring is a structure in which a plurality of (for example, 2 to 6) rings are fused as a ring structure.

In the present specification, in a case where a plurality of identical symbols indicating a type or the number of groups are present in the formula, which indicates a chemical structure, contents of these plurality of identical symbols indicating a type or the number of groups are independent of each other, and the contents of the identical symbols may be the same or different from each other, unless otherwise specified.

In the present specification, in a case where a plurality of identical groups (for example, alkyl groups and the like) are present in the formula, which indicates a chemical structure, specific contents between these plurality of identical groups are independent of each other, and the specific contents between the plurality of identical groups may be the same or different from each other, unless otherwise specified.

A bonding direction of a divalent group (for example, -CO-O-) described in the present specification is not limited unless otherwise specified. For example, in a case where Y in a compound represented by a formula "X-Y-Z" is -CO-O-, the compound may be any of "X-O-CO-Z" or "X-CO-O-Z".

In the present specification, regarding a compound which may have a geometric isomer (cis-trans isomer), a general formula or a structural formula representing the compound may be described only in the form of either a cis isomer or a trans isomer for convenience. Even in such a case, unless otherwise specified, the form of the compound is not limited to either the cis isomer or the trans isomer, and the compound may be the cis isomer, the trans isomer, or a mixture thereof.

In the present specification, in regard to a compound having an asymmetric atom, a general formula or a structural formula representing the compound may be described without distinguishing stereoisomers for convenience. Even in such a case, unless otherwise specified, a form of the above-described compound is not limited to any one form, and may be any one form or a mixture thereof. For example, the compound having an asymmetric carbon atom may be either an S form or an R form, or may be a mixture thereof, unless otherwise specified.

In the present specification, unless otherwise specified, * in the formula represents a bonding position.

[Photoelectric conversion element]

The photoelectric conversion element according to the embodiment of the present invention includes a photoelectric conversion film and a transparent conductive film in this order, in which the photoelectric conversion film contains a compound represented by Formula (1) described later (hereinafter, referred to as "specific compound").

The reason why the photoelectric conversion element having the above-described configuration can achieve the object of the present invention is not necessarily clear, but the present inventors speculate as follows.

The mechanism by which the effect is obtained is not limited by the following supposition. In other words, even in a case where an effect is obtained by a mechanism other than the following, it is included in the scope of the present invention.

The specific compound is a so-called ADA-type compound having a donor site (D) in a center of a molecule and an acceptor site (A) at both ends. In a case where the donor site of the specific compound has only the specific fused-ring structure, excessive aggregation of the specific compounds due to $\pi$-$\pi$ stacking is suppressed, charge separation in the photoelectric conversion film proceeds efficiently, and as a result, quantum efficiency of the photoelectric conversion element is excellent. In addition, in a case where a conjugated plane of the acceptor site is small, absorbance tends to decrease, but by setting at least one acceptor site of the specific compound to a specific monocyclic structure, surprisingly, the internal quantum efficiency in a case of receiving red and green light is improved, and as a result, the quantum efficiency of the photoelectric conversion element in a case of receiving red and green light is excellent. As described above, since the donor site and the acceptor site of the specific compound each have the specific structure, the quantum efficiency of the photoelectric conversion element in a case of receiving red and green light is excellent.

Hereinafter, the fact that the quantum efficiency in a case where the photoelectric conversion element receives red and green light is more excellent is also referred to as "effect of the present invention is more excellent".

Fig. 1 shows a schematic cross-sectional view of one embodiment of the photoelectric conversion element according to the embodiment of the present invention.

A photoelectric conversion element 10a shown in Fig. 1 has a configuration in which a conductive film (hereinafter, also referred to as "lower electrode") 11 functioning as a lower electrode, an electron blocking film 16A, a photoelectric conversion film 12 containing the specific compound, and a transparent conductive film (hereinafter, also referred to as "upper electrode") 15 functioning as an upper electrode are laminated in this order.

Fig. 2 shows a configuration example of another photoelectric conversion element. A photoelectric conversion element 10b shown in Fig. 2 has a configuration in which the electron blocking film 16A, the photoelectric conversion film 12, a hole blocking film 16B, and the upper electrode 15 are laminated on the lower electrode 11 in this order. The lamination order of the electron blocking film 16A, the photoelectric conversion film 12, and the hole blocking film 16B in Figs. 1 and 2 may be appropriately changed according to the application and the characteristics.

In the photoelectric conversion element 10a (or 10b), it is preferable that light is incident to the photoelectric conversion film 12 through the upper electrode 15.

In a case where the photoelectric conversion element 10a (or 10b) is used, a voltage can be applied. In this case, it is preferable that the lower electrode 11 and the upper electrode 15 form a pair of electrodes, and a voltage of $1 \times 10^{-5}$ to $1 \times 10^{7}$ V/cm is applied between the pair of electrodes. From the viewpoint of performance and power consumption, the applied voltage is more preferably $1 \times 10^{-4}$ to $1 \times 10^{7}$ V/cm and still more preferably $1 \times 10^{-3}$ to $5 \times 10^{6}$ V/cm.

Regarding the voltage application method, in Figs. 1 and 2, it is preferable that the voltage is applied such that the electron blocking film 16A side is a cathode and the photoelectric conversion film 12 side is an anode. In a case where the photoelectric conversion element 10a (or 10b) is used as an optical sensor, or also in a case of being incorporated in an imaging element, the voltage can be applied by the same method.

As described in detail below, the photoelectric conversion element 10a (or 10b) can be suitably applied to applications of an imaging element.

Hereinafter, the form of each layer constituting the photoelectric conversion element according to the embodiment of the present invention will be described in detail.

[Photoelectric conversion film]

The photoelectric conversion element includes a photoelectric conversion film.

<Specific compound>

The photoelectric conversion film contains the specific compound which is a compound represented by Formula (1).

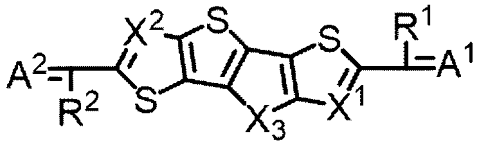

(1)

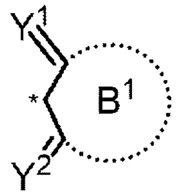

(A-1)

In Formula (1), $R^1$ and $R^2$ each independently represent a hydrogen atom or a substituent,

$X^1$ and $X^2$ each independently $-CR^{a1}=$ or a nitrogen atom, $R^{a1}$ represents a hydrogen atom or a substituent, $X^3$ represents $-C(R^{c1}R^{c2})-$, $-O-C(R^{c3}R^{c4})-$, $-NR^{N1}-C(R^{c5}R^{c6})-$, or $-NR^{N11}-$ $R^{c1}$, $R^{c2}$, $R^{c3}$, $R^{c4}$, $R^{c5}$, $R^{c6}$, $R^{N1}$, and $R^{N11}$ each independently represent a hydrogen atom or a substituent, $R^{c1}$ and $R^{c2}$, $R^{c3}$ and $R^{c4}$, or $R^{c5}$ and $R^{c6}$ may be bonded to each other to form a ring which may have a substituent, in a case where the ring which may have a substituent has a plurality of the substituents, the substituents may be bonded to each other to form a ring which may have a substituent, and

$A^1$ and $A^2$ each independently represent Formula (A-1).

In Formula (A-1), $B^1$ represents a monocyclic ring or a polycyclic ring, which contains at least three or more carbon atoms and may have a substituent, provided that, in a case where $B^1$ represents the monocyclic ring and the monocyclic ring has two or more of the substituents, the number of aromatic ring groups among the substituents in the monocyclic ring is 1 or less,

$Y^1$ and $Y^2$ each independently represent an oxygen atom, a sulfur atom, $=NR^{Y1}$, or $=CR^{Y2}R^{Y3}$, $R^{Y1}$ represents a hydrogen atom, an aliphatic hydrocarbon group which may have a substituent, or an aromatic ring group which may have a substituent, $R^{Y2}$ and $R^{Y3}$ each independently represent a cyano group, $-COOR^{Y4}$, $-C(=O)R^{Y5}$, $-S(=O)R^{Y6}$, or $-SO_2R^{Y7}$, $R^{Y4}$, $R^{Y5}$, $R^{Y6}$, and $R^{Y7}$ each independently represent an aliphatic hydrocarbon group which may have a substituent, an aromatic ring group which may have a substituent, or an aliphatic heterocyclic group which may have a substituent, and

* represents a bonding position,

provided that at least one of $A^1$ or $A^2$ represents the group represented by Formula (A-1), in which $B^1$ is represented by the monocyclic ring.

In Formula (1), $R^1$ and $R^2$ each independently represent a hydrogen atom or a substituent.

Examples of the above-described substituent represented by $R^1$ and $R^2$ include the substituents exemplified as the substituent W.

From the viewpoint that the effect of the present invention is more excellent, $R^1$ and $R^2$ are preferably hydrogen atoms.

In Formula (1), $X^1$ and $X^2$ each independently $-CR^{a1}=$ or a nitrogen atom.

$R^{a1}$ represents a hydrogen atom or a substituent.

Examples of the above-described substituent represented by $R^{a1}$ include the substituents exemplified as the substituent W, and a substituent represented by $R^{a2}$, which will be described later, is preferable.

In a case where a plurality of $R^{a1}$'s are present, groups represented by the plurality of $R^{a1}$'s may be the same or different from each other.

From the viewpoint that the effect of the present invention is more excellent, it is preferable that at least one of $X^1$ or $X^2$ represents $-CR^{a1}=$, and it is more preferable that $X^1$ and $X^2$ represent $-CR^{a1}=$. In other words, the specific compound is more preferably a compound represented by Formula (1-1). The compound represented by Formula (1-1) is a compound in which $X^1$ and $X^2$ in Formula (1) are represented by $-CR^{a1}=$.

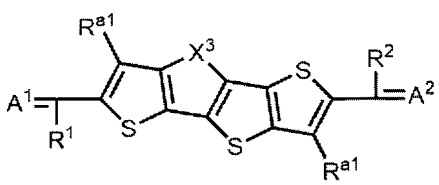

(1-1)

In Formula (1-1), $A^1$, $A^2$, $R^1$, $R^2$, and $X^3$ have the same definitions as the respective groups in Formula (1).

In Formula (1-1), the definition and suitable aspect of $R^{a1}$ are as described above, and a plurality of $R^{a1}$'s may be the same or different from each other.

From the viewpoint of more excellent response speed and manufacturing suitability, it is also preferable that at least one of $X^1$ or $X^2$ represents $-CR^{a2}=$.

$R^{a2}$ represents an aliphatic hydrocarbon group which may have a substituent, an aromatic ring group which may have a substituent, a halogen atom, an alkoxy group which may have a substituent, an acyl group which may have a substituent, an aliphatic heterocyclic group which may have a substituent, a cyano group, a nitro group, an amino group, or $-Si(R^{Si1}R^{Si2}R^{Si3})$.

In a case where a plurality of $R^{a2}$'s are present, groups represented by the plurality of $R^{a2}$'s may be the same or different from each other.

As the group represented by $R^{a2}$, an aliphatic hydrocarbon group which may have a substituent, an aromatic ring group which may have a substituent, a halogen atom, an alkoxy group which may have a substituent, an acyl group which may have a substituent, an aliphatic hetero group which may have a substituent, or $-Si(R^{Si1}R^{Si2}R^{Si3})$ is preferable; and an aliphatic hydrocarbon group which may have a substituent, an aromatic ring group which may have a substituent, a halogen atom, an alkoxy group which may have a substituent, an acyl group which may have a substituent, or $-Si(R^{Si1}R^{Si2}R^{Si3})$ is more preferable.

Examples of the above-described aliphatic hydrocarbon group represented by $R^{a2}$ include a linear aliphatic hydrocarbon group, a branched aliphatic hydrocarbon group, and a cyclic aliphatic hydrocarbon group.

The number of carbon atoms in the above-described linear aliphatic hydrocarbon group is, for example, 1 to 20, and from the viewpoint of more excellent manufacturing suitability, it is preferably 1 to 6, more preferably 1 to 3, and still more preferably 1 or 2. Specific examples thereof include a methyl group, an ethyl group, an n-propyl group, an n-butyl group, an n-pentyl group, an n-hexyl group, an n-heptyl group, an n-octyl group, an n-nonyl group, an n-decyl group, an n-undecyl group, and an n-dodecyl group; and a methyl group, an ethyl group, or an n-propyl group is preferable.

The number of carbon atoms in the above-described branched aliphatic hydrocarbon group is, for example, 3 to 20, and from the viewpoint of more excellent manufacturing suitability, it is preferably 3 to 10, more preferably 3 to 6, and still more preferably 3 or 4. Specific examples thereof include an isopropyl group, a sec-butyl group, an iso-butyl group, a tert-butyl group, a neopentyl group, a 2-ethylhexyl group, a 3,7-dimethyloctyl group, a 2-butyloctyl group, a 2-hexyloctyl group, a 2-hexyldodecyl group, and a 2-octyldodecyl group; and an isopropyl group or a tert-butyl group is preferable.

The above-described cyclic aliphatic hydrocarbon group may be a monocyclic ring or a polycyclic ring.

The number of carbon atoms in the above-described cyclic aliphatic hydrocarbon group is preferably 3 to 10, more preferably 3 to 8, and still more preferably 3 to 6. Specific examples thereof include a cyclopropyl group, a cyclobutyl group, a cyclopentyl group, a cyclohexyl group, a cycloheptyl group, a cyclooctyl group, a cyclodecanyl group, a dicyclobutanyl group, a bicyclo[1.1.1]pentyl group, and a bicyclo[2.2.2]pentyl group; and a cyclopropyl group, a cyclobutyl group, a cyclopentyl group, or a cyclohexyl group is preferable, and a cyclopropyl group is more preferable.

Examples of the substituent which may be included in the above-described aliphatic hydrocarbon group include the substituents exemplified as the substituent W; and a substituent selected from a substituent group S described later is preferable.

The above-described aromatic ring group which may have a substituent, represented by $R^{a2}$, may be a monocyclic ring or a polycyclic ring, preferably a monocyclic ring. In addition, the above-described aromatic ring group may be an aromatic hydrocarbon ring group or an aromatic heterocyclic group, and an aromatic hydrocarbon ring group is preferable.

The number of ring members in the above-described aromatic ring group is preferably 5 to 20, more preferably 5 to 12, and still more preferably 5 to 8.

The number of carbon atoms in the above-described aromatic ring group which may have a substituent is preferably 30 or less, more preferably 20 or less, and still more preferably 10 or less. The lower limit thereof is preferably 1 or more, more preferably 3 or more, and still more preferably 4 or more.

Examples of a heteroatom which is included in the above-described aromatic heterocyclic group include a sulfur atom, an oxygen atom, a nitrogen atom, a selenium atom, a tellurium atom, a phosphorus atom, a silicon atom, and a boron atom; and a sulfur atom, an oxygen atom, or a nitrogen atom is preferable.

Examples of the above-described aromatic ring group include an aromatic hydrocarbon ring group such as a phenyl group, a naphthyl group, an anthryl group, a pyrenyl group, a phenanthrenyl group, and a fluorenyl group, and an aromatic heterocyclic group such as a pyridine ring group, a pyrimidine ring group, a pyridazine ring group, a pyrazine ring group, a triazine ring group, a tetrazine ring group, a quinoxaline ring group, a pyrrole ring group, a furan ring group, a thiophene ring group, an imidazole ring group, an oxazole ring group, a thiazole ring group, a benzopyrrole ring group, a benzofuran ring group, a benzothiophene ring group, a benzimidazole ring group, a benzoxazole ring group, and a benzothiazole ring group; and a phenyl group, a thiophene ring group, a furan ring group, or a pyridine ring group is preferable, a phenyl group or a thiophene ring group is more preferable, and a phenyl group is still more preferable.

Examples of the substituent which may be included in the above-described aromatic ring group include the substituents exemplified as the substituent W; and a substituent selected from the substituent group S described later is preferable.

In a case where the above-described aromatic ring group has a substituent, the number of substituents is not particularly limited, but is preferably 1 to 6 and more preferably 1 to 3.

Examples of the above-described halogen atom represented by $R^{a2}$ include a fluorine atom, a chlorine atom, a bromine atom, and an iodine atom; and a fluorine atom or a chlorine atom is preferable.

An alkyl group which may have a substituent in the above-described alkoxy group which may have a substituent, represented by $R^{a2}$, may be linear, branched, or cyclic.

Specific examples of the above-described alkyl group include a linear alkyl group such as a methyl group, an ethyl group, an n-propyl group, an n-butyl group, an n-pentyl group, an n-hexyl group, an n-heptyl group, an n-octyl group, an n-nonyl group, an n-decyl group, an n-undecyl group, and an n-dodecyl group, a branched alkyl group such as an isopropyl group, a sec-butyl group, an iso-butyl group, a tert-butyl group, a neopentyl group, a 1-ethylpentyl group, a 2,6-dimethylheptyl group, a 1-butylheptyl group, a 1-hexylheptyl group, a 1-hexylundecyl group, and a 1-octylundecyl group, and a cyclic alkyl group such as a cyclopropyl group, a cyclobutyl group, a cyclopentyl group, a cyclohexyl group, a cycloheptyl group, a cyclooctyl group, a cyclodecanyl group, a dicyclobutanyl group, a bicyclo[1.1.1]pentyl group, and a bicyclo[2.2.2]pentyl group; and a methyl group, an ethyl group, an n-propyl group, an isopropyl group, or a tert-butyl group is preferable, and a methyl group or an ethyl group is more preferable.

The number of carbon atoms in the above-described alkoxy group is, for example, 1 to 20, and from the viewpoint of more excellent manufacturing suitability, it is preferably 1 to 6, more preferably 1 to 3, and still more preferably 1 or 2.

Specific examples of the above-described alkoxy group include a methoxy group, an ethoxy group, a propoxy group, an isopropoxy group, an n-butoxy group, a tert-butoxy group, and a cyclopropoxy group; and a methoxy group or an ethoxy group is preferable.

Examples of the substituent which may be included in the above-described alkoxy group include the substituents exemplified as the substituent W; and a substituent selected from the substituent group S described later is preferable.

A hydrocarbon group in the above-described acyl group which may have a substituent, represented by $R^{a2}$, may be an aliphatic hydrocarbon group or an aromatic hydrocarbon ring group, and is preferably an aliphatic hydrocarbon group.

The aliphatic hydrocarbon group included in the above-described acyl group may be linear, branched, or cyclic.

The number of carbon atoms in the above-described aliphatic hydrocarbon group of the above-described acyl group is, for example, 1 to 20, and from the viewpoint of more excellent manufacturing suitability, it is preferably 1 to 6, more preferably 1 to 3, and still more preferably 1 or 2. Specific examples thereof include a linear aliphatic hydrocarbon group such as a methyl group, an ethyl group, an n-propyl group, an n-butyl group, an n-pentyl group, an n-hexyl group, an n-heptyl group, an n-octyl group, an n-nonyl group, an n-decyl group, an n-undecyl group, and an n-dodecyl group, a branched aliphatic hydrocarbon group such as an isopropyl group, a sec-butyl group, an iso-butyl group, a tert-butyl group, a neopentyl group, a 1-ethylpentyl group, a 2,6-dimethylheptyl group, a 1-butylheptyl group, a 1-hexylheptyl group, a 1-hexylundecyl group, and a 1-octylundecyl group, and a cyclic aliphatic hydrocarbon group such as a cyclopropyl group, a cyclobutyl group, a cyclopentyl group, a cyclohexyl group, a cycloheptyl group, a cyclooctyl group, a cyclodecanyl group, a dicyclobutanyl group, a bicyclo[1.1.1]pentyl group, and a bicyclo[2.2.2]pentyl group; and a methyl group, an ethyl group, an n-propyl group, an isopropyl group, or a tert-butyl group is preferable, and a methyl group or an ethyl group is more preferable.

The number of carbon atoms in the aromatic hydrocarbon ring group included in the above-described acyl group is preferably 6 to 20, more preferably 6 to 10, and still more preferably 6. Specific examples thereof include a phenyl group, a naphthyl group, an anthryl group, a pyrenyl group, a phenanthrenyl group, and a fluorenyl group; and a phenyl group is preferable.

The number of carbon atoms in the above-described acyl group is, for example, 2 to 21, and from the viewpoint of more excellent manufacturing suitability, it is preferably 2 to 11, more preferably 2 to 5, and still more preferably 2 or 3.

Examples of the above-described acyl group include an acetyl group, a propionyl group, a butyryl group, an isobutyryl group, a pivaloyl group, a hexanoyl group, and a benzoyl group; and an acetyl group or a propionyl group is preferable.

Examples of the substituent which may be included in the above-described acyl group include the substituents exemplified as the substituent W; and a substituent selected from the substituent group S described later is preferable.

The above-described aliphatic heterocyclic group which may have a substituent, represented by $R^{a2}$, may be a monocyclic ring or a polycyclic ring.

The number of ring members in the above-described aliphatic heterocyclic group is preferably 6 to 20, more preferably 6 to 12, and still more preferably 6 to 8.

The number of carbon atoms in the above-described aliphatic heterocyclic group which may have a substituent is preferably 1 to 30, more preferably 3 to 20, and still more preferably 4 to 10.

Examples of a heteroatom which is included in the above-described aliphatic heterocyclic group include a sulfur atom, an oxygen atom, a nitrogen atom, a selenium atom, a tellurium atom, a phosphorus atom, a silicon atom, and a boron atom; and a sulfur atom, an oxygen atom, or a nitrogen atom is preferable.

Examples of the above-described aliphatic heterocyclic group include a pyrrolidine ring group, an oxolane ring group, a thiolane ring group, a piperidine ring group, a tetrahydrofuran ring group, a tetrahydropyran ring group, a thiane ring group, a piperazine ring group, a morpholine ring group, a quinocyclidine ring group, a pyrrolidine ring group, an azetidine ring group, an oxetane ring group, an aziridine ring group, a dioxane ring group, a pentamethylenesulfide ring group, and a γ-butyrolactone ring group; and a piperidine ring group is preferable.

Examples of the substituent which may be included in the above-described aliphatic heterocyclic group include the substituents exemplified as the substituent W; and a substituent selected from a substituent group S described later is preferable.

In a case where the above-described aliphatic heterocyclic group has a substituent, the number of substituents is not particularly limited, but is preferably 1 to 4 and more preferably 1 to 3.

The above-described amino group represented by $R^{a2}$ may be any of a primary amino group, a secondary amino group, or a tertiary amino group, preferably a tertiary amino group.

In the above-described secondary amino group and the above-described tertiary amino group, a hydrocarbon group to be substituted with an amino group is preferably an alkyl group or an aryl group, and more preferably an alkyl group having 1 to 3 carbon atoms or a phenyl group.

In $-Si(R^{Si1}R^{Si2}R^{Si3})$, $R^{Si1}$, $R^{Si2}$, and $R^{Si3}$ each independently represent an aliphatic hydrocarbon group which may have a substituent or an aromatic ring group which may have a substituent.

Definition and suitable aspects of the aliphatic hydrocarbon group which may have a substituent and the aromatic ring group which may have a substituent, represented by $R^{Si1}$, $R^{Si2}$, and $R^{Si3}$, are the same as those of the aliphatic hydrocarbon group which may have a substituent and the aromatic ring group which may have a substituent, represented by $R^{a2}$, respectively.

Among these, $R^{Si1}$, $R^{Si2}$, and $R^{Si3}$ are preferably an aliphatic hydrocarbon group having 1 to 8 carbon atoms or an aromatic ring group which may have a substituent selected from the substituent group S described later, and more preferably an aliphatic hydrocarbon group having 1 to 4 carbon atoms or a phenyl group.

Specific examples of $-Si(R^{Si1}R^{Si2}R^{Si3})$ include a trimethylsilyl group, a triethylsilyl group, a dimethylisopropylsilyl group, a diethylisopropylsilyl group, a cyclohexyldimethylsilyl group, a dimethylphenylsilyl group, and a tert-butyldimethylsilyl group; and a trimethylsilyl group or a triethylsilyl group is preferable, and a trimethylsilyl group is more preferable.

The substituent group S described above is shown below.

Substituent group S: a linear aliphatic hydrocarbon group having 1 to 4 carbon atoms, a branched aliphatic hydrocarbon group having 3 to 5 carbon atoms, a cyclic aliphatic hydrocarbon having 3 to 8 carbon atoms, an alkoxy group having 1 to 5 carbon atoms, a halogen atom, and $-Si(R^{Si1}R^{Si2}R^{Si3})$

The linear aliphatic hydrocarbon having 1 to 4 carbon atoms, the branched aliphatic hydrocarbon group having 3 to 5 carbon atoms, and the cyclic aliphatic hydrocarbon having 3 to 8 carbon atoms in the above-described substituent group S (hereinafter, also referred to as "aliphatic hydrocarbon group in the substituent group S") may have a halogen atom.

Examples of the linear aliphatic hydrocarbon group having 1 to 4 carbon atoms in the above-described substituent group S include a methyl group, an ethyl group, an n-propyl group, and an n-butyl group; and a methyl group, an ethyl group, or an n-propyl group is preferable, and a methyl group is more preferable.

Examples of the branched-chain aliphatic hydrocarbon group having 3 to 5 carbon atoms in the above-described substituent group S include an isopropyl group, a sec-butyl group, an iso-butyl group, a tert-butyl group, and a neopentyl group; and an isopropyl group or a tert-butyl group is preferable, and an isopropyl group is more preferable.

Examples of the cyclic aliphatic hydrocarbon having 3 to 8 carbon atoms in the above-described substituent group S include a cyclopropyl group, a cyclobutyl group, a cyclopentyl group, a cyclohexyl group, a cycloheptyl group, and a cyclooctyl group; and a cyclopropyl group, a cyclobutyl group, a cyclopentyl group, or a cyclohexyl group is preferable, and a cyclopropyl group is more preferable.

Examples of the halogen atom which may be included in the above-described aliphatic hydrocarbon group in the substituent group S include a fluorine atom, a chlorine atom, a bromine atom, and an iodine atom; and a fluorine atom or a chlorine atom is preferable.

Examples of the alkoxy group having 1 to 5 carbon atoms in the above-described substituent group S include a methoxy group, an ethoxy group, an isopropoxy group, and a cyclopropoxy group; and a methoxy group is preferable.

Examples of the halogen atom in the above-described substituent group S include a fluorine atom, a chlorine atom, a bromine atom, and an iodine atom; and a fluorine atom or a chlorine atom is preferable.

The definition and suitable aspect of $-Si(R^{Si1}R^{Si2}R^{Si3})$ in the above-described substituent group S are the same as those of $-Si(R^{Si1}R^{Si2}R^{Si3})$ represented by $R^{a2}$.

Among these, the group selected from the above-described substituent group S is preferably a linear aliphatic hydrocarbon group having 1 or 2 carbon atoms, a branched aliphatic hydrocarbon group having 3 or 4 carbon atoms, a cyclic aliphatic hydrocarbon group having 3 to 6 carbon atoms, or a halogen atom.

In Formula (1), $X^3$ represents $-C(R^{c1}R^{c2})-$, $-O-C(R^{c3}R^{c4})-$, $-NR^{N1}-C(R^{c5}R^{c6})-$, or $-NR^{N11}-$.

A bonding direction of the group represented by $X^3$ is not particularly limited. Specifically, in a case where $X^3$ in Formula (1) is $-C(R^{c1}R^{c2})-$, the specific compound is a compound represented by Formula (1-X1); in a case where $X^3$ is $-O-C(R^{c3}R^{c4})-$, the specific compound is a compound represented by Formula (1-X2) or a compound represented by Formula (1-X3); in a case where $X^3$ is $-NR^{N1}-C(R^{c5}R^{c6})-$, the specific compound is a compound represented by Formula (1-X4) or a compound represented by Formula (1-X5); and in a case where $X^3$ is $-NR^{N11}-$, the specific compound is a compound represented by Formula (1-X6).

Among these, $X^3$ is preferably $-C(R^{c1}R^{c2})-$. That is, the specific compound is preferably a compound represented by Formula (1-X1).

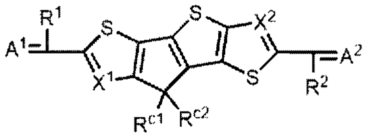
(1-X1)

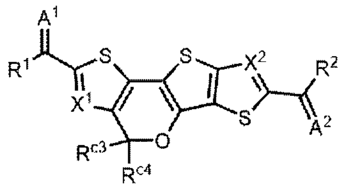
(1-X2)

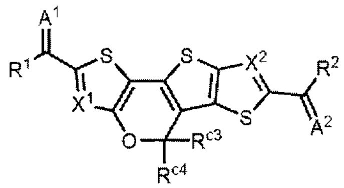
(1-X3)

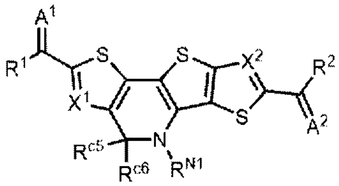
(1-X4)

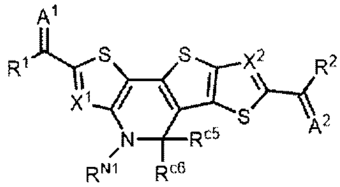
(1-X5)

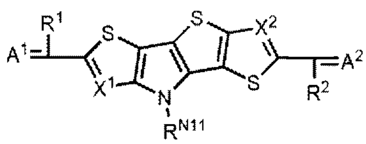
(1-X6)

$A^1$, $A^2$, $R^1$, $R^2$, $X^1$, $X^2$, $R^{c1}$, $R^{c2}$, $R^{c3}$, $R^{c4}$, $R^{c5}$, $R^{c6}$, $R^{N1}$, and $R^{N11}$ in Formula (1-X1) to Formula (1-X6) have the same definitions as the respective groups in Formula (1).

$R^{c1}$, $R^{c2}$, $R^{c3}$, $R^{c4}$, $R^{c5}$, $R^{c6}$, $R^{N1}$, and $R^{N11}$ each independently represent a hydrogen atom or a substituent. $R^{c1}$ and $R^{c2}$, $R^{c3}$ and $R^{c4}$, or $R^{c5}$ and $R^{c6}$ may be bonded to each other to form a ring which may have a substituent. The substituents of the above-described ring which may have a substituent may be bonded to each other to form a ring which may have a substituent.

Examples of the substituent represented by $R^{c1}$, $R^{c2}$, $R^{c3}$, $R^{c4}$, $R^{c5}$, $R^{c6}$, $R^{N1}$ and $R^{N11}$ include the substituents exemplified as the substituent W.

From the viewpoint of more excellent manufacturing suitability, a molecular weight of the substituent represented by $R^{c1}$, $R^{c2}$, $R^{c3}$, $R^{c4}$, $R^{c5}$, $R^{c6}$, $R^{N1}$ and $R^{N11}$ is preferably 150 or less, more preferably 90 or less, and still more preferably 50 or less. The lower limit thereof is not particularly limited, but is preferably 15 or more.

From the viewpoint of more excellent manufacturing suitability, the number of carbon atoms in the substituent represented by $R^{c1}$, $R^{c2}$, $R^{c3}$ , $R^{c4}$, $R^{c5}$, $R^{c6}$, $R^{N1}$, and $R^{N11}$ is preferably 7 or less, more preferably 5 or less, and still more preferably 3 or less. The lower limit thereof is not particularly limited, but is preferably 1 or more.

Specifically, the above-described substituent is preferably an aliphatic hydrocarbon group which may have a substituent, an aromatic ring group which may have a substituent, or an aliphatic heterocyclic group which may have a substituent.

Examples of the above-described aliphatic hydrocarbon group represented by $R^{c1}$, $R^{c2}$, $R^{c3}$, $R^{c4}$, $R^{c5}$, $R^{c6}$, $R^{N1}$ and $R^{N11}$ include a linear aliphatic hydrocarbon group, a branched aliphatic hydrocarbon group, and a cyclic aliphatic hydrocarbon group.

The number of carbon atoms in the above-described linear aliphatic hydrocarbon group is, for example, 1 to 20, preferably 1 to 6, more preferably 1 to 3, and still more preferably 1 or 2. Specific examples thereof include a methyl group, an ethyl group, an n-propyl group, an n-butyl group, an n-pentyl group, an n-hexyl group, an n-heptyl group, an n-octyl group, an n-nonyl group, an n-decyl group, an n-undecyl group, and an n-dodecyl group; and a methyl group, an ethyl group, or an n-propyl group is preferable, and a methyl group or an ethyl group is more preferable.

The number of carbon atoms in the above-described branched aliphatic hydrocarbon group is, for example, 3 to 20, preferably 3 to 10, more preferably 3 to 6, and still more preferably 3 or 4. Specific examples thereof include an isopropyl group, a sec-butyl group, an iso-butyl group, a tert-butyl group, a neopentyl group, a 2-ethylhexyl group, a 3,7-dimethyloctyl group, a 2-butyloctyl group, a 2-hexyloctyl group, a 2-hexyldodecyl group, and a 2-octyldodecyl group;

and an isopropyl group, a sec-butyl group, an iso-butyl group, or a tert-butyl group is preferable.

The above-described cyclic aliphatic hydrocarbon group may be monocyclic or polycyclic.

The number of carbon atoms in the above-described cyclic aliphatic hydrocarbon group is preferably 3 to 10, more preferably 3 to 8, and still more preferably 3 to 6. Specific examples thereof include a cyclopropyl group, a cyclobutyl group, a cyclopentyl group, a cyclohexyl group, a cycloheptyl group, a cyclooctyl group, a cyclodecanyl group, a dicyclobutanyl group, a bicyclo[1.1.1]pentyl group, and a bicyclo[2.2.2]pentyl group; and a cyclopropyl group, a cyclobutyl group, a cyclopentyl group, or a cyclohexyl group is preferable, and a cyclopropyl group is more preferable.

Examples of the substituent which may be included in the above-described aliphatic hydrocarbon group include the substituents exemplified as the substituent W; and a substituent selected from the above-described substituent group S is preferable.

The above-described aromatic ring group which may have a substituent, represented by $R^{c1}$, $R^{c2}$, $R^{c3}$, $R^{c4}$, $R^{c5}$, $R^{c6}$, $R^{N1}$ and $R^{N11}$, may be a monocyclic ring or a polycyclic ring.

The above-described aromatic ring group may be an aromatic hydrocarbon ring group or an aromatic heterocyclic group, and an aromatic hydrocarbon ring group is preferable.

The number of ring members in the above-described aromatic ring group is preferably 5 to 20, more preferably 5 to 12, and still more preferably 5 to 8.

The number of carbon atoms in the above-described aromatic ring group which may have a substituent is preferably 30 or less, more preferably 20 or less, and still more preferably 10 or less. The lower limit thereof is preferably 1 or more, more preferably 3 or more, and still more preferably 4 or more.

Examples of a heteroatom which is included in the above-described aromatic heterocyclic group include a sulfur atom, an oxygen atom, a nitrogen atom, a selenium atom, a tellurium atom, a phosphorus atom, a silicon atom, and a boron atom; and a sulfur atom, an oxygen atom, or a nitrogen atom is preferable.

Examples of the above-described aromatic ring group include an aromatic hydrocarbon ring group such as a phenyl group, a naphthyl group, an anthryl group, a pyrenyl group, a phenanthrenyl group, and a fluorenyl group, and an aromatic heterocyclic group such as a pyridine ring group, a pyrimidine ring group, a pyridazine ring group, a pyrazine ring group, a triazine ring group, a tetrazine ring group, a quinoxaline ring group, a pyrrole ring group, a furan ring group, a thiophene ring group, an imidazole ring group, an oxazole ring group, a thiazole ring group, a benzopyrrole ring group, a benzofuran ring group, a benzothiophene ring group, a benzimidazole ring group, a benzoxazole ring group, and a benzothiazole ring group; and a phenyl group, a thiophene ring group, a furan ring group, or a pyridine ring group is preferable, a phenyl group or a thiophene ring group is more preferable, and a phenyl group is still more preferable.

Examples of the substituent which may be included in the above-described aliphatic hydrocarbon group include the substituents exemplified as the substituent W; and a substituent selected from the above-described substituent group S is preferable.

In a case where the above-described aromatic ring group has a substituent, the number of substituents is not particularly limited, but is preferably 1 to 6 and more preferably 1 to 3.

The above-described aliphatic heterocyclic group which may have a substituent, represented by $R^{c1}$, $R^{c2}$, $R^{c3}$, $R^{c4}$, $R^{c5}$, $R^{c6}$, $R^{N1}$, and $R^{N11}$, may be a monocyclic ring or a polycyclic ring.

The number of ring members in the above-described aliphatic heterocyclic group is preferably 6 to 20, more preferably 6 to 12, and still more preferably 6 to 8.

The number of carbon atoms in the above-described aliphatic heterocyclic group is preferably 1 to 30, more preferably 3 to 20, and still more preferably 4 to 10.

Examples of a heteroatom which is included in the above-described aliphatic heterocyclic group include a sulfur atom, an oxygen atom, a nitrogen atom, a selenium atom, a tellurium atom, a phosphorus atom, a silicon atom, and a boron atom; and a sulfur atom, an oxygen atom, or a nitrogen atom is preferable.

Examples of the above-described aliphatic heterocyclic group include a pyrrolidine ring group, an oxolane ring group, a thiolane ring group, a piperidine ring group, a tetrahydrofuran ring group, a tetrahydropyran ring group, a thiane ring group, a piperazine ring group, a morpholine ring group, a quinocyclidine ring group, a pyrrolidine ring group, an azetidine ring group, an oxetane ring group, an aziridine ring group, a dioxane ring group, a pentamethylenesulfide ring group, and a $\gamma$-butyrolactone ring group; and a piperidine ring group is preferable.

Examples of the substituent which may be included in the above-described aliphatic heterocyclic group include the substituents exemplified as the substituent W; and a substituent selected from the above-described substituent group S is preferable.

In a case where the above-described aliphatic heterocyclic group has a substituent, the number of substituents is not particularly limited, but is preferably 1 to 4 and more preferably 1 to 3.

Among these, $R^{c1}$, $R^{c2}$, $R^{c3}$, $R^{c4}$, $R^{c5}$, $R^{c6}$, $R^{N1}$, and $R^{N11}$ are preferably a substituent, more preferably an aliphatic hydrocarbon group which may have a substituent or an aromatic ring group which may have a substituent, and still more preferably an aliphatic hydrocarbon group which may have a substituent.

As described above, $R^{c1}$ and $R^{c2}$, $R^{c3}$ and $R^{c4}$, or $R^{c5}$ and $R^{c6}$ may be bonded to each other to form a ring which

may have a substituent.

The above-described ring is preferably a monocyclic ring.

The ring may be an aliphatic hydrocarbon ring, an aliphatic heterocyclic ring, or an aromatic ring, but is preferably an aliphatic hydrocarbon ring.

The number of ring members in the above-described ring is preferably 3 to 10, more preferably 3 to 8, and still more preferably 4 to 7.

Specific examples of the above-described aliphatic hydrocarbon ring include a cyclopropane ring, a cyclobutane ring, a cyclopentane ring, a cyclopentadiene ring, a cyclohexane ring, a cycloheptane ring, a cyclooctane ring, and a cyclodecane ring; and a cyclobutane ring, a cyclopentane ring, a cyclohexane ring, or a cycloheptane ring is preferable, and a cyclohexane ring is more preferable.

Examples of a heteroatom which is included in the above-described aliphatic heterocyclic ring include a sulfur atom, an oxygen atom, a nitrogen atom, a selenium atom, a tellurium atom, a phosphorus atom, a silicon atom, and a boron atom; and a sulfur atom, an oxygen atom, or a nitrogen atom is preferable.

Examples of the above-described aliphatic heterocyclic ring include a pyrrolidine ring, an oxolane ring, a thiolane ring, a piperidine ring, a tetrahydrofuran ring, a tetrahydropyran ring, a thiacine ring, a piperazine ring, a morpholine ring, a quinocyclidine ring, a pyrrolidine ring, an azetidine ring, an oxetane ring, an aziridine ring, a dioxane ring, a pentamethylene sulfide ring, and a $\gamma$-butyrolactone ring; and a piperidine ring is preferable.

The above-described ring may have a substituent, and from the viewpoint that the effect of the present invention is more excellent, it is preferable that the above-described ring has a substituent.

Examples of the substituent include the substituents exemplified as the substituent W; and an alkyl group, an aromatic ring group, or a halogen atom is preferable, and an alkyl group or a halogen atom is more preferable.

The above-described alkyl group may be linear, branched, or cyclic, and is preferably linear or branched. The number of carbon atoms of the above-described alkyl group is preferably 1 to 10, more preferably 1 to 3, and still more preferably 1.

In a case where the above-described ring has a substituent, the number of substituents is preferably 1 to 6 and more preferably 1 to 3.

In addition, in a case where the above-described ring has a plurality of substituents, the substituents may be bonded to each other to further form a ring. The ring formed by bonding the substituents to each other may be an aliphatic hydrocarbon ring, an aliphatic heterocyclic ring, or an aromatic ring, but is preferably an aliphatic hydrocarbon ring.

Examples of such an aspect include an aspect in which alkyl groups substituted with a cyclohexane ring are bonded to each other to form a norbornene ring as a whole. Examples of the structure formed by bonding the substituents of the above-described ring to each other to further form a ring include a norbornene ring structure, a bicyclo[3.1.1]heptane ring structure, a bicyclo[1.1.1]pentane, a bicyclo[2.2.2]pentane ring structure, and a fluorene ring structure.

The number of carbon atoms in the structure formed by bonding the substituents of the above-described ring to each other to further form a ring is preferably 4 to 12 and more preferably 4 to 8.

From the viewpoint that electric field strength dependence of the quantum efficiency is smaller, $R^{N11}$ is preferably a group represented by Formula (C-1) or a group represented by Formula (C-2).

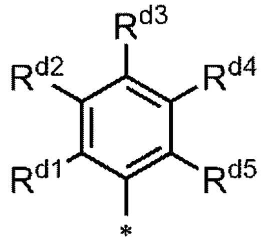

(C-1)

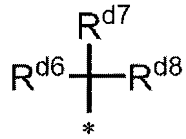

(C-2)

In Formula (C-1), $R^{d1}$ to $R^{d5}$ each independently represent a hydrogen atom or a substituent. Here, at least one of the following requirement C1 or the following requirement C2 is satisfied.

Requirement C1: $R^{d1}$ and $R^{d5}$ are groups different from each other,
Requirement C2: $R^{d2}$ and $R^{d4}$ are groups different from each other.

Examples of the substituent represented by $R^{d1}$ to $R^{d5}$ include the substituents exemplified as the substituent W; and a substituent selected from the above-described substituent group S is preferable.

In Formula (C-2), $R^{d6}$ to $R^{d8}$ each independently represent a hydrogen atom or a substituent. Here, $R^{d6}$ to $R^{d8}$ are

groups different from each other.

Examples of the substituent represented by $R^{d6}$ to $R^{d8}$ include the substituents exemplified as the substituent W; and an aliphatic hydrocarbon group which may have a substituent, an aromatic ring group which may have a substituent, or an aliphatic heterocyclic group which may have a substituent is preferable, an aliphatic hydrocarbon group which may have a substituent or an aromatic ring group which may have a substituent is more preferable, and an aliphatic hydrocarbon group which may have a substituent is still more preferable.

Definition and suitable aspects of the aliphatic hydrocarbon group which may have a substituent, the aromatic ring group which may have a substituent, and the aliphatic heterocyclic group which may have a substituent, represented by $R^{d6}$ to $R^{d8}$, are the same as those of the respective groups described above as the substituent represented by $R^{c1}$, $R^{c2}$, $R^{c3}$, $R^{c4}$, $R^{c5}$, $R^{c6}$, $R^{N1}$, and $R^{N11}$.

From the viewpoint that the effect of the present invention is more excellent, it is preferable that the specific compound satisfies any one of the following requirements 1 to 4 for $X^3$.

In addition, from the viewpoint of more excellent manufacturing suitability, it is also preferable that the specific compound satisfies any one of the requirements 1 to 3.

Requirement 1: $X^3$ represents $-C(R^{c1}R^{c2})-$, and $R^{c1}$ and $R^{c2}$ are groups different from each other, or $R^{c1}$ and $R^{c2}$ are bonded to form a monocyclic ring having a substituent.

Requirement 2: $X^3$ represents $-O-C(R^{c3}R^{c4})-$, and $R^{c3}$ and $R^{c4}$ are groups different from each other, or $R^{c3}$ and $R^{c4}$ are bonded to form a monocyclic ring having a substituent.

Requirement 3: $X^3$ represents $-NR^{N1}-C(R^{c5}R^{c6})-$, and $R^{c5}$ and $R^{c6}$ are groups different from each other, or $R^{c5}$ and $R^{c6}$ are bonded to form a monocyclic ring having a substituent.

Requirement 4: $X^3$ represents $-NR^{N11}-$, and $R^{N11}$ is the above-described group represented by Formula (C-1) or the above-described group represented by Formula (C-2).

In a case where the monocyclic ring having a substituent in the requirements 1 to 3 has a plurality of substituents, the substituents may be bonded to each other to form a ring which may have a substituent.

From the viewpoint that the effect of the present invention is more excellent, it is more preferable that the specific compound satisfies any one of the following requirements 5 to 7 or the above-described requirement 4 for $X^3$.

From the viewpoint of more excellent manufacturing suitability, it is also preferable that the specific compound satisfies any one of the requirements 5 to 7.

Requirement 5: $X^3$ represents $-C(R^{c1}R^{c2})-$, and $R^{c1}$ and $R^{c2}$ are groups different from each other.

Requirement 6: $X^3$ represents $-O-C(R^{c3}R^{c4})-$, and $R^{c3}$ and $R^{c4}$ are groups different from each other.

Requirement 7: $X^3$ represents $-NR^{N1}-C(R^{c5}R^{c6})-$, and $R^{c5}$ and $R^{c6}$ are groups different from each other.

In Formula (1), $A^1$ and $A^2$ each independently represent Formula (A-1). However, at least one of $A^1$ or $A^2$ represents the group represented by Formula (A-1), in which $B^1$ is a monocyclic ring. From the viewpoint that the effect of the present invention is more excellent, it is preferable that both $A^1$ and $A^2$ represent the group represented by Formula (A-1), in which $B^1$ is a monocyclic ring.

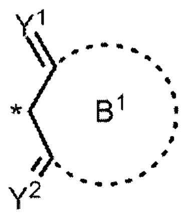

(A-1)

In Formula (A-1), $B^1$ represents a monocyclic ring or a polycyclic ring, which contains at least three or more carbon atoms and may have a substituent, and is preferably a monocyclic ring which may have a substituent. At least three carbon atoms contained in $B^1$ are the three carbon atoms specified in Formula (A-1).

In a case where $B^1$ is a monocyclic ring, the number of ring members is preferably 3 to 20, more preferably 3 to 10, and still more preferably 4 to 8. Among these, the above-described ring is preferably a 5-membered ring or a 6-membered ring, and more preferably a 6-membered ring.

In addition, the number of carbon atoms in the above-described monocyclic ring is preferably 3 to 20, more preferably 3 to 10, and still more preferably 3 to 6.

In a case where $B^1$ is a polycyclic ring, the number of ring members is preferably 3 to 30, more preferably 3 to 20, and still more preferably 3 to 10. In addition, the above-described ring is preferably a fused ring including at least one of a 5-membered ring or a 6-membered ring.

The number of ring members and the number of carbon atoms in the monocyclic ring and the polycyclic ring

described above are the numbers including the three carbon atoms specified in the formula.

The above-described monocyclic ring and polycyclic ring may be an aromatic ring or a non-aromatic ring.

The above-described monocyclic ring and polycyclic ring may have a heteroatom. Examples of the heteroatom include a nitrogen atom, a sulfur atom, an oxygen atom, a selenium atom, a tellurium atom, a phosphorus atom, a silicon atom, and a boron atom; and a sulfur atom, a nitrogen atom, or an oxygen atom is preferable.

The number of heteroatoms in the above-described monocyclic ring is preferably 0 to 10, more preferably 0 to 5, and still more preferably 1 to 4.

Among the carbon atoms constituting the monocyclic ring and the polycyclic ring represented by $B^1$, a carbon atom at the bonding position to which * is attached in Formula (A-1) and a carbon atom other than a carbon atom bonded to $Y^1$ or $Y^2$ (that is, a carbon atom other than the carbon atoms specified in the formula) may be substituted with a carbonyl carbon (>C=O) or a thiocarbonyl carbon (>C=S).

As described above, the monocyclic ring and the polycyclic ring may have a substituent. However, in a case where the above-described monocyclic ring has two or more substituents, the number of aromatic ring groups among the substituents included in the monocyclic ring is 1 or less, preferably 0.

Examples of the substituent which may be included in the above-described monocyclic ring or polycyclic ring include the groups exemplified as the substituent W; and a halogen atom, an alkyl group, an aromatic ring group, or a silyl group is preferable, and a halogen atom or an alkyl group is more preferable.

The above-described alkyl group may be linear, branched, or cyclic, and is preferably linear.

The number of carbon atoms in the above-described alkyl group is preferably 1 to 10 and more preferably 1 to 3.

The above-described alkyl may further have a substituent. As the substituent which may be included in the above-described alkyl group, a halogen atom, an aromatic ring group, or a silyl group is preferable.

In addition, the above-described aromatic ring group may have a substituent. As the substituent which may be included in the above-described aromatic ring group, a halogen atom, an alkyl group, or a silyl group is preferable.

The "substituent included in the ring" is intended to be a monovalent group other than a hydrogen atom, which is bonded to a ring member atom constituting the ring, and also includes a group bonded to a heteroatom constituting the ring. Examples thereof include a substituent on a carbon atom constituting the ring (for example, R in -CHR- and R in =CR-) and a substituent on a nitrogen atom constituting the ring (for example, R in -NR-).

In addition, the number of aromatic ring groups in the substituent included in the ring is the number of aromatic ring groups as a monovalent group bonded to the ring member atom, and does not include an aromatic ring group as a substituent, which is substituted with a monovalent group bonded to the ring member atom. For example, an aromatic ring group in an alkyl group substituted with an aromatic ring group, such as a phenyl group included in a phenylmethyl group ($Ph-CH_2-$; Ph is a phenyl group), is not included.

$Y^1$ and $Y^2$ each independently represent an oxygen atom, a sulfur atom, $=NR^{Y1}$, or $=CR^{Y2}R^{Y3}$.

$R^{Y1}$ represents a hydrogen atom, an aliphatic hydrocarbon group which may have a substituent, or an aromatic ring group which may have a substituent.

Examples of the substituent which may be included in the group represented by $R^{Y1}$ include the groups exemplified as the substituent W.

The above-described aliphatic hydrocarbon group represented by $R^{Y1}$ may be linear, branched, or cyclic, and preferably has 1 to 3 carbon atoms.

The above-described aromatic ring group represented by $R^{Y1}$ may be an aromatic hydrocarbon ring group or an aromatic heterocyclic group, and a phenyl group is preferable.

$R^{Y2}$ and $R^{Y3}$ each independently represent a cyano group, $-COOR^{Y4}$, $-C(=O)R^{Y5}$, $-S(=O)R^{Y6}$, or $-SO_2R^{Y7}$.

$R^{Y4}$, $R^{Y5}$, $R^{Y6}$, and $R^{Y7}$ each independently represent an aliphatic hydrocarbon group which may have a substituent, an aromatic ring group which may have a substituent, or an aliphatic heterocyclic group which may have a substituent.

Examples of the substituent which may be included in the group represented by $R^{Y4}$, $R^{Y5}$, $R^{Y6}$, and $R^{Y7}$ include the groups exemplified as the substituent W.

The above-described aliphatic hydrocarbon group represented by $R^{Y4}$, $R^{Y5}$, $R^{Y6}$, and $R^{Y7}$ may be linear, branched, or cyclic, and preferably has 1 to 3 carbon atoms.

The above-described aromatic ring group represented by $R^{Y4}$, $R^{Y5}$, $R^{Y6}$, and $R^{Y7}$ may be an aromatic hydrocarbon ring group or an aromatic heterocyclic group, and a phenyl group is preferable.

The number of ring members in the above-described aliphatic heterocyclic group represented by $R^{Y4}$, $R^{Y5}$, $R^{Y6}$, and $R^{Y7}$ is preferably 5 to 20, more preferably 5 to 12, and still more preferably 6 to 8.

Examples of a heteroatom which is included in the above-described aliphatic heterocyclic group represented by $R^{Y4}$, $R^{Y5}$, $R^{Y6}$, and $R^{Y7}$ include a sulfur atom, an oxygen atom, a nitrogen atom, a selenium atom, a tellurium atom, a phosphorus atom, a silicon atom, and a boron atom; and a sulfur atom, an oxygen atom, or a nitrogen atom is preferable.

Examples of an aliphatic heterocyclic ring constituting the above-described aliphatic heterocyclic group include a pyrrolidine ring, an oxolane ring, a thiolane ring, a piperidine ring, a tetrahydrofuran ring, a tetrahydropyran ring, a thiacine

ring, a piperazine ring, a morpholine ring, a quinocyclidine ring, a pyrrolidine ring, an azetidine ring, an oxetane ring, an aziridine ring, a dioxane ring, a pentamethylene sulfide ring, and γ-butyrolactone.

From the viewpoint that the effect of the present invention is more excellent, $Y^1$ and $Y^2$ are each independently preferably an oxygen atom, a sulfur atom, or $=CR^{Y2}R^{Y3}$, more preferably an oxygen atom or a sulfur atom, and still more preferably an oxygen atom.

As the above-described group represented by Formula (A-1) (the monocyclic ring or the polycyclic ring), a ring used as an acidic nucleus (for example, an acidic nucleus of a merocyanine coloring agent) is preferable; and examples thereof include the following nuclei.

(a) 1,3-dicarbonyl nucleus: for example, 1,3-cyclohexanedione, 5,5-dimethyl-1,3-cyclohexanedione, 1,3-dioxane-4,6-dione, and the like

(b) 2,4,6-trioxohexahydropyrimidine nucleus: for example, barbituric acid, 2-thibarbituric acid and derivatives thereof, and the like; examples of the above-described derivatives include a 1-alkyl form such as 1-methyl and 1-ethyl, a 1,3-dialkyl form such as 1,3-dimethyl, 1,3-diethyl, and 1,3-dibutyl, and a 1-alkyl-1-aryl form such as 1-ethyl-3-phenyl.

(c) 3,5-pyrazolidinedione nuclei: for example, 1,2-dimethyl-3,5-pyrazolidinedione and the like

(d) 1,3-dicarbonyl nucleus: for example, 1,3-indandione nucleus, 1,3-cyclohexanedione, 5,5-dimethyl-1,3-cyclohexanedione, 1,3-dioxane-4,6-dione, and the like

The group represented by Formula (A-1) in which $B^1$ is a monocyclic ring is represented by Formula (A-11); and the group represented by Formula (A-1) in which $B^1$ is a polycyclic ring is represented by Formula (A-12). That is, in the compound represented by Formula (1), at least one of $A^1$ or $A^2$ represents the group represented by Formula (A-11), and the other represents the group selected from Formula (A-11) or Formula (A-12). From the viewpoint that the effect of the present invention is more excellent, it is preferable that both $A^1$ and $A^2$ are the group represented by Formula (A-11).

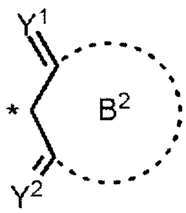

(A-11)

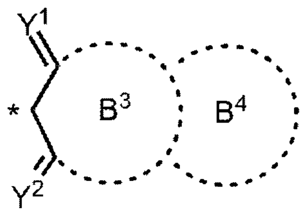

(A-12)

In Formula (A-11) and Formula (A-12), $Y^1$ and $Y^2$ are the same as $Y^1$ and $Y^2$ in Formula (A-1).

In Formula (A-11), $B^2$ represents a monocyclic ring which contains at least three or more carbon atoms and may have a substituent. However, in a case where the monocyclic ring represented by $B^2$ has two or more substituents, the number of aromatic ring groups among the substituents is 1 or less, preferably 0.

**[0242]** * represents a bonding position.

In Formula (A-12), $B^3$ represents a monocyclic ring which contains at least three or more carbon atoms and may have a substituent.

$B^4$ represents an aromatic ring which may have a substituent. $B^4$ is fused with the monocyclic ring represented by $B^3$. $B^4$ may be a monocyclic ring or a polycyclic ring.

**[0245]** * represents a bonding position.

From the viewpoint that the effect of the present invention is more excellent, it is preferable that the group represented by Formula (A-1) is a group represented by Formula (A-2), and it is more preferable that $A^1$ and $A^2$ are each independently a group represented by Formula (A-2).

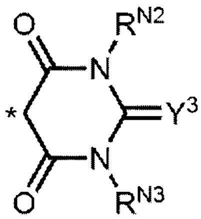

(A-2)

In Formula (A-2), $R^{N2}$ and $R^{N3}$ each independently represent a hydrogen atom or a substituent. However, one of $R^{N2}$ or $R^{N3}$ represents a hydrogen atom or a substituent other than an aromatic ring group.

Examples of the above-described substituent include the groups exemplified as the substituent W; and an aliphatic hydrocarbon group which may have a substituent or an aromatic ring group which may have a substituent is preferable, an alkyl group or an aryl group is more preferable, and an alkyl group is still more preferable.

The above-described alkyl group may be linear, branched, or cyclic, and is preferably linear. The number of carbon atoms in the above-described alkyl group is preferably 1 to 20, more preferably 1 to 6, still more preferably 1 to 3, and particularly preferably 1 or 2.

The above-described aryl group may be a monocyclic ring or a polycyclic ring, and a phenyl group is preferable. The above-described phenyl group may further have a substituent, and examples of the substituent include the groups exemplified as the substituent W.

Examples of the above-described aliphatic hydrocarbon group include an alkyl group, an alkenyl group, and an alkynyl group; and an alkyl group is preferable. The suitable aspect of the alkyl group is as described above.

Examples of the substituent which may be included in the above-described aliphatic hydrocarbon group include the groups exemplified as the substituent W; and a halogen atom, an aryl group, or a silyl group is preferable, and a halogen atom or a silyl group is more preferable.

In addition, it is also preferable that one of $R^{N2}$ or $R^{N3}$ represents a hydrogen atom or an aliphatic hydrocarbon group which may have a substituent, and the other of $R^{N2}$ or $R^{N3}$ represents a hydrogen atom or a substituent.

From the viewpoint that symmetry of the group represented by Formula (A-2) is reduced, aggregation of the specific compound is further suppressed, and the electric field strength dependence of the quantum efficiency is more excellent, it is preferable that $R^{N2}$ and $R^{N3}$ are groups different from each other.

$Y^3$ represents an oxygen atom or a sulfur atom, and is preferably an oxygen atom.

In Formula (1), in a case where $A^1$ and $A^2$ are the group represented by Formula (A-1), the specific compound is represented by Formula (1-A1); and in a case where $A^1$ and $A^2$ are the group represented by Formula (A-2), the specific compound is represented by Formula (1-A2).

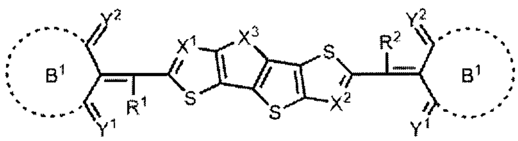

(1-A1)

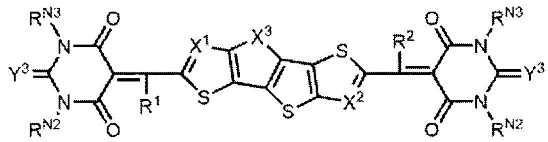

(1-A2)

The specific compound is preferably the compound represented by Formula (1-A2).

In Formula (1-A1) and Formula (1-A2), groups represented by the same reference numerals, which are present in a plural number, may be the same or different from each other.

Specific examples of the specific compound are shown below, but the present invention is not limited thereto. In the formulae, a group represented by "TMS" represents a trimethylsilyl group.

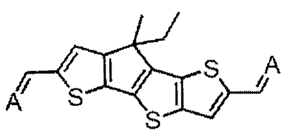

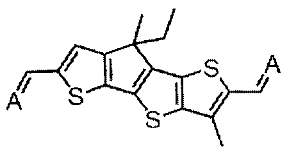

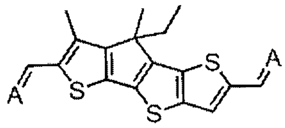

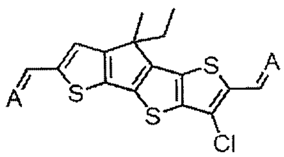

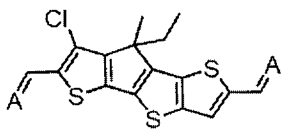

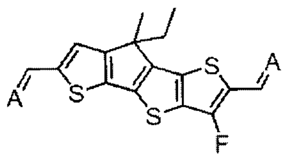

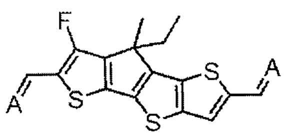

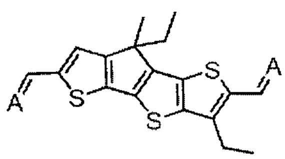

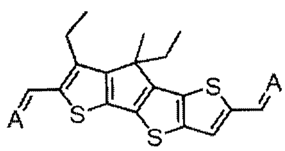

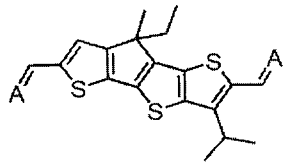 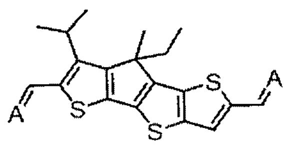 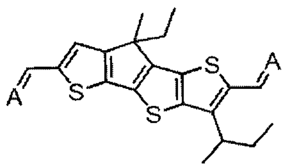

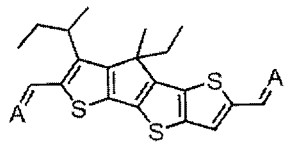 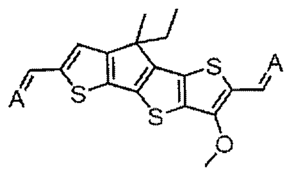 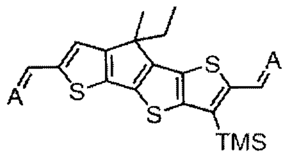

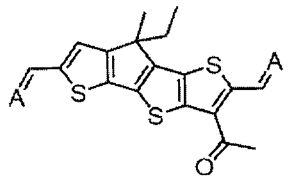 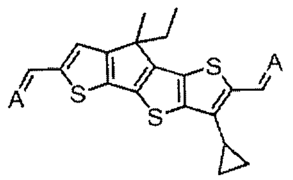 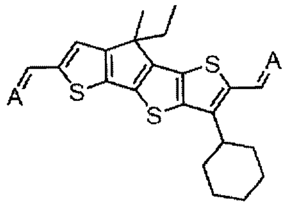

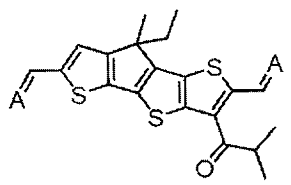 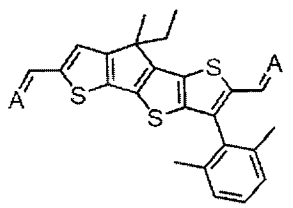 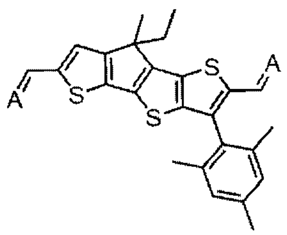

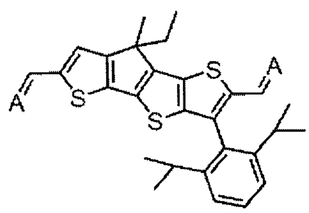 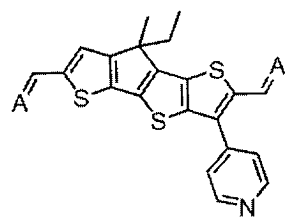 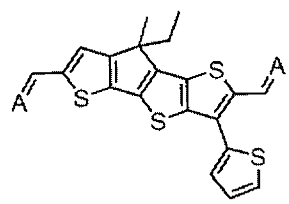

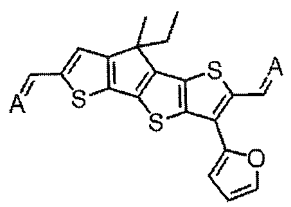 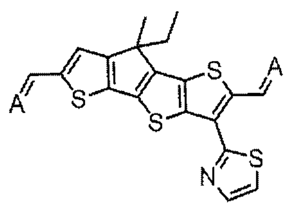 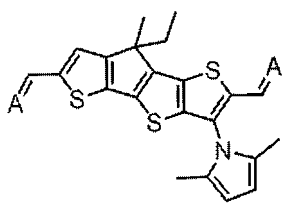

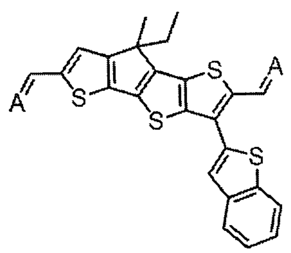 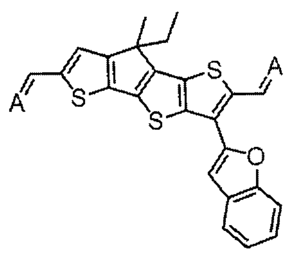 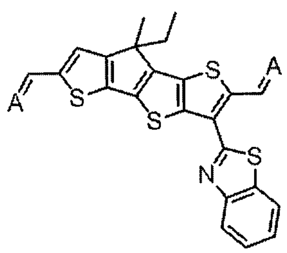

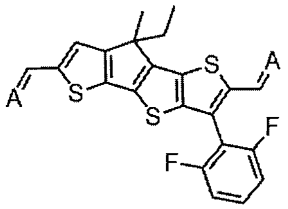 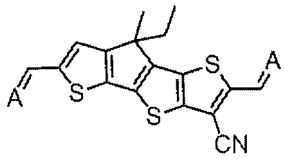 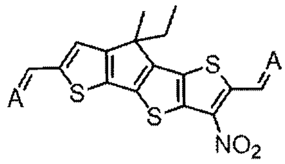

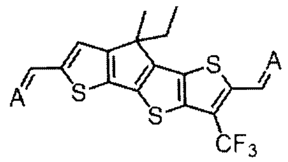 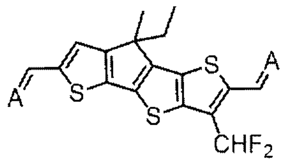 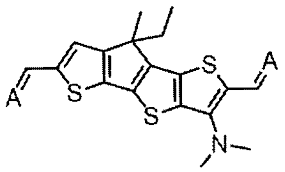

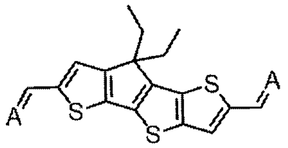 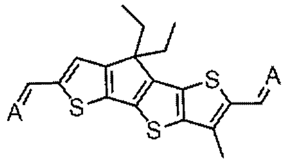 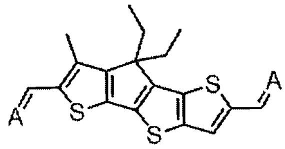

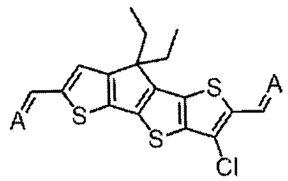 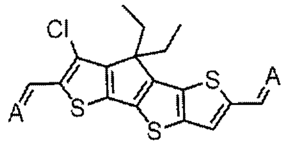 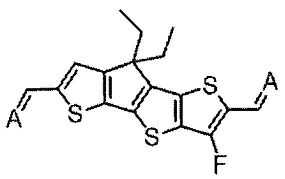

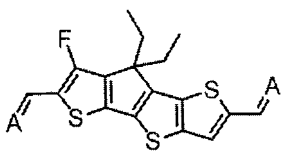 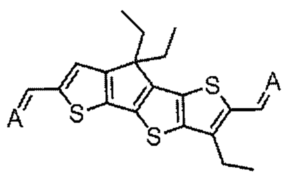 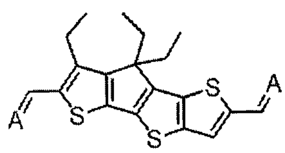

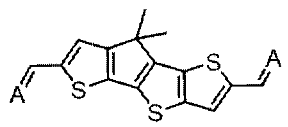 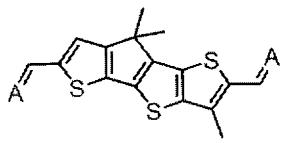 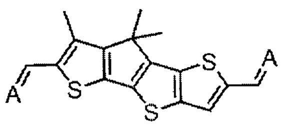

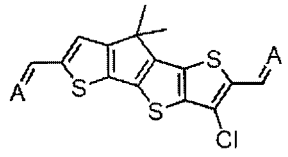 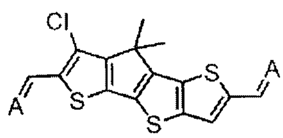 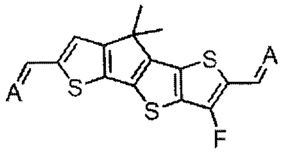

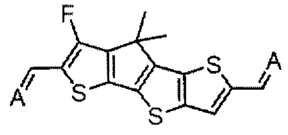 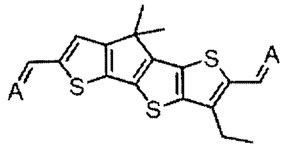 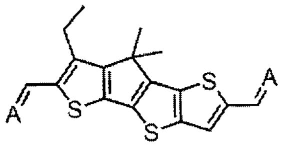

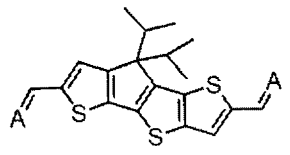 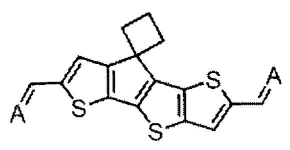 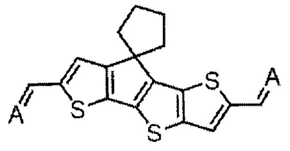

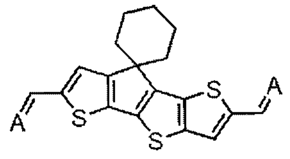 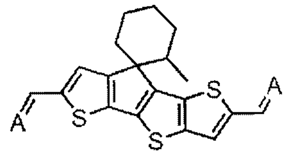 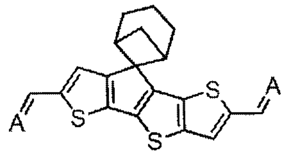

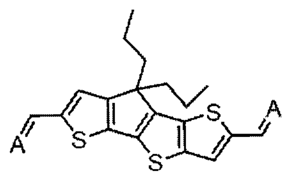 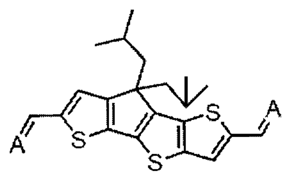 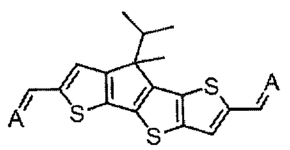

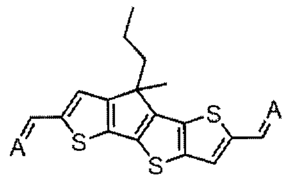 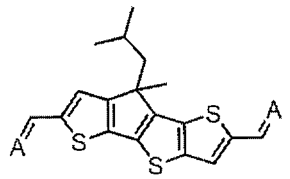 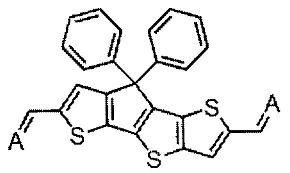

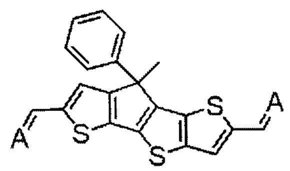 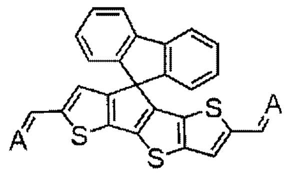 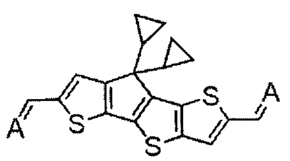

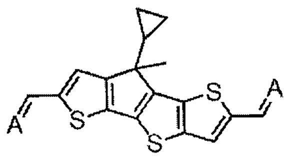 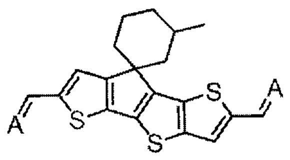 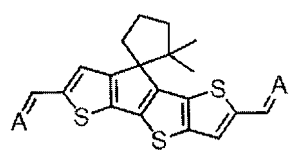

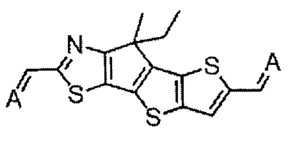 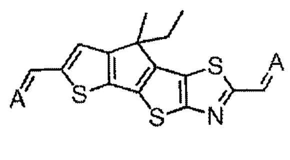 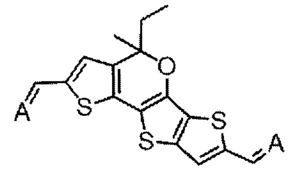

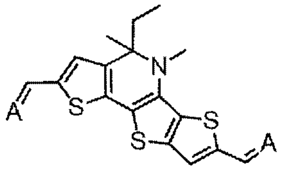 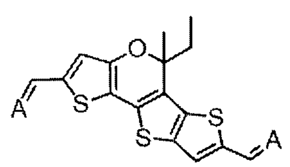 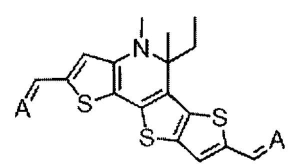

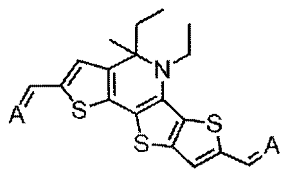
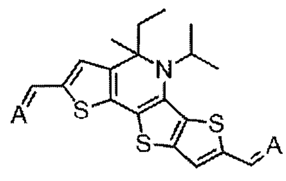
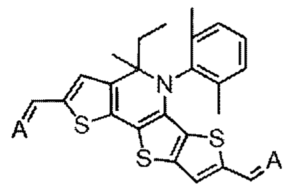

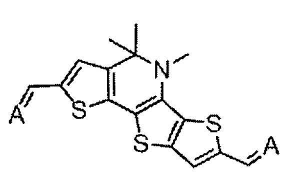
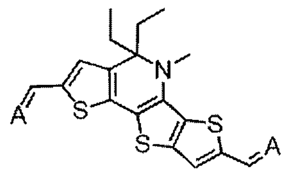
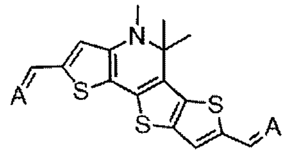

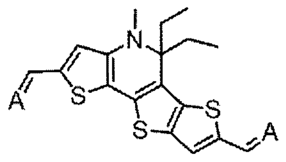
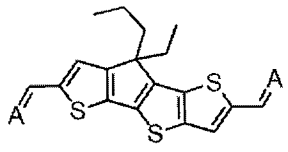
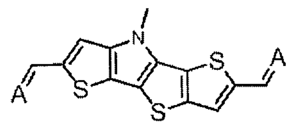

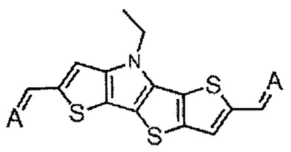
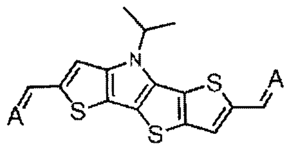
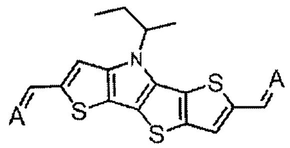

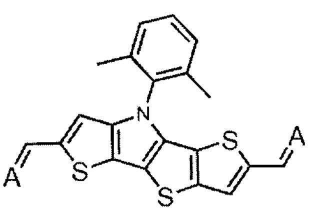
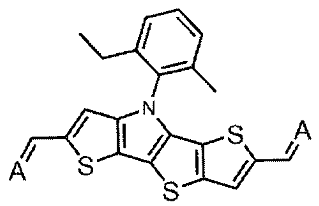
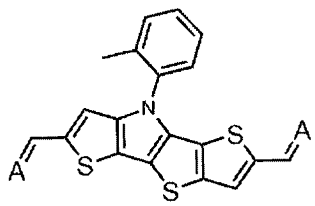

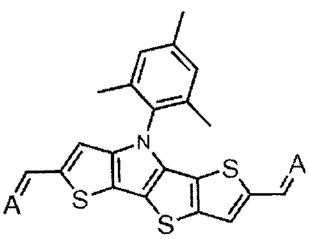
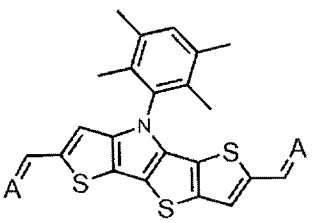
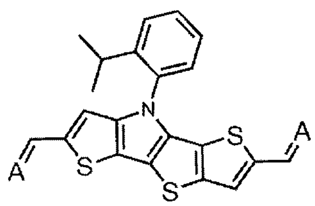

A's in the specific compounds exemplified above are each independently represented by any of the following groups. However, at least one of A's is a monocyclic group.

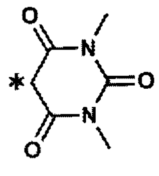
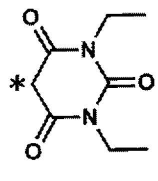
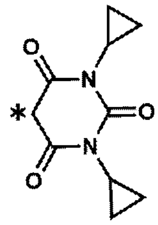
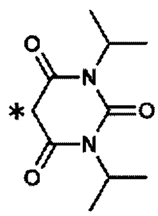

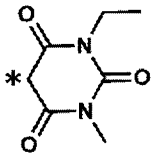 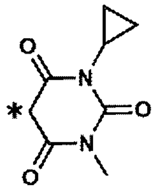 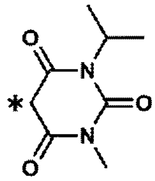 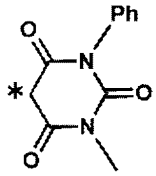

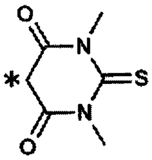 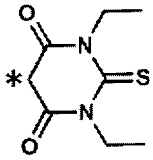 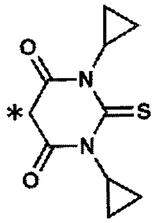 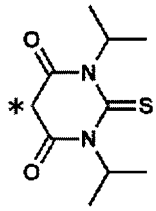

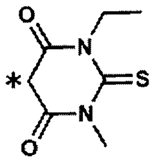 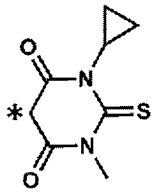 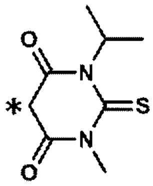 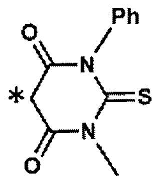

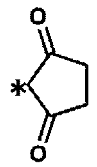 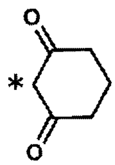 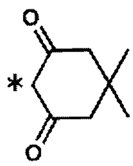 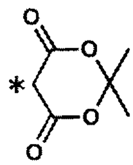 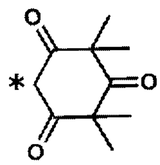

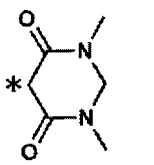 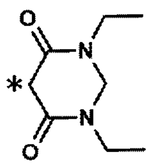 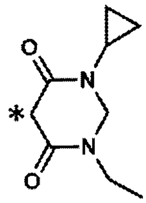 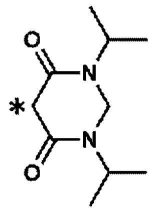

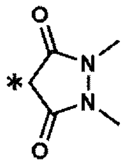 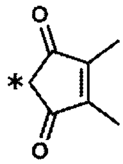 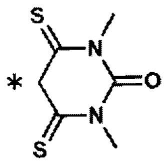 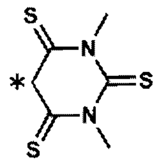 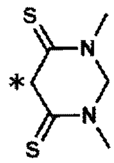

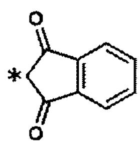 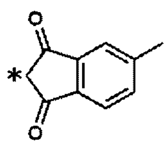 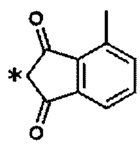 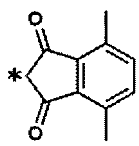 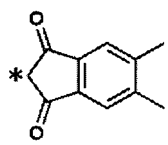

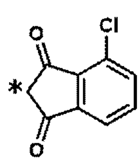 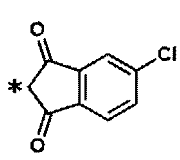 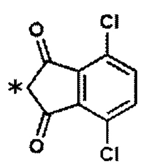 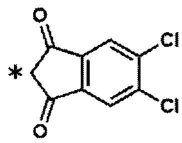 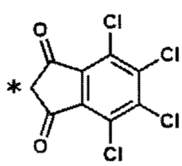

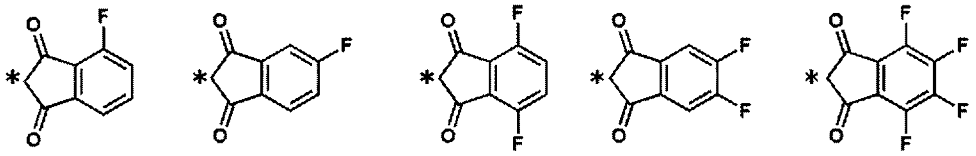
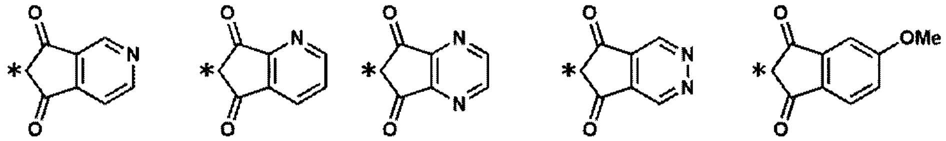
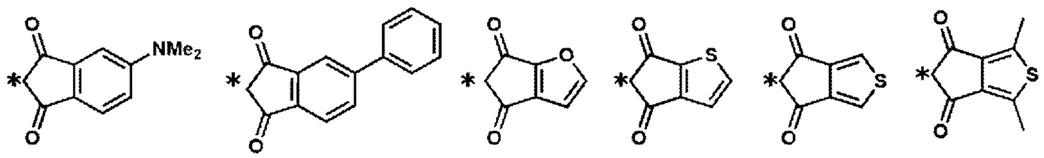
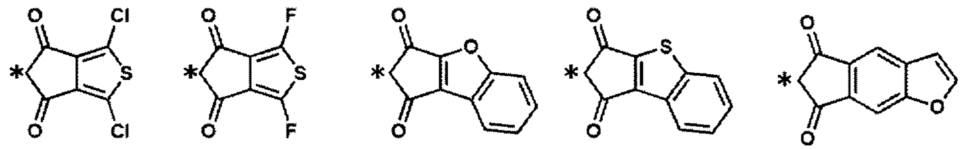
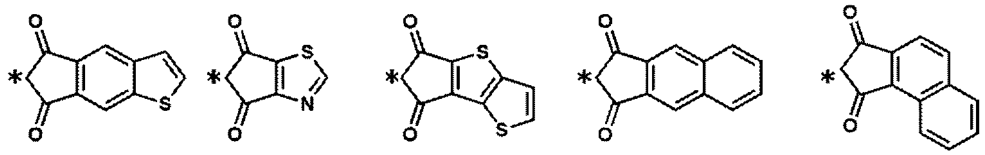
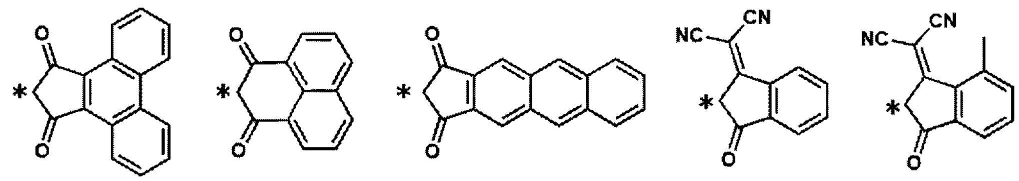
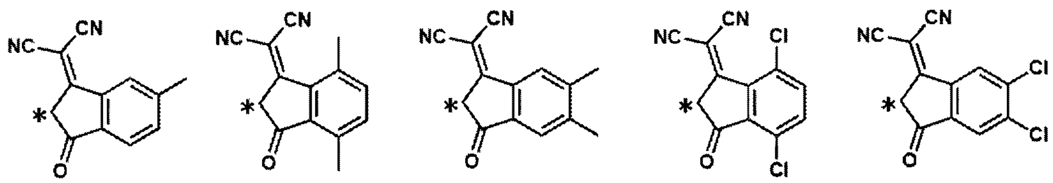
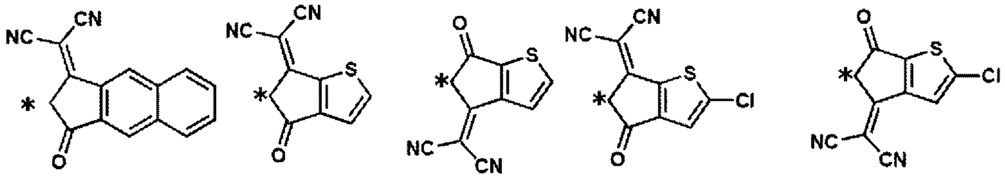

A molecular weight of the specific compound is preferably 450 to 900, more preferably 500 to 800, and still more preferably 550 to 700.

In a case where the molecular weight is in the above-described range, it is presumed that sublimation temperature of the specific compound is low, and thus the manufacturing suitability is excellent.

From the viewpoint of stability in a case of using the specific compound as a p-type organic semiconductor and matching of energy levels between the specific compound and an n-type organic semiconductor, an ionization potential of

the specific compound in a single film is preferably -6.0 to -5.0 eV.

A maximal absorption wavelength of the specific compound is preferably in a wavelength range of 500 to 700 nm, and more preferably in a wavelength range of 500 to 650 nm.

The above-described maximal absorption wavelength is a value measured in a solution state (solvent: chloroform) by adjusting the absorption spectrum of the specific compound to a concentration such that the light absorbance is 0.5 to 1.0. However, in a case where the specific compound is not soluble in chloroform, a value measured by using the specific compound in which the specific compound is vapor-deposited and formed into a film state is defined as the maximal absorption wavelength of the specific compound.

The specific compound is particularly useful as a material of a photoelectric conversion film used for an imaging element, an optical sensor, or a photoelectric cell. The specific compound usually functions as a coloring agent in the photoelectric conversion film. In addition, the specific compound can also be used as a coloring material, a liquid crystal material, an organic semiconductor material, a charge transport material, a pharmaceutical material, and a fluorescent diagnostic material.

The specific compound may be purified as necessary.

Examples of a purification method of the specific compound include sublimation purification, purification using silica gel column chromatography, purification using gel permeation chromatography, re-slurry washing, re-purification by re-precipitation, purification using an adsorbent such as activated carbon, and recrystallization purification.

A content of the specific compound in the photoelectric conversion film (= Film thickness of specific compound in terms of single layer/Film thickness of photoelectric conversion film $\times$ 100) is not particularly limited, but is preferably 5% to 75% by volume, more preferably 10% to 50% by volume, and still more preferably 15% to 40% by volume.

The specific compound may be used alone or in combination of two or more types thereof. In a case where two or more types thereof are used, it is preferable that the total amount thereof is within the above-described range.

<n-type organic semiconductor>

The photoelectric conversion film preferably contains an n-type organic semiconductor, in addition to the specific compound.

The n-type organic semiconductor is a compound different from the above-described specific compound.

The n-type organic semiconductor is an acceptor-type organic semiconductor material (compound), and refers to an organic compound having a property of easily accepting an electron. That is, the n-type organic semiconductor refers to an organic compound having a larger electron affinity in a case where two organic compounds used in contact with each other. That is, any organic compound having an electron accepting property can be used as the acceptor-type organic semiconductor.

Examples of the n-type organic semiconductor include fullerenes selected from the group consisting of a fullerene and derivatives thereof; fused aromatic carbocyclic compounds (for example, a naphthalene derivative, an anthracene derivative, a phenanthrene derivative, a tetracene derivative, a pyrene derivative, a perylene derivative, a fluoranthene derivative, and the like); heterocyclic compounds with a 5- to 7-membered ring having at least one selected from the group consisting of a nitrogen atom, an oxygen atom, and a sulfur atom (for example, pyridine, pyrazine, pyrimidine, pyridazine, triazine, quinoline, quinoxaline, quinazoline, phthalazine, cinnoline, isoquinoline, pteridine, acridine, phenazine, phenanthroline, tetrazole, pyrazole, imidazole, thiazole, and the like); polyarylene compounds; fluorene compounds; cyclopentadiene compounds; silyl compounds; 1,4,5,8-naphthalenetetracarboxylic acid anhydride; 1,4,5,8-naphthalenetetracarboxylic acid anhydride imide derivatives and oxadiazole derivatives; anthraquinodimethane derivatives; diphenylquinone derivatives; bathocuproine, bathophenanthroline, and derivatives thereof; triazole compounds; distyrylarylene derivatives; metal complexes having a nitrogen-containing heterocyclic compound as a ligand; silole compounds; and compounds described in paragraphs [0056] and [0057] of JP2006-100767A.

The n-type organic semiconductor (compound) is preferably fullerenes selected from the group consisting of a fullerene and derivatives thereof.

Examples of the fullerene include a fullerene $C_{60}$, a fullerene $C_{70}$, a fullerene $C_{76}$, a fullerene $C_{78}$, a fullerene $C_{80}$, a fullerene $C_{82}$, a fullerene $C_{84}$, a fullerene $C_{90}$, a fullerene $C_{96}$, a fullerene $C_{240}$, a fullerene $C_{540}$, and a mixed fullerene.

Examples of the derivatives of fullerene include compounds in which a substituent is added to the above-described fullerenes. The above-described substituent is preferably an alkyl group, an aryl group, or a heterocyclic group. As the derivatives of fullerene, compounds described in JP2007-123707A are preferable.

A molecular weight of the n-type organic semiconductor is preferably 200 to 1,200 and more preferably 200 to 900.

A maximal absorption wavelength of the n-type organic semiconductor is preferably in a wavelength of 400 nm or less or in a wavelength range of 500 to 600 nm.

It is preferable that the photoelectric conversion film has a bulk hetero structure formed in a state in which the specific compound and the n-type organic semiconductor are mixed with each other. The bulk hetero structure refers to a

layer in which the specific compound and the n-type organic semiconductor are mixed and dispersed in the photoelectric conversion film. The photoelectric conversion film having the bulk hetero structure can be formed by a wet method or a dry method. The bulk hetero structure is as described in detail in paragraphs [0013] and [0014] of JP2005-303266A.

A difference in electron affinity between the specific compound and the n-type organic semiconductor is preferably 0.1 eV or more.

The n-type organic semiconductor may be used alone or in combination of two or more types thereof.

In a case where the photoelectric conversion film contains the n-type organic semiconductor, a content of the n-type organic semiconductor in the photoelectric conversion film (Film thickness of n-type organic semiconductor in terms of single layer/Film thickness of photoelectric conversion film × 100) is preferably 15% to 75% by volume, more preferably 20% to 60% by volume, and still more preferably 20% to 50% by volume.

In a case where the n-type organic semiconductor includes fullerenes, a content of the fullerenes to the total content of the n-type organic semiconductor (Film thickness of fullerenes in terms of single layer/Total film thickness of n-type organic semiconductors in terms of single layer × 100) is preferably 50% to 100% by volume, and more preferably 80% to 100% by volume. The fullerenes may be used alone or in combination of two or more types thereof.

From the viewpoint of response speed of the photoelectric conversion element, the content of the specific compound to the total content of the specific compound and the n-type organic semiconductor (Film thickness of specific compound in terms of single layer/(Film thickness of specific compound in terms of single layer + Film thickness of n-type organic semiconductor in terms of single layer) × 100) is preferably 20% to 80% by volume, and more preferably 40% to 80% by volume.

In a case where the photoelectric conversion film contains the n-type organic semiconductor and a p-type organic semiconductor, the content of the specific compound (Film thickness of specific compound in terms of single layer/(Film thickness of specific compound in terms of single layer + Film thickness of n-type organic semiconductor in terms of single layer + Film thickness of p-type organic semiconductor in terms of single layer) × 100) is preferably 10% to 75% by volume, and more preferably 15% to 50% by volume.

It is preferable that the photoelectric conversion film substantially contains the specific compound, the n-type organic semiconductor, and the p-type organic semiconductor contained as desired. The term "substantially" indicates that the total content of the specific compound, the n-type organic semiconductor, and the p-type organic semiconductor is 90% to 100% by volume, preferably 95% to 100% by volume, and more preferably 99% to 100% by volume with respect to the total mass of the photoelectric conversion film.

<p-type organic semiconductor>

The photoelectric conversion film preferably contains a p-type organic semiconductor in addition to the above-described specific compound.

The p-type organic semiconductor is a compound different from the above-described specific compound.

The p-type organic semiconductor is a donor-type organic semiconductor material (compound), and refers to an organic compound having a property of easily donating an electron. That is, the p-type organic semiconductor refers to an organic compound having a smaller ionization potential in a case where two organic compounds are used in contact with each other.

The p-type organic semiconductor may be used alone or in combination of two or more types thereof.

Examples of the p-type organic semiconductor include triarylamine compounds (for example, N,N'-bis(3-methylphenyl)-(1,1'-biphenyl)-4,4'-diamine (TPD), 4,4'-bis[N-(naphthyl)-N-Phenyl-amino] biphenyl (α-NPD), compounds described in paragraphs [0128] to [0148] of JP2011-228614A, compounds described in paragraphs [0052] to [0063] of JP2011-176259A, compounds described in paragraphs [0119] to [0158] of JP2011-225544A, compounds described in paragraphs [0044] to [0051] of JP2015-153910A, compounds described in paragraphs [0086] to [0090] of JP2012-094660A, and the like), pyrazoline compounds, styrylamine compounds, hydrazone compounds, polysilane compounds, thiophene compounds (for example, a thienothiophene derivative, a dibenzothiophene derivative, a benzodithiophene derivative, a dithienothiophene derivative, a [1]benzothieno[3,2-b]thiophene (BTBT) derivative, a thieno[3,2-f:4,5-f']bis[1]benzothiophene (TBBT) derivative, compounds described in paragraphs [0031] to [0036] of JP2018-014474A, compounds described in paragraphs [0043] to [0045] of WO2016/194630A, compounds described in paragraphs [0025] to [0037], and [0099] to [0109] of WO2017/159684A, compounds described in paragraphs [0029] to [0034] of JP2017-076766A, compounds described in paragraphs [0015] to [0025] of WO2018/207722A, compounds described in paragraphs [0045] to [0053] of JP2019-054228A, compounds described in paragraphs [0045] to [0055] of WO2019/058995A, compounds described in paragraphs [0063] to [0089] of WO2019/081416A, compounds described in paragraphs [0033] to [0036] of JP2019-80052A, compounds described in paragraphs [0044] to [0054] of WO2019/054125A, compounds described in paragraphs [0041] to [0046] of WO2019/093188A, compounds described in paragraphs [0034] to [0037] of JP2019-050398A, compounds described in paragraphs [0033] to [0036] of JP2018-206878A, compounds described n paragraph [0038] of JP2018-190755A, compounds described in paragraphs

[0019] to [0021] of JP2018-026559A, compounds described in paragraphs [0031] to [0056] of JP2018-170487A, compounds described in paragraphs [0036] to [0041] of JP2018-078270A, compounds described in paragraphs [0055] to [0082] of JP2018-166200A, compounds described in paragraphs [0041] to [0050] of JP2018-113425A, compounds described in paragraphs [0044] to [0048] of JP2018-085430A, compounds described in paragraphs [0041] to [0045] of JP2018-056546A, compounds described in paragraphs [0042] to [0049] of JP2018-046267A, compounds described in paragraphs [0031] to [0036] of JP2018-014474A, compounds described in paragraphs [0036] to [0046] of WO2018/016465A, compounds described in paragraphs [0045] to [0048] of JP2020-010024A, and the like), a cyanine compound, an oxonol compound, a polyamine compound, an indole compound, a pyrrole compound, a pyrazole compound, a polyarylene compound, a fused aromatic carbocyclic compound (for example, a naphthalene derivative, an anthracene derivative, a phenanthrene derivative, a tetracene derivative, a pentacene derivative, a pyrene derivative, a perylene derivative, a fluoranthene derivative, and the like), a porphyrin compound, a phthalocyanine compound, a triazole compound, an oxadiazole compound, an imidazole compound, a polyarylalkane compound, a pyrazolone compound, an amino-substituted chalcone compound, an oxazole compound, a fluorenone compound, a silazane compound, and a metal complex having a nitrogen-containing heterocyclic compound as a ligand.

In addition, examples of the p-type organic semiconductor also include compounds described in JP2022-123944A, compounds described in JP2022-122839A, compounds described in JP2022-120323A, compounds described in JP2022-120273A, compounds described in JP2022-115832A, compounds described in JP2022-108268A, compounds described in JP2023-005703A, compounds described in JP2022-100258A, compounds described in JP2022-181226A, compounds described in JP2022-27575A, and compounds described in JP2021-163968A.

Examples of the p-type organic semiconductor also include compounds having an ionization potential smaller than that of the n-type organic semiconductor, and in a case where this condition is satisfied, the organic coloring agent exemplified as the n-type organic semiconductor can be used.

Compounds which can be used as the p-type organic semiconductor compound are exemplified below.

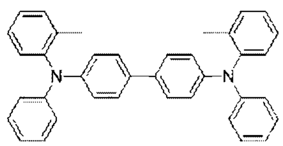
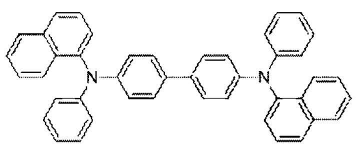
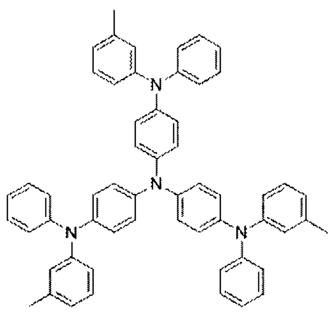
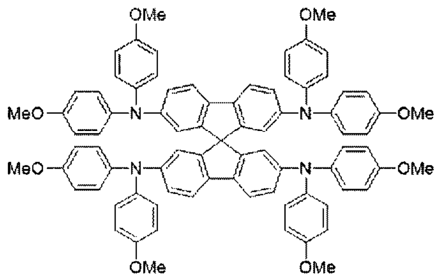

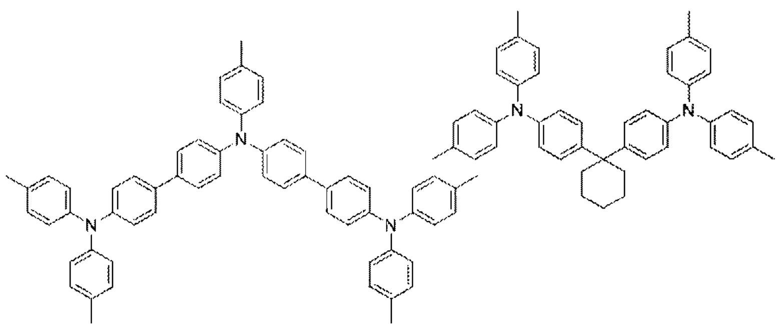
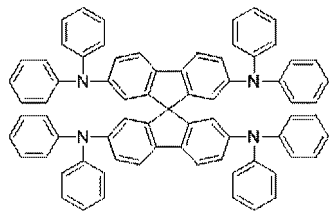

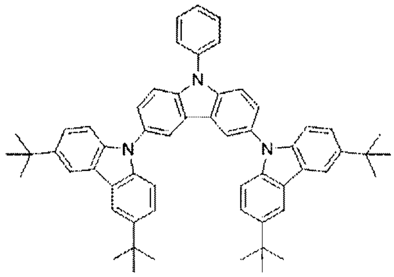
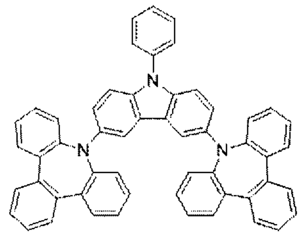
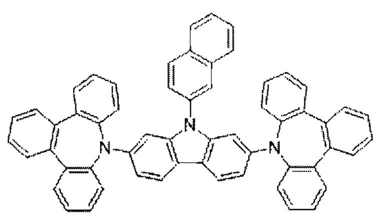

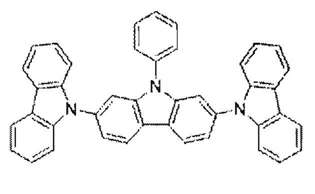
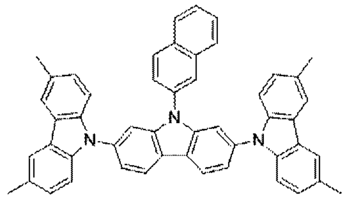
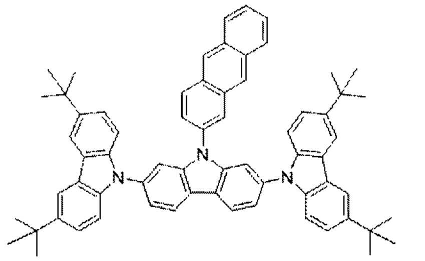

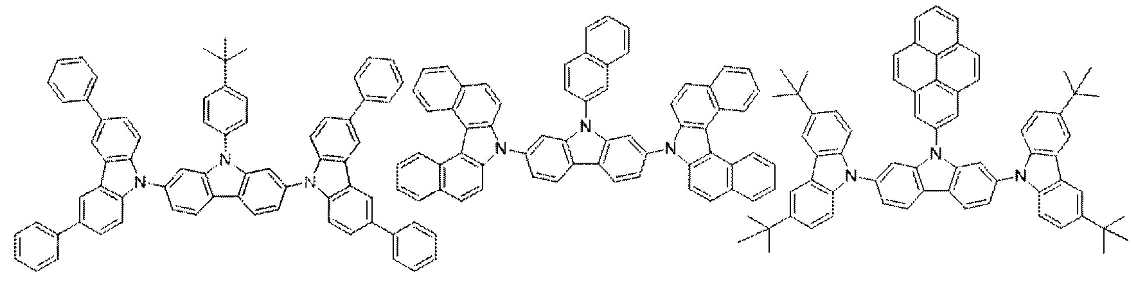

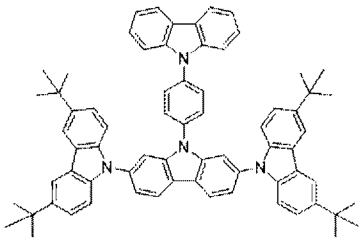
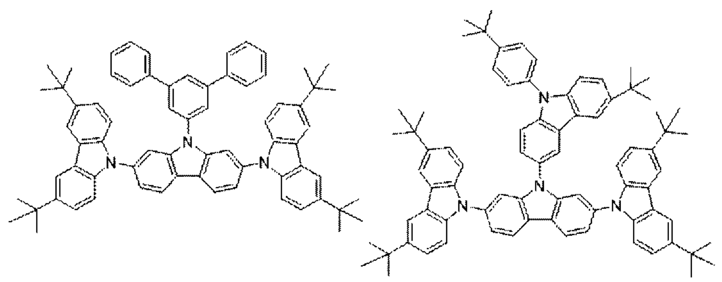

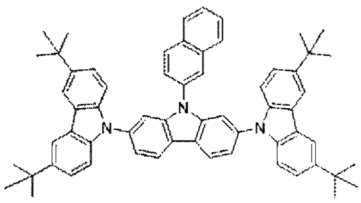
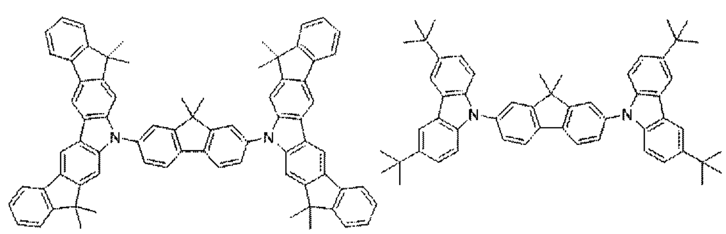

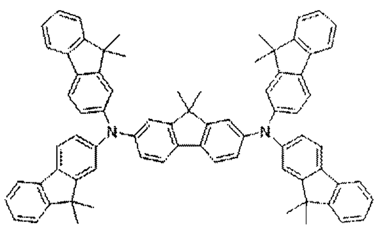
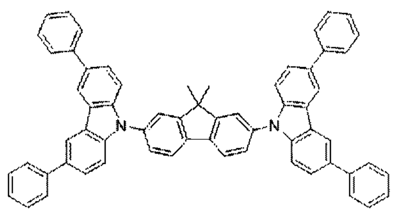
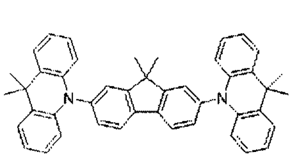

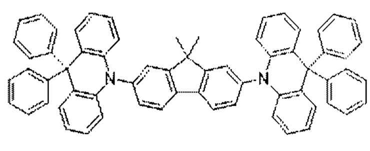
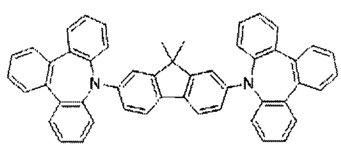
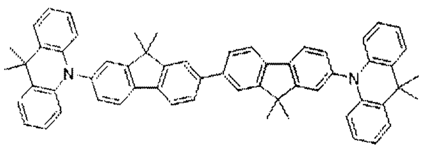

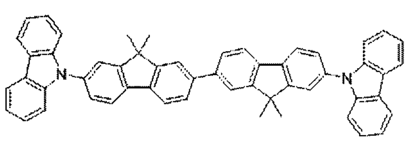
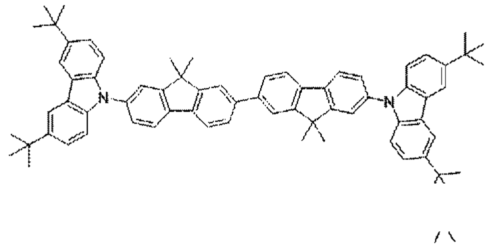

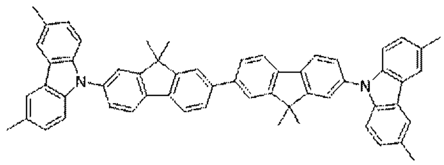
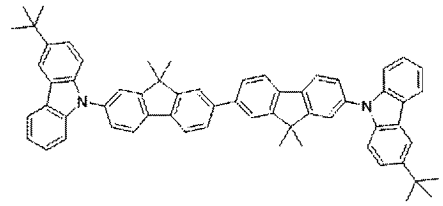

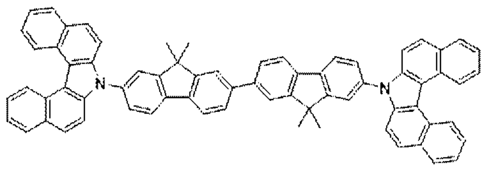
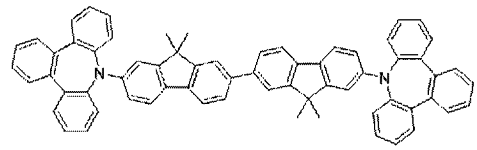

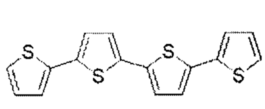
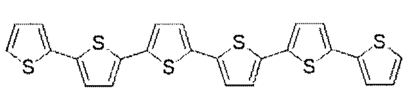
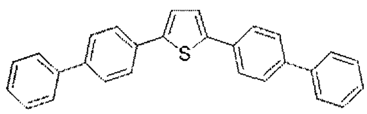

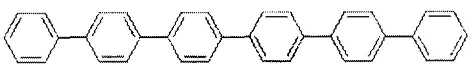
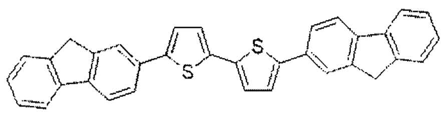

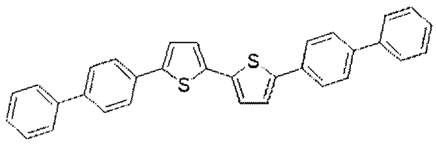
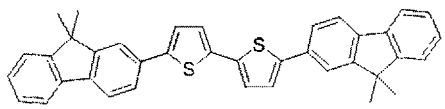

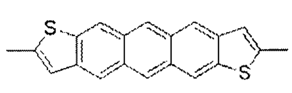
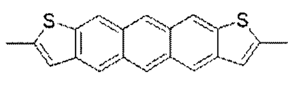
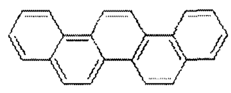
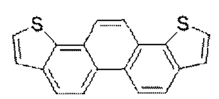

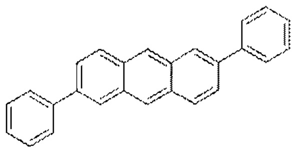
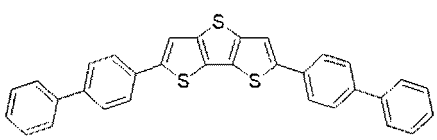
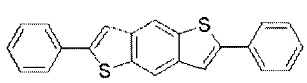

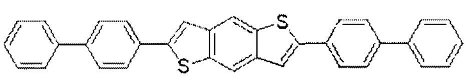
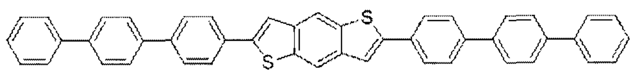

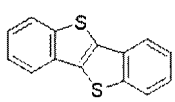
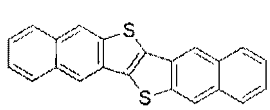
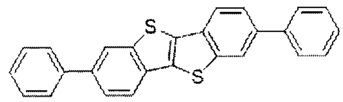

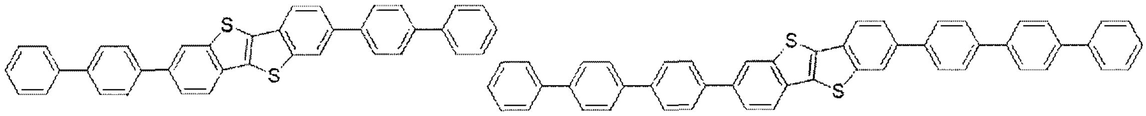

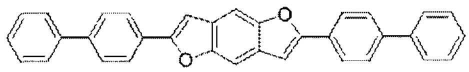

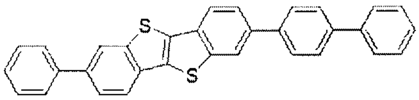

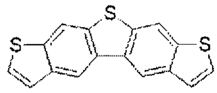

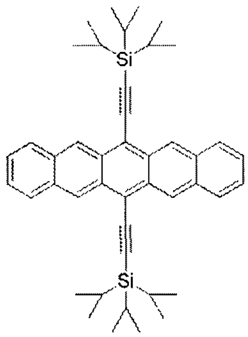

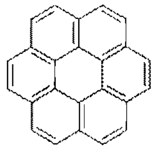

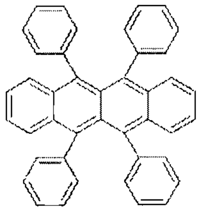

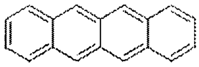

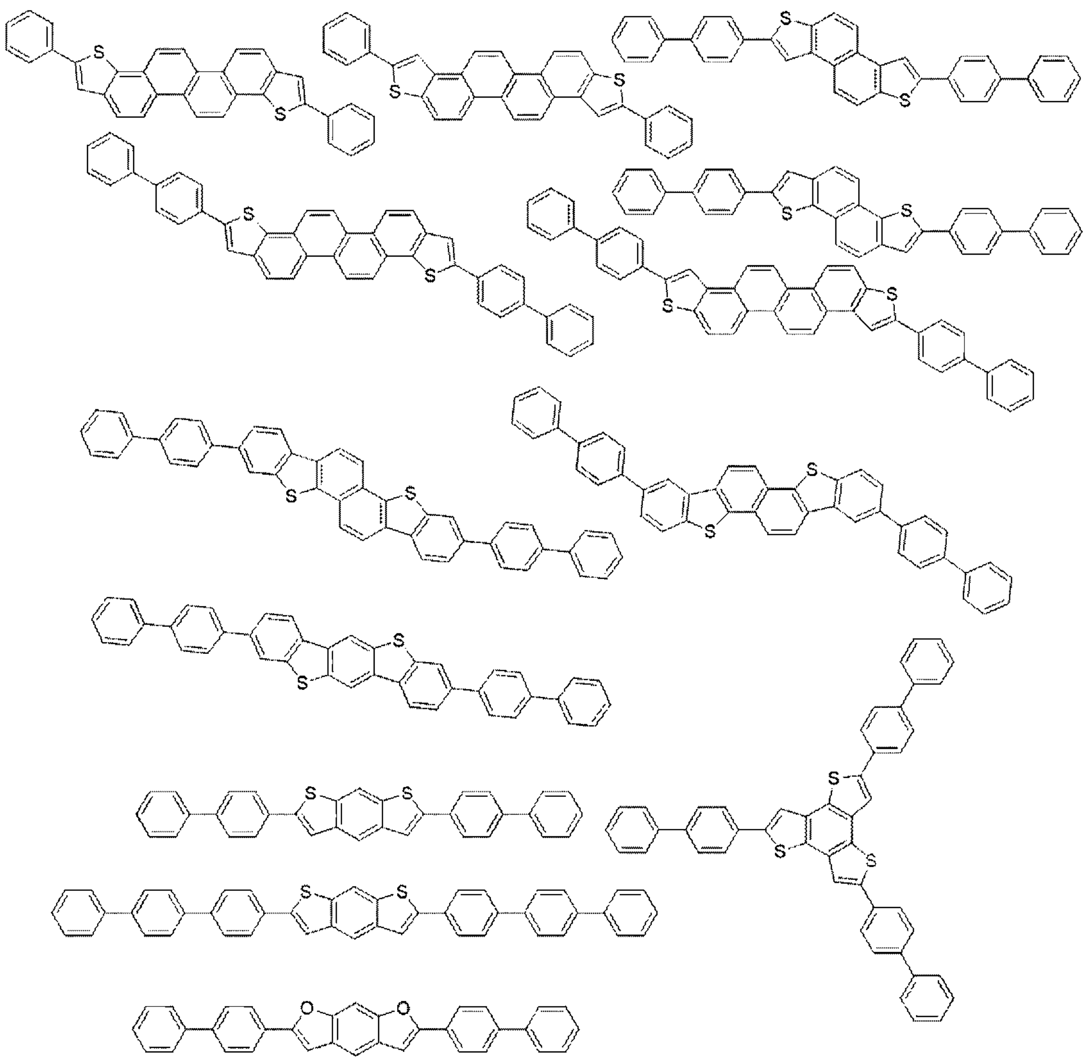

A difference in ionization potential between the specific compound and the p-type organic semiconductor is preferably 0.1 eV or more.

The p-type organic semiconductor material may be used alone or in combination of two or more types thereof.

In a case where the photoelectric conversion film contains the p-type organic semiconductor, a content of the p-type organic semiconductor in the photoelectric conversion film (Film thickness of p-type organic semiconductor in terms of single layer/Film thickness of photoelectric conversion film × 100) is preferably 15% to 75% by volume, more preferably 20% to 60% by volume, and still more preferably 25% to 50% by volume.
The photoelectric conversion film containing the specific compound is a non-luminescent film, and has a feature different from an organic light emitting diode (OLED). The non-luminescent film refers to a film having a light emission quantum efficiency of 1% or less, and the light emission quantum efficiency is preferably 0.5% or less and more preferably 0.1% or less. The lower limit thereof is often 0% or more.

<Coloring agent>

The photoelectric conversion film preferably contains a coloring agent in addition to the above-described specific compound.
The coloring agent is a compound different from the above-described specific compound.
As the coloring agent, an organic coloring agent is preferable.
Examples of the organic coloring agent include a cyanine coloring agent, a styryl coloring agent, a hemicyanine coloring agent, a merocyanine coloring agent (including zeromethine merocyanine (simple merocyanine)), a rhodacyanine coloring agent, an allopolar coloring agent, an oxonol coloring agent, a hemioxonol coloring agent, a squarylium coloring agent, a croconium coloring agent, an azamethine coloring agent, a coumarin coloring agent, an arylidene coloring agent, an anthraquinone coloring agent, a triphenylmethane coloring agent, an azo coloring agent, an azomethine coloring agent, a metallocene coloring agent, a fluorenone coloring agent, a flugide coloring agent, a perylene coloring agent, a phenazine coloring agent, a phenothiazine coloring agent, a quinone coloring agent, a diphenylmethane coloring agent, a polyene coloring agent, an acridine coloring agent, an acridinone coloring agent, a diphenylamine coloring agent, a quinophthalone coloring agent, a phenoxazine coloring agent, a phthaloperylene coloring agent, a dioxane coloring agent, a porphyrin coloring agent, a chlorophyll coloring agent, a phthalocyanine coloring agent, a subphthalocyanine coloring agent, a metal complex, an imidazoquinoxaline coloring agent described in WO2020/013246A, WO2022/168856A, JP2023-10305A, and JP2023-10299A, an acceptor-donor-acceptor type coloring agent in which two acidic nuclei are bonded to a donor, and a donor-acceptor-donor type coloring agent in which two donors are bonded to an acceptor. Among these, from the viewpoint of maximal absorption wavelength and the like, a cyanine coloring agent, an imidazoquinoxaline coloring agent, or an acceptor-donor-acceptor type coloring agent is preferable.
A maximal absorption wavelength of the coloring agent is preferably in the visible light region, more preferably in a wavelength range of 400 to 650 nm, and still more preferably in a wavelength range of 400 to 550 nm.
The coloring agent may be used alone or in combination of two or more types thereof.
A content of the coloring agent with respect to the total content of the specific compound and the coloring agent in the photoelectric conversion film (= (Film thickness of coloring agent in terms of single layer/(Film thickness of specific compound in terms of single layer + Film thickness of coloring agent in terms of single layer) × 100)) is preferably 5% to 75% by volume, more preferably 5% to 60% by volume, and still more preferably 5% to 50% by volume.

<Film formation method>

Examples of a film formation method of the above-described photoelectric conversion film include a dry film formation method.
Examples of the dry film formation method include a physical vapor deposition method such as a vapor deposition method (particularly, a vacuum vapor deposition method), a sputtering method, an ion plating method, and a molecular beam epitaxy (MBE) method, and a chemical vapor deposition (CVD) method such as plasma polymerization; and a vacuum vapor deposition method is preferable. In a case where the photoelectric conversion film is formed by the vacuum vapor deposition method, manufacturing conditions such as a degree of vacuum and a vapor deposition temperature can be set according to the conventional method.
A film thickness of the photoelectric conversion film is preferably 10 to 1,000 nm, more preferably 50 to 800 nm, and still more preferably 50 to 500 nm.

[Electrode]

The photoelectric conversion element preferably includes an electrode.
The electrode (the upper electrode (transparent conductive film) 15 and the lower electrode (conductive film) 11) contains a conductive material. Examples of the conductive material include metals, alloys, metal oxides, electrically conductive compounds, and mixtures thereof.

Since light is incident through the upper electrode 15, the upper electrode 15 is preferably transparent to light to be detected. Examples of a material constituting the upper electrode 15 include conductive metal oxides such as tin oxide doped with antimony, fluorine, or the like (antimony tin oxide (ATO) and fluorine doped tin oxide (FTO)), tin oxide, zinc oxide, indium oxide, indium tin oxide (ITO), and indium zinc oxide (IZO); metal thin films such as gold, silver, chromium, and nickel; mixtures or laminates of these metals and the conductive metal oxides; and organic conductive materials such as polyaniline, polythiophene, and polypyrrole; and nano carbon materials such as carbon nanotubes and graphene. From the viewpoint of high conductivity and transparency, conductive metal oxides are preferable.

In general, in a case where the conductive film is thinner than a certain range, a resistance value rapidly increases in many cases. In a solid-state imaging element in which the photoelectric conversion element according to the present embodiment is incorporated, a sheet resistance may be 100 to 10,000 Ω/□, and a degree of freedom of the film thickness range which can be reduced is large.

In addition, as the film thickness of the upper electrode (transparent conductive film) 15 is thinner, the amount of light which is absorbed in the upper electrode is smaller, and thus light transmittance usually increases. The increase in the light transmittance causes an increase in light absorbance in the photoelectric conversion film and an increase in the photoelectric conversion ability, which is preferable. Considering suppression of leakage current, increase in resistance value of the thin film, and increase in transmittance accompanied by the thinning, the thickness of the upper electrode 15 is preferably 5 to 100 nm, and more preferably 5 to 20 nm.

There is a case where the lower electrode 11 has transparency or an opposite case where the lower electrode does not have transparency and reflects light, depending on use. Examples of a material constituting the lower electrode 11 include conductive metal oxides such as tin oxide doped with antimony, fluorine, or the like (ATO and FTO), tin oxide, zinc oxide, indium oxide, indium tin oxide (ITO), and indium zinc oxide (IZO); metals such as gold, silver, chromium, nickel, titanium, tungsten, and aluminum; conductive compounds such as oxides or nitrides of these metals (for example, titanium nitride (TiN)); mixtures or laminates of these metals and conductive metal oxides; organic conductive materials such as polyaniline, polythiophene, and polypyrrole; and carbon materials such as carbon nanotubes and graphene.

A method of forming the electrode can be appropriately selected in accordance with the electrode material. Specific examples thereof include a wet method such as a printing method and a coating method; a physical method such as a vacuum vapor deposition method, a sputtering method, and an ion plating method; and a chemical method such as a CVD method and a plasma CVD method.

In a case where the electrode material is ITO, examples thereof include an electron beam method, a sputtering method, a resistance thermal vapor deposition method, a chemical reaction method (such as a sol-gel method), and a coating method with a dispersion of indium tin oxide.

[Charge blocking film: electron blocking film and hole blocking film]

It is preferable that the photoelectric conversion element includes one or more interlayers between the conductive film and the transparent conductive film, in addition to the photoelectric conversion film.

Examples of the above-described interlayer include a charge blocking film. In a case where the photoelectric conversion element includes the film, the characteristics (such as the quantum efficiency and the response speed) of the photoelectric conversion element to be obtained are more excellent. Examples of the charge blocking film include an electron blocking film and a hole blocking film.

<Electron blocking film>

The electron blocking film is a donor-type organic semiconductor material (compound), and the above-described p-type organic semiconductor can be used.

In addition, a polymer material can also be used in the electron blocking film.

Specific examples of the polymer material include a polymer such as phenylenevinylene, fluorene, carbazole, indole, pyrene, pyrrole, picoline, thiophene, acetylene, and diacetylene, and derivatives thereof.

The electron blocking film may be configured by a plurality of films.

The electron blocking film may be formed of an inorganic material. In general, since an inorganic material has a dielectric constant larger than that of an organic material, in a case where the inorganic material is used in the electron blocking film, a large voltage is applied to the photoelectric conversion film. Therefore, the quantum efficiency increases. Examples of the inorganic material which can be used in the electron blocking film include calcium oxide, chromium oxide, copper chromium oxide, manganese oxide, cobalt oxide, nickel oxide, copper oxide, copper gallium oxide, copper strontium oxide, niobium oxide, molybdenum oxide, copper indium oxide, silver indium oxide, and iridium oxide.

<Hole blocking film>

The hole blocking film is an acceptor-type organic semiconductor material (compound), and the above-described n-type organic semiconductor described above can be used.
The hole blocking film may be configured by a plurality of films.
Examples of a method of manufacturing the charge blocking film include a dry film formation method and a wet film formation method. Examples of the dry film formation method include a vapor deposition method and a sputtering method. The vapor deposition method may be a physical vapor deposition (PVD) method or a chemical vapor deposition (CVD) method, and a physical vapor deposition method such as a vacuum vapor deposition method is preferable. Examples of the wet film formation method include an ink jet method, a spray method, a nozzle printing method, a spin coating method, a dip coating method, a casting method, a die coating method, a roll coating method, a bar coating method, and a gravure coating method; and an inkjet method is preferable from the viewpoint of high-precision patterning.
Each film thickness of the charge blocking films (the electron blocking film and the hole blocking film) is preferably 3 to 200 nm, more preferably 5 to 100 nm, and still more preferably 5 to 30 nm.

[Substrate]

The photoelectric conversion element may further include a substrate.
Examples of the substrate include a semiconductor substrate, a glass substrate, and a plastic substrate.
As a position of the substrate, in general, the conductive film, the photoelectric conversion film, and the transparent conductive film are laminated on the substrate in this order.

[Sealing layer]

The photoelectric conversion element may further include a sealing layer.
The performance of the photoelectric conversion material may deteriorate significantly due to the presence of deterioration factors such as water molecules. The deterioration can be prevented by coating and sealing the entirety of the photoelectric conversion film with a sealing layer such as diamond-like carbon (DLC) and ceramics such as metal oxide, metal nitride, or metal nitride oxide, which are dense and into which water molecules do not permeate.
Examples of the sealing layer include sealing layers described in paragraphs [0210] to [0215] of JP2011-082508A, the contents of which are incorporated herein by reference.

[Manufacturing method of photoelectric conversion element]

Examples of a method for manufacturing the photoelectric conversion element include known manufacturing methods.
Specific examples thereof include a method for manufacturing the photoelectric conversion element, which includes a step of forming the conductive film on the substrate, a step of forming the photoelectric conversion film, and a step of forming the transparent conductive film.
The method for manufacturing the photoelectric conversion element may include a step other than the above-described steps (for example, a step of forming the charge blocking film and a step of forming the sealing layer).
The method of forming each layer is as described above.

[Imaging element]

Examples of the application of the photoelectric conversion element include an imaging element.
The imaging element is an element which converts optical information of an image into the electric signal, and is usually an element in which a plurality of photoelectric conversion elements are arranged in a matrix on the same plane, optical signals are converted into electric signals in each photoelectric conversion element (a pixel), and the electric signals can be sequentially output to the outside of the imaging elements for each pixel. Therefore, each pixel is formed of one or more photoelectric conversion elements and one or more transistors.

[Optical sensor]

Examples of another application of the photoelectric conversion element include a photoelectric cell and an optical sensor, but it is preferable that the photoelectric conversion element according to the embodiment of the present invention is used as the optical sensor. The above-described photoelectric conversion element may be used alone as the optical sensor, or may be used as a line sensor in which the photoelectric conversion elements are linearly arranged or as a

two-dimensional sensor in which the photoelectric conversion elements are arranged on a plane.

[Compound]

The present invention also includes the specific compound.

Examples

Hereinbelow, the present invention will be described in more detail with reference to Examples.

The materials, the amounts of materials used, the proportions, the treatment details, the treatment procedure, and the like shown in Examples below may be modified as appropriate as long as the modifications do not depart from the spirit of the present invention. Therefore, the scope of the present invention should not be construed as being limited to Examples shown below.

[Compound used for photoelectric conversion film]

Hereinafter, each material used for a photoelectric conversion film is shown.

[Synthesis of compound (1-1)]

A compound (1-1) was synthesized according to the following scheme.

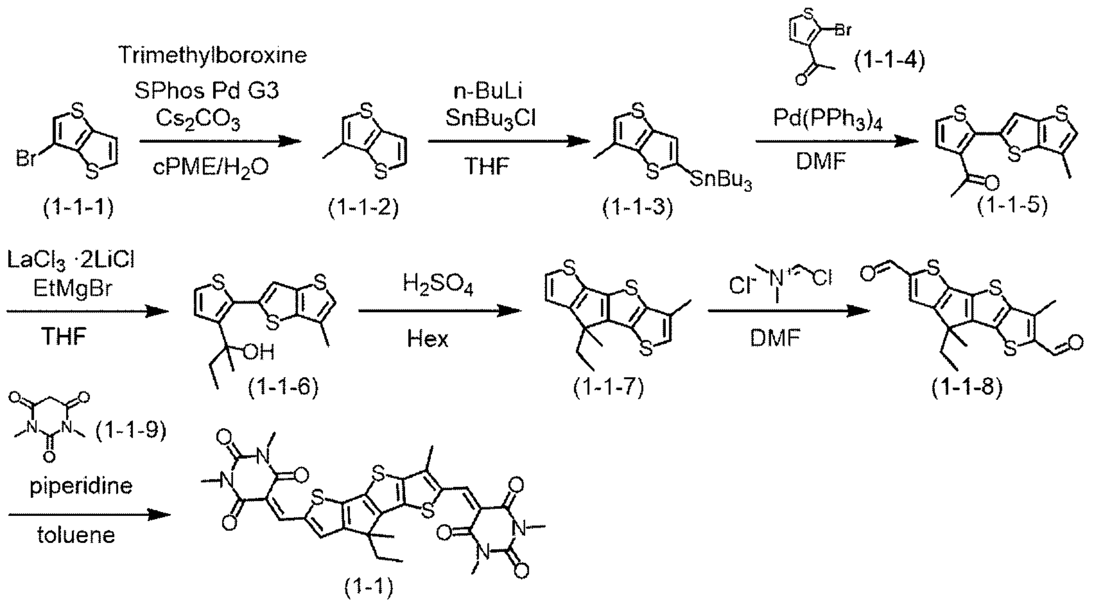


<Synthesis of compound (1-1-2)>

A compound (1-1-1) (10.0 g, 42.9 mmol), trimethylboroxine (9.06 mL, 64.3 mmol), cesium carbonate (28.0 g, 85.8 mmol), cyclopentyl methyl ether (180 mL), and water (20 mL) were mixed with each other, subjected to vacuum degassing, and then SPhos Pd G3 (1.76 g, 2.14 mmol) was added thereto, followed by stirring at 100°C for 2 hours. The mixture was allowed to cool to room temperature, and the insoluble matter was removed by Celite filtration. The filtrate was diluted with ethyl acetate, the aqueous layer was removed with a separating funnel, and the obtained organic layer was dried over magnesium sulfate, and then filtered and concentrated under reduced pressure. The obtained crude product was purified by silica gel chromatography (eluent (volume ratio): hexane/ethyl acetate = 98/2) to obtain a compound (1-1-2) (6.5 g, 98%).

<Synthesis of compound (1-1-3)>

The compound (1-1-2) (6.20 g, 40.2 mmol) and tetrahydrofuran (THF, 201 mL) were mixed with each other, and an n-butyllithium solution 2.66 M in hexane (21.2 mL, 56.3 mmol) was slowly added dropwise to the mixture at -78°C,

followed by stirring at -78°C for 30 minutes. Tributyltin chloride (16 mL, 60.3 mmol) was slowly added dropwise to the reaction solution, and the reaction solution was stirred in an ice bath for 30 minutes. A saturated ammonium chloride aqueous solution (120 mL) was slowly added to the reaction solution, and then the reaction solution was stirred at room temperature for 15 minutes, extracted with hexane using a separating funnel, and the obtained organic layer was dried over magnesium sulfate, filtered, and concentrated under reduced pressure. The obtained crude product was purified by aminosilica gel chromatography (eluent: hexane) to obtain a compound (1-1-3) (17.0 g, 95%).

<Synthesis of compound (1-1-5)>

The compound (1-1-3) (15.8 g, 35.7 mmol), a compound (1-1-4) (7.0 g, 32.4 mmol), and DMF (N,N-dimethylformamide, 105 mL) were mixed with each other, subjected to vacuum degassing, and then tetrakis(triphenylphosphine) palladium (0) (1.12 g, 0.97 mmol) was added thereto, followed by stirring at 100°C for 2 hours. The mixture was allowed to cool to room temperature, and the solvent was distilled off under reduced pressure. The obtained crude product was purified by silica gel chromatography (eluent (volume ratio): hexane/ethyl acetate = 80/20) to obtain a compound (1-1-5) (5.0 g, 55%).

<Synthesis of compound (1-1-5)>

The compound (1-1-3) (15.8 g, 35.7 mmol), a compound (1-1-4) (7.0 g, 32.4 mmol), and DMF (N,N-dimethylformamide, 105 mL) were mixed with each other, subjected to vacuum degassing, and then tetrakis(triphenylphosphine) palladium (0) (1.12 g, 0.97 mmol) was added thereto, followed by stirring at 100°C for 2 hours. The mixture was allowed to cool to room temperature, and the solvent was distilled off under reduced pressure. The obtained crude product was purified by silica gel chromatography (eluent (volume ratio): hexane/ethyl acetate = 80/20) to obtain a compound (1-1-5) (5.0 g, 55%).

<Synthesis of compound (1-1-6)>

The compound (1-1-5) (5.0 g, 18.0 mmol), a lanthanum(III) chloride bis(lithium chloride) complex solution 0.6 M in THF (33 mL, 19.8 mmol), and THF (50 mL) were mixed with each other, and an ethylmagnesium bromide solution 1.0 M in THF (26.9 mL, 26.9 mmol) was slowly added dropwise thereto, followed by stirring in an ice bath for 30 minutes. A saturated ammonium chloride aqueous solution (50 mL) was slowly added to the reaction solution, ethyl acetate (75 mL) and 1 N hydrochloric acid (25 mL) were added thereto, and the mixture was stirred at room temperature for 10 minutes. The aqueous layer was removed with a separating funnel, and the obtained organic layer was dried over magnesium sulfate, and then filtered and concentrated under reduced pressure, thereby obtaining a compound (1-1-6) (5.5 g, 99%).

<Synthesis of compound (1-1-7)>

The compound (1-1-6) (5.4 g, 17.5 mmol) and hexane (540 mL) were mixed with each other, and sulfuric acid (4.7 mL, 87.5 mmol) was added thereto, followed by stirring at room temperature for 6 hours. Water (200 mL) was added to the reaction solution, the mixture was stirred at room temperature for 10 minutes, a 1 N sodium hydroxide aqueous solution (200 mL) was added thereto, and the mixture was further stirred at room temperature for 1 hour. The mixture was extracted with dichloromethane using a separating funnel, and the obtained organic layer was dried over magnesium sulfate, filtered, and concentrated under reduced pressure. The obtained crude product was purified by silica gel chromatography (eluent (volume ratio): hexane/ethyl acetate = 98/2) to obtain a compound (1-1-7) (3.1 g, 61%).

<Synthesis of compound (1-1-8)>

The compound (1-1-7) (3.1 g, 10.7 mmol) and DMF (62 mL) were mixed with each other, (chloromethylene) dimethyliminium chloride (5.46 g, 42.7 mmol) was added thereto while stirring at room temperature, and the mixture was stirred at 70°C for 5 hours. The mixture was allowed to cool to room temperature, water (93 mL) was added dropwise thereto while stirring under water cooling, the mixture was stirred at room temperature for 30 minutes, and then the precipitate was collected by filtration. The obtained crude product was purified by silica gel chromatography (eluent (volume ratio): dichloromethane/ethyl acetate = 95/5) to obtain a compound (1-1-8) (2.37 g, 64%).

<Synthesis of compound (1-1)>

The compound (1-1-8) (700 mg, 2.0 mmol), a compound (1-1-9) (780 mg, 5.0 mmol), toluene (60 mL), and piperidine (3.9 μL, 0.02 mmol) were mixed and stirred at 100°C for 4 hours. The precipitated solid was collected by

filtration, and the obtained crude product was purified by column chromatography (eluent (volume ratio): dichloromethane/ethyl acetate = 90/10). Thereafter, the obtained crude product was sublimated and purified to obtain a compound (1-1) (896 mg, 72%). A structure of the compound (1-1) was confirmed by LDI-MS.

LDI-MS (Compound (1-1)): 623.1 (M+)

[Synthesis of compound (1-41)]

A compound (1-41) was synthesized according to the following scheme.

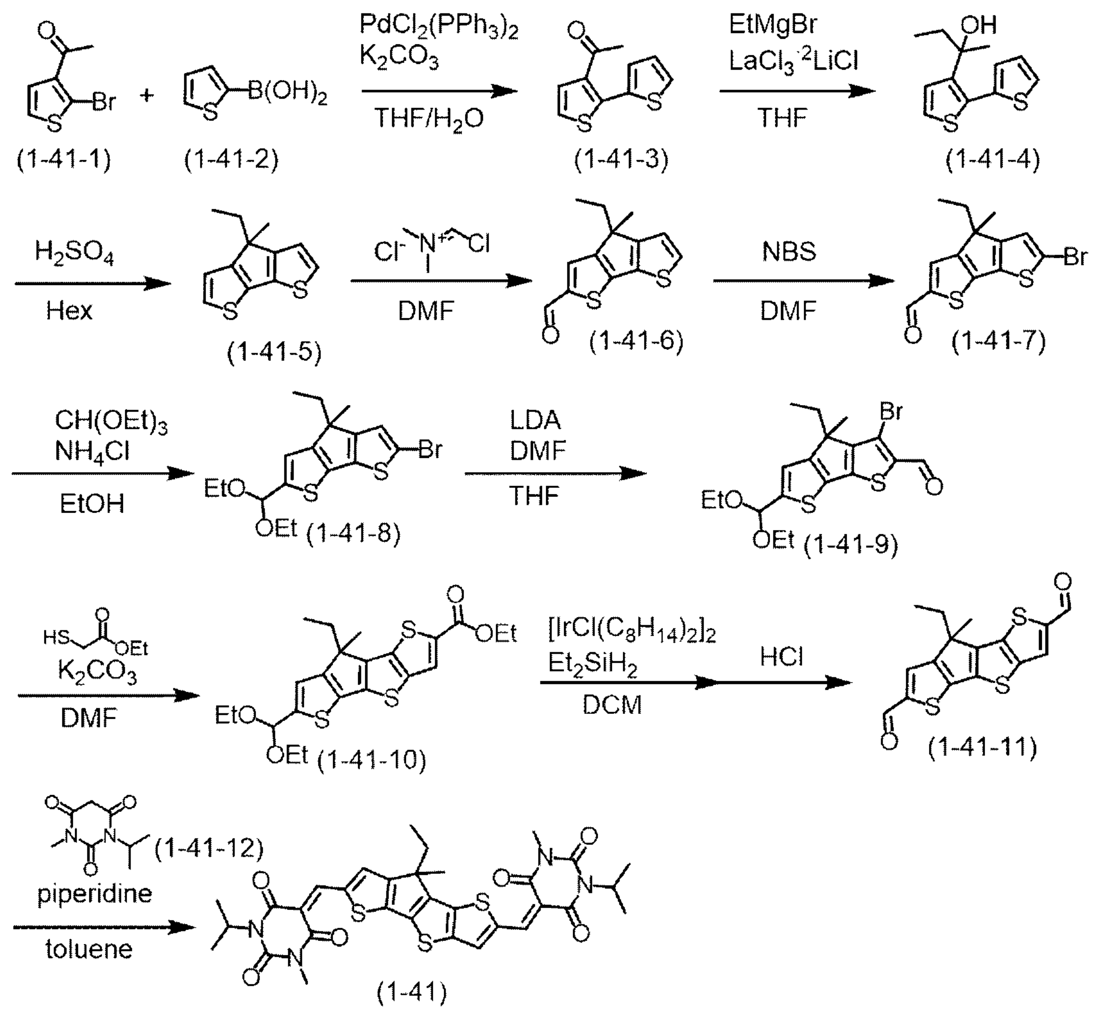


<Synthesis of compound (1-41-3)>

A compound (1-41-1) (5.0 g, 23.6 mmol), a compound (1-41-2) (3.3 g, 26.0 mmol), potassium carbonate (6.54 g, 47.3 mmol), THF (100 mL), and water (20 mL) were mixed with each other, subjected to vacuum degassing, and then bis(triphenylphosphine)palladium(II) dichloride (830 mg, 1.18 mmol) was added thereto, followed by stirring at 70°C for 1 hour. The mixture was allowed to cool to room temperature and diluted with ethyl acetate, the aqueous layer was removed with a separating funnel, and the obtained organic layer was dried over magnesium sulfate, and then filtered and concentrated under reduced pressure. The obtained crude product was purified by silica gel chromatography (eluent (volume ratio): hexane/ethyl acetate = 3/1) to obtain a compound (1-41-3) (3.4 g, 69%).

<Synthesis of compound (1-41-4)>

The compound (1-41-3) (3.4 g, 16.3 mmol), a lanthanum(III) chloride bis(lithium chloride) complex solution 0.6 M in THF (30 mL, 18.0 mmol), and THF (34 mL) were mixed with each other, and an ethylmagnesium bromide solution 1.0 M in THF (34.5 mL, 24.5 mmol) was slowly added dropwise thereto, followed by stirring in an ice bath for 30 minutes. A saturated ammonium chloride aqueous solution (50 mL) was slowly added to the reaction solution, ethyl acetate (75 mL)

and 1 N hydrochloric acid (25 mL) were added thereto, and the mixture was stirred at room temperature for 10 minutes. The aqueous layer was removed with a separating funnel, and the obtained organic layer was dried over magnesium sulfate, and then filtered and concentrated under reduced pressure, thereby obtaining a compound (1-41-4) (3.9 g, 100%).

<Synthesis of compound (1-41-5)>

The compound (1-41-4) (3.9 g, 16.4 mmol) and hexane (390 mL) were mixed with each other, and sulfuric acid (4.4 mL, 81.8 mmol) was added thereto, followed by stirring at room temperature for 6 hours. Water (200 mL) was added to the reaction solution, the mixture was stirred at room temperature for 10 minutes, a 1 N sodium hydroxide aqueous solution (200 mL) was added thereto, and the mixture was further stirred at room temperature for 1 hour. The mixture was extracted with dichloromethane using a separating funnel, and the obtained organic layer was dried over magnesium sulfate, filtered, and concentrated under reduced pressure. The obtained crude product was purified by silica gel chromatography (eluent (volume ratio): hexane/ethyl acetate = 98/2) to obtain a compound (1-41-5) (2.7 g, 75%).

<Synthesis of compound (1-41-6)>

The compound (1-41-5) (2.5 g, 11.3 mmol) and DMF (25 mL) were mixed with each other, (chloromethylene) dimethyliminium chloride (1.53 g, 11.9 mmol) was added thereto while stirring at room temperature, and the mixture was stirred at room temperature for 3 hours. Water (75 mL) was added dropwise thereto while stirring under water cooling, the mixture was stirred at room temperature for 30 minutes, and then the precipitate was collected by filtration, and washed with methanol to obtain a compound (D-41-6) (2.6 g, 93%).

<Synthesis of compound (1-41-7)>

The compound (1-41-6) (2.6 g, 10.5 mmol), NBS (1.96 g, 11.0 mmol), and DMF (52 mL) were mixed and stirred at room temperature for 30 minutes. Water was added to the reaction solution, extraction was carried out with ethyl acetate using a separating funnel, and the obtained organic layer was dried over magnesium sulfate, and then filtered and concentrated under reduced pressure. The obtained crude product was recrystallized from dichloromethane and methanol to obtain (1-41-7) (2.89 g, 88%).

<Synthesis of compound (1-41-8)>

The compound (1-41-7) (2.0 g, 6.11 mmol), triethyl orthoformate (6.10 mL, 36.7 mmol), ammonium chloride (327 mg, 6.11 mmol), and ethanol (40 mL) were mixed and stirred under heating reflux for 2 hours. The mixture was allowed to cool to room temperature, triethylamine (1.28 mL, 9.17 mmol) was added to the reaction solution, and the mixture was stirred at room temperature for 30 minutes. The reaction solution was diluted with ethyl acetate and hexane, washed with water and saturated saline, and the obtained organic layer was dried over magnesium sulfate, filtered, and concentrated under reduced pressure. The obtained crude product was purified by aminosilica gel chromatography (eluent (volume ratio): hexane/ethyl acetate = 99/1) to obtain a compound (1-41-8) (2.3 g, 94%).

<Synthesis of compound (1-41-9)>

The compound (1-41-8) (2.3 g, 5.73 mmol) and THF (46 mL) were mixed with each other, and a lithium isopropylamine solution 1.07 M in hexane/THF (8.03 mL, 8.59 mmol) was slowly added dropwise thereto at -78°C, followed by stirring at -78°C for 1 hour. DMF (1.11 mL, 14.3 mmol) was slowly added dropwise to the reaction solution, and the reaction solution was stirred in an ice bath for 30 minutes. Saturated saline (20 mL) was slowly added to the reaction solution, and then the reaction solution was stirred at room temperature for 15 minutes, extracted with ethyl acetate using a separating funnel, and the obtained organic layer was dried over magnesium sulfate, filtered, and concentrated under reduced pressure. The obtained crude product was purified by silica gel chromatography (eluent (volume ratio): hexane/ethyl acetate/triethylamine = 80/20/2) to obtain a compound (1-41-9) (1.77 g, 72%).

<Synthesis of compound (1-41-10)>

The compound (1-41-9) (1.77 g, 4.12 mmol), ethyl mercaptoacetate (0.68 mL, 6.18 mmol), potassium carbonate (1.71 g, 12.4 mmol), and DMF (41 mL) were mixed and stirred at 40°C for 4 hours. The reaction solution was allowed to cool to room temperature, water (40 mL) was added to the reaction solution, ethyl acetate (20 mL) and hexane (20 mL) were added thereto, and the mixture was stirred at room temperature for 10 minutes. The aqueous layer was removed with a separating funnel, and the obtained organic layer was dried over magnesium sulfate, and then filtered and concentrated

under reduced pressure. The obtained crude product was purified by aminosilica gel chromatography (eluent (volume ratio): hexane/ethyl acetate = 90/10) to obtain a compound (1-41-10) (1.8 g, 97%).

<Synthesis of compound (1-41-11)>

The compound (1-41-10) (1.7 g, 3.77 mmol), diethylsilane (0.73 mL, 5.66 mmol), di-$\mu$-chlorobis(cyclooctene) iridium (I) (16.9 mg, 0.019 mmol), and dichloromethane (7.6 mL) were mixed and stirred under heating reflux for 4 hours. The reaction solution was allowed to cool to room temperature, diluted with dichloromethane, 1 N hydrochloric acid (7.6 mL) was added thereto, and the mixture was stirred at room temperature for 30 minutes. The aqueous layer was removed with a separating funnel, and the obtained organic layer was dried over magnesium sulfate, and then filtered and concentrated under reduced pressure. The obtained crude product was purified by silica gel chromatography (eluent (volume ratio): dichloromethane/ethyl acetate = 98/2) to obtain a compound (1-41-10) (640 mg, 51%).

<Synthesis of compound (1-41)>

The compound (1-41-11) (450 mg, 1.35 mmol), a compound (1-41-12) (598 mg, 3.25 mmol), piperidine (26.8 $\mu$L, 0.27 mmol), and toluene (23 mL) were mixed and stirred at 100°C for 2 hours. The mixture was allowed to cool to room temperature and concentrated under reduced pressure. The obtained crude product was recrystallized and purified with dichloromethane and methanol. Thereafter, the obtained crude product was sublimated and purified to obtain a compound (1-41) (666 mg, 74%).

The obtained compound (1-41) was identified by nuclear magnetic resonance (NMR).

Compound (1-41): $^1$H NMR (400 MHz, $CDCl_3$); $\delta$ = 8.73 (0.5H, s), 8.73 (0.5H, s), 8.71 (0.2H, s), 8.70 (0.8H, s), 8.09 (0.5H, s), 8.07 (0.5H, s), 7.73 (0.2H, s), 7.72 (0.8H, s) ), 5.17 to 5.33 (2H, m), 3.40 to 3.45 (6H, m), 2.06 to 2.23 (2H, m), 1.65 (1.5H, s), 1.64 (1.5H, s), 1.49 to 1.54 (12H, m), 0.65 (3H, t)

Specific compounds used for a photoelectric conversion film, other than the compound (1-1) and the compound (1-41), were synthesized according to the synthesis method of the compound (1-1) and compound (1-41) described above.

[Specific compound]

The specific compounds used for the photoelectric conversion film and comparative compounds of Comparative Examples are shown below.

All of the compounds (1-1) to (1-50) correspond to the specific compounds according to the present invention, and the compounds (C-1) to (C-4) are comparative compounds of Comparative Examples. In the formulae, a group represented by "TMS" represents a trimethylsilyl group.

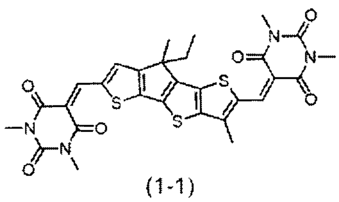
(1-1)

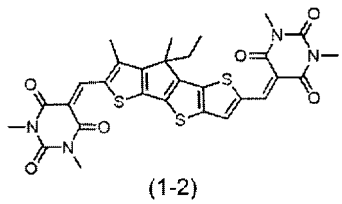
(1-2)

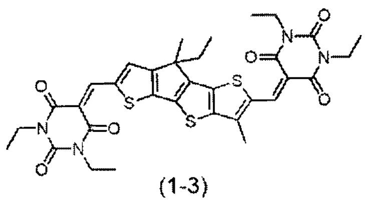
(1-3)

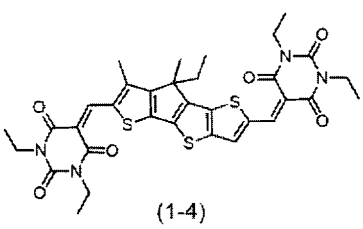
(1-4)

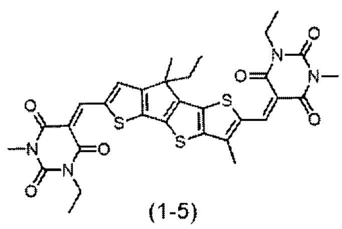
(1-5)

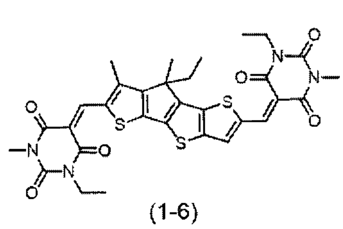
(1-6)

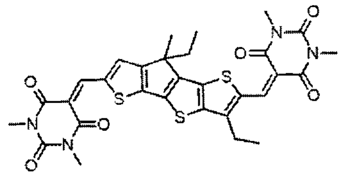

(1-7)

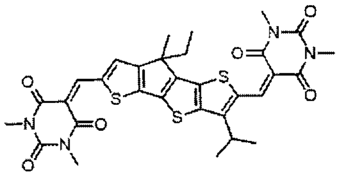

(1-8)

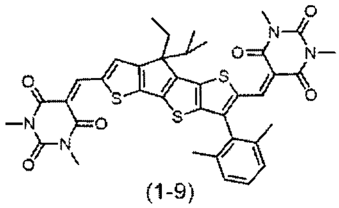

(1-9)

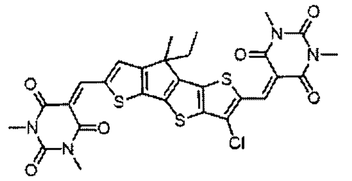

(1-10)

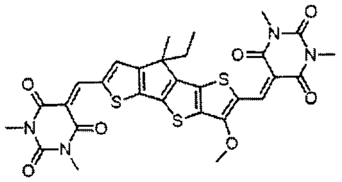

(1-11)

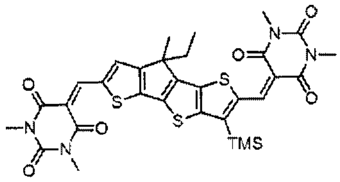

(1-12)

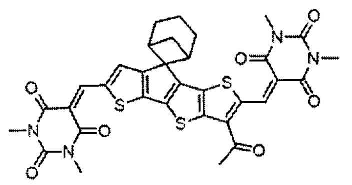

(1-13)

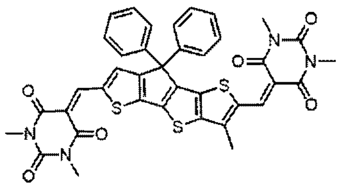

(1-14)

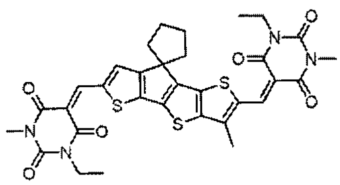

(1-15)

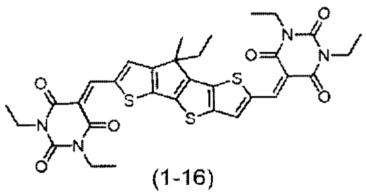

(1-16)

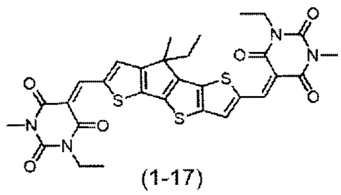

(1-17)

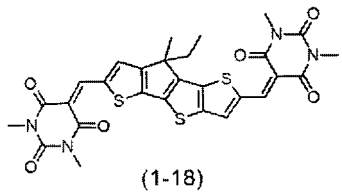

(1-18)

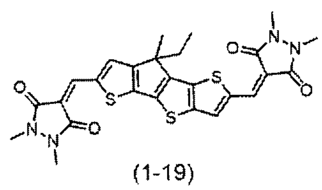

(1-19)

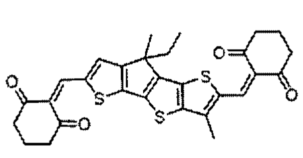

(1-20)

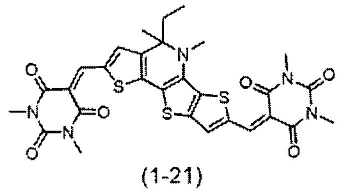

(1-21)

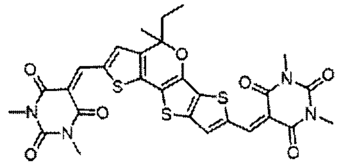

(1-22)

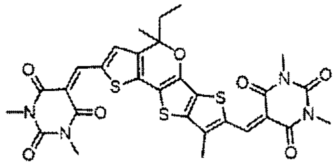

(1-23)

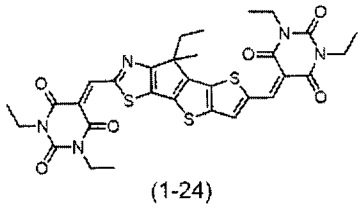

(1-24)

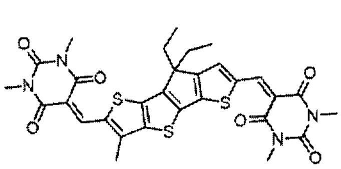

(1-25)

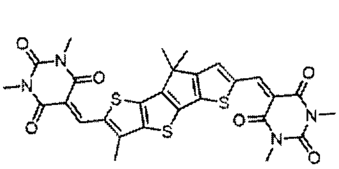

(1-26)

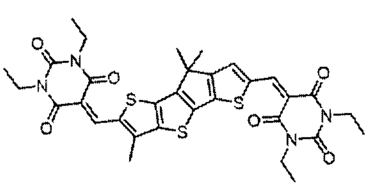

(1-27)

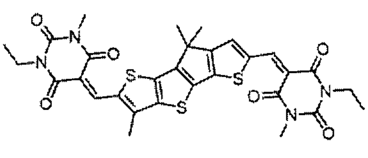
(1-28)

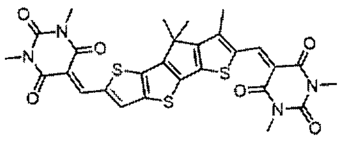
(1-29)

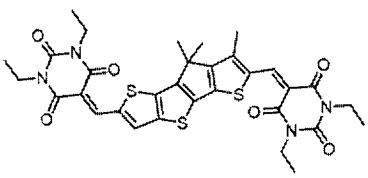
(1-30)

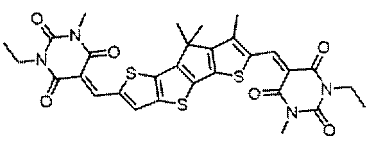
(1-31)

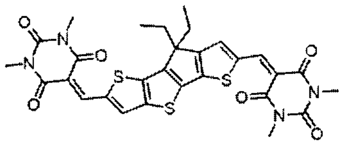
(1-32)

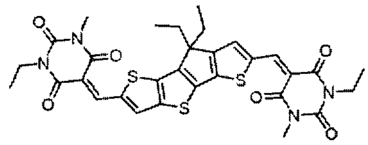
(1-33)

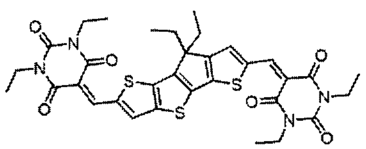
(1-34)

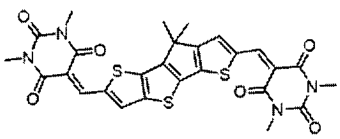
(1-35)

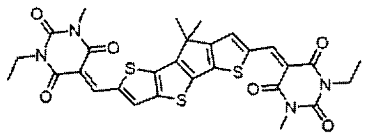
(1-36)

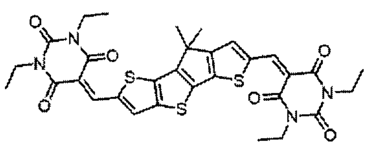
(1-37)

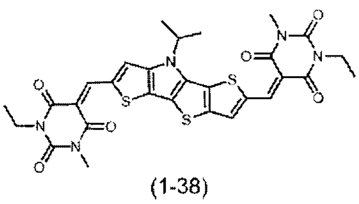
(1-38)

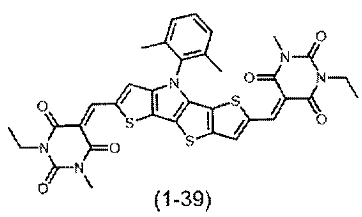
(1-39)

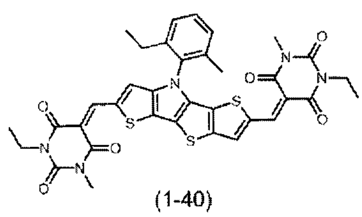
(1-40)

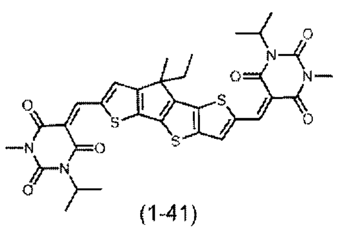
(1-41)

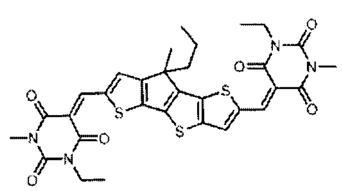
(1-42)

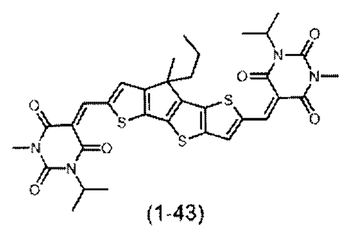
(1-43)

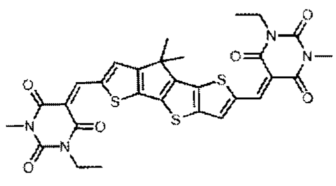

(1-44)

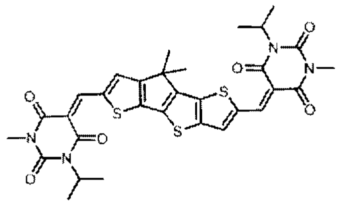

(1-45)

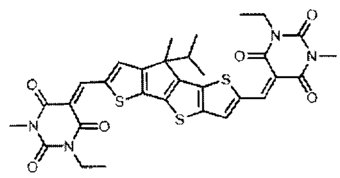

(1-46)

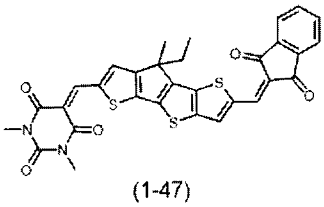

(1-47)

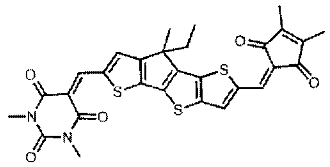

(1-48)

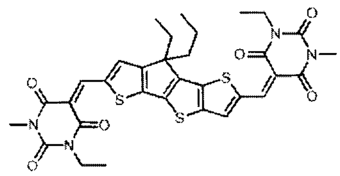

(1-49)

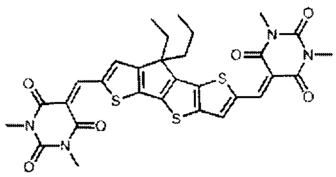

(1-50)

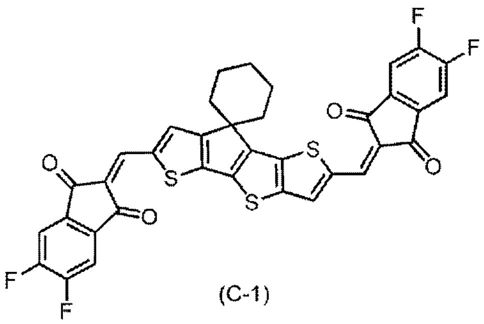

(C-1)

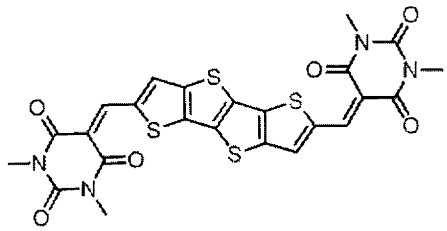

(C-2)

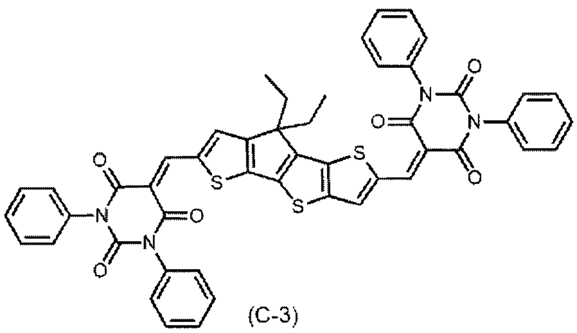

(C-3)

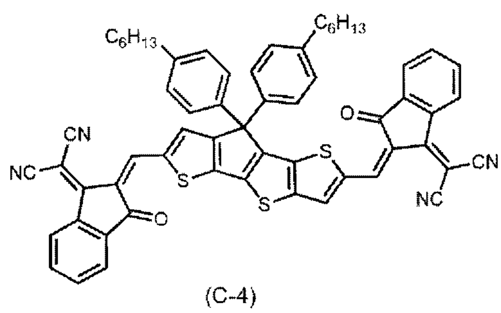

(C-4)

[n-type organic semiconductor]

- C60: fullerene ($C_{60}$)

[p-type organic semiconductor]

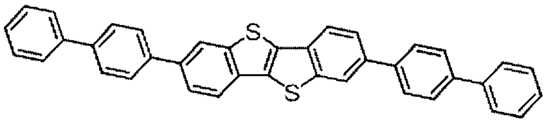

(P-1)

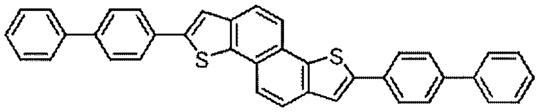

(P-2)

[Coloring agent]

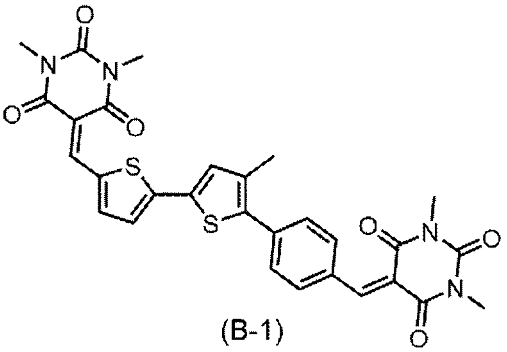

(B-1)

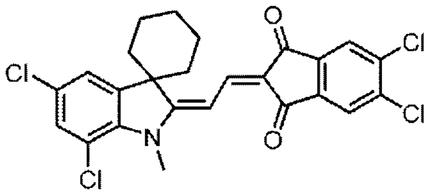

(B-2)

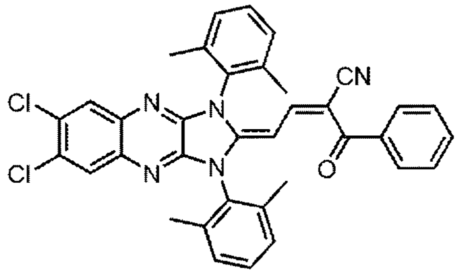

(B-3)

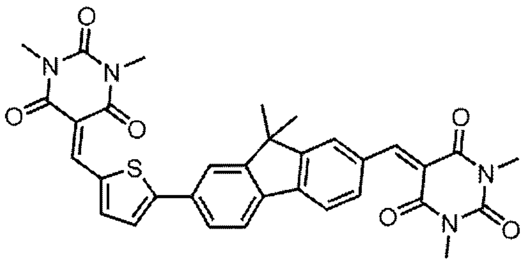

(B-4)

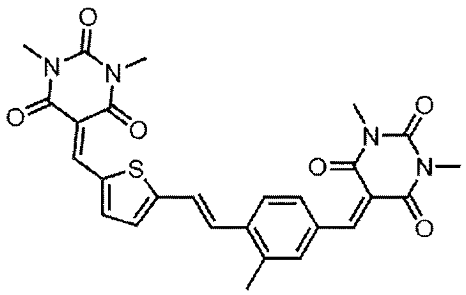

(B-5)

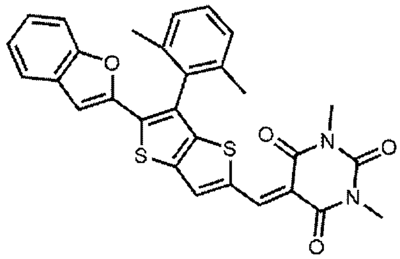

(B-6)

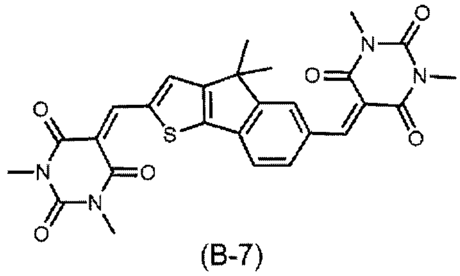

(B-7)

[Evaluation]

[Test X]

The quantum efficiency, the response speed, the electric field strength dependence of the response speed, and the manufacturing suitability in a case where the photoelectric conversion element received red and green light (600 nm) were evaluated by the following methods.

<Production of photoelectric conversion element (A)>

A photoelectric conversion element (A) of the form shown in Fig. 2 was produced using the obtained compound. Here, the photoelectric conversion element included a lower electrode 11, an electron blocking film 16A, a photoelectric conversion film 12, a hole blocking film 16B, and an upper electrode 15.

Specifically, an amorphous ITO was formed into a film on a glass substrate by a sputtering method to form the lower electrode 11 (thickness: 30 nm), and the compound (EB-1) was further formed into a film on the lower electrode 11 by a vacuum thermal vapor deposition method to form the electron blocking film 16A (thickness: 30 nm).

Furthermore, in a state in which the temperature of the glass substrate was controlled to 25°C, each specific compound or each comparative compound shown in Table 1, an n-type organic semiconductor (fullerene ($C_{60}$)), and a p-type organic semiconductor shown in Table 1 were subjected to co-vapor deposition by a vacuum vapor deposition method at a predetermined ratio (specific compound:n-type organic semiconductor:p-type organic semiconductor = 1:1:1; in terms of thickness) to form a film on the electron blocking film 16A. As a result, the photoelectric conversion film 12 having a bulk hetero structure with 300 nm was formed. In this case, a film formation rate of the photoelectric conversion film 12 was set to 1.0 Å/sec.

Furthermore, the compound (EB-2) was vapor-deposited on the photoelectric conversion film 12 to form the hole blocking film 16B (thickness: 10 nm). Amorphous ITO was formed into a film on the hole blocking film 16B by a sputtering method to form the upper electrode 15 (transparent conductive film) (film thickness: 10 nm). After the SiO film was formed as a sealing layer on the upper electrode 15 by a vacuum vapor deposition method, an aluminum oxide ($Al_2O_3$) layer was formed thereon by an atomic layer chemical vapor deposition (ALCVD) method to produce each photoelectric conversion element (A).

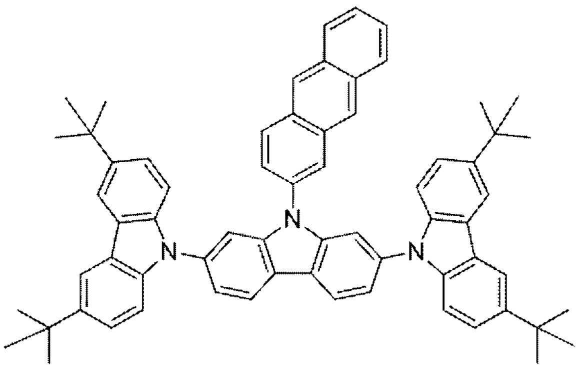

EB-1

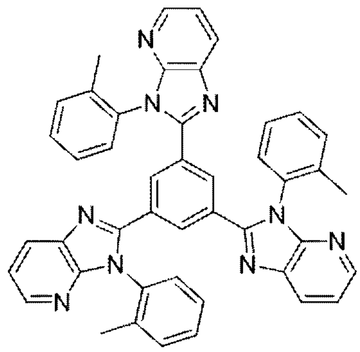

EB-2

<Dark current>

A dark current of each obtained photoelectric conversion element (A) was measured by the following method.

A voltage was applied to the lower electrode and the upper electrode of each of the photoelectric conversion elements (A) with an electric field strength of $2.5 \times 10^5$ V/cm, and a current value (dark current) in a dark place was measured. As a result, it was confirmed that all of the photoelectric conversion elements (A) had a dark current of 50 $nA/cm^2$ or less, which indicates that all of the photoelectric conversion elements had a sufficiently low dark current.

<Quantum efficiency (external quantum efficiency)>

Quantum efficiency (external quantum efficiency) of each photoelectric conversion element (A) was evaluated by the following method.

A voltage was applied to each photoelectric conversion element (A) with an electric field strength of $2.0 \times 10^5$

V/cm. Thereafter, light was emitted from an upper electrode (transparent conductive film) side, and the quantum efficiency (photoelectric conversion efficiency) at a wavelength of 600 nm was measured.
Using the obtained quantum efficiency of each photoelectric conversion element (A) at a wavelength of 600 nm, a relative ratio of the quantum efficiency at each wavelength was calculated according to Expression (S1). From the obtained values, the quantum efficiency was evaluated according to the following evaluation standard.

Expression (S1): Relative ratio of quantum efficiency = (Photoelectric conversion efficiency of each photoelectric conversion element (A))/(Photoelectric conversion efficiency of photoelectric conversion element (A) of Example 1-1) Expression (S1):

A: relative ratio of the quantum efficiency was 0.95 or more.
B: relative ratio of the quantum efficiency was 0.80 or more and less than 0.95.
C: relative ratio of the quantum efficiency was 0.60 or more and less than 0.80.
D: relative ratio of quantum efficiency was less than 0.60.

<Response speed>

A response speed of each photoelectric conversion element (A) was evaluated by the following method.
A voltage was applied to each photoelectric conversion element (A) with a strength of $2.0 \times 10^5$ V/cm. Thereafter, a light emitting diode (LED) was turned on for an instant to emit light from the upper electrode (transparent conductive film) side, a photocurrent at a wavelength of 600 nm was measured with an oscilloscope, and a rise time until the signal intensity rose from 0% to 97% was measured. Using the obtained rise time of each photoelectric conversion element (A) at a wavelength of 600 nm, a relative response speed was calculated according to Expression (S2). From the obtained values, the response speed was evaluated according to the following evaluation standard.

Expression (S2): Relative response speed = (Rise time of each photoelectric conversion element (A))/(Rise time of photoelectric conversion element (A) of Example 1-1) Expression (S2):

A: relative response speed was less than 1.1.
B: relative response speed was 1.1 or more and less than 1.5.
C: relative response speed was 1.5 or more and less than 2.0.
D: relative response speed was 2.0 or more.

<Electric field strength dependence of response speed>

For each photoelectric conversion element (A), electric field strength dependence of the response speed was evaluated by the following method.
A rise time at an applied voltage of $7.5 \times 10^4$ V/cm was measured by the same procedure as in <Response speed>, except that the voltage applied to each photoelectric conversion element (A) was changed to $7.5 \times 10^4$ V/cm.
Using the rise time of each photoelectric conversion element (A) at a wavelength of 600 nm at each applied voltage, a relative ratio of the rise time was calculated according to Expression (S3). From the obtained values, electric field strength dependence of the response speed was evaluated according to the following evaluation standard.

Expression (S3): Relative ratio of rise time = (Rise time of each photoelectric conversion element (A) at applied voltage of $7.5 \times 10^4$ V/cm)/(Rise time of each photoelectric conversion element (A) at applied voltage of $2.0 \times 10^5$ V/cm) Expression (S3):

A: relative ratio of the rise time was less than 2.0.
B: relative ratio of the rise time was 2.0 or more and less than 3.0.
C: relative ratio of the rise time was 3.0 or more and less than 4.0.
D: relative ratio of the rise time was 4.0 or more.

<Evaluation of manufacturing suitability>

Manufacturing suitability of the photoelectric conversion element having the configuration of each of Examples and Comparative Examples was evaluated by the following method.
A photoelectric conversion element (B) of each of Examples or Comparative Examples was produced by the same procedure as that of the photoelectric conversion element (A), except that the film formation rate of the photoelectric conversion film 12 was set to 3.0 Å/sec. Next, a photoelectric conversion efficiency of the obtained photoelectric conversion element (B) at 600 nm was measured by the same method as in <Evaluation of quantum efficiency (external quantum efficiency)>.
Using the measured values of the photoelectric conversion efficiency at 600 nm of the photoelectric conversion element (A) having the same configuration in Examples or Comparative Examples and the photoelectric conversion element (B), a relative ratio B/A of the photoelectric conversion efficiency was calculated according to Expression (S4). From the obtained values, the manufacturing suitability was evaluated according to the following evaluation standard.
As the value of the relative ratio B/A was closer to 1, the characteristics of the photoelectric conversion element were less likely to be deteriorated in a case where the film formation rate was increased, that is, the manufacturing suitability was more excellent.

Expression (S4): Relative ratio B/A of photoelectric conversion efficiency = (Photoelectric conversion efficiency of photoelectric conversion element (B))/(Photoelectric conversion efficiency of photoelectric conversion element (A)) Expression (S4):

A: relative ratio B/A was 0.90 or more.
B: relative ratio B/A was 0.85 or more and less than 0.90.
C: relative ratio B/A was 0.80 or more and less than 0.85.
D: relative ratio B/A was less than 0.80.

<Electric field strength dependence of quantum efficiency>

For the photoelectric conversion element having the configuration of each of Examples and Comparative Examples, the electric field strength dependence of the quantum efficiency in a case of receiving red and green light (600 nm) was evaluated by the following method.
The quantum efficiency (external quantum efficiency) at an electric field strength of $7.0 \times 10^4$ V/cm was measured for each photoelectric conversion element (A) by the same procedure as in the evaluation of <Quantum efficiency> described above.
The electric field strength dependence of the quantum efficiency was calculated according to Expression (SX1), and the electric field strength dependence of the quantum efficiency was evaluated according to the following evaluation standard. In Expression (SX1), the numerator and the denominator are values measured for the same photoelectric conversion element of Examples of Comparative Examples. In addition, the numerator and the denominator are both the quantum efficiency measured at 600 nm. It is preferable that the electric field strength dependence of the quantum efficiency was evaluated as C or more.

Expression (SX1): Electric field strength dependence of quantum efficiency = (Quantum efficiency of each photoelectric conversion element (A) at electric field strength of $7.0 \times 10^4$ V/cm)/(Quantum efficiency of each photoelectric conversion element (A) at electric field strength of $2.0 \times 10^5$ V/cm) Expression (SX1):

A: electric field strength dependence of the quantum efficiency was 0.80 or more.
B: electric field strength dependence of the quantum efficiency was 0.75 or more and less than 0.80.
C: electric field strength dependence of the quantum efficiency was 0.70 or more and less than 0.75.
D: electric field strength dependence of the quantum efficiency was less than 0.70.

<Result>

The evaluation results are shown in the tables below.
In the column of "$X^3$" of the tables, a case where the specific compound was a compound in which $X^3$ in Formula

(1) was -C($R^{c1}R^{c2}$)- is indicated as "A", and a case other than the case is indicated as "B".

In the column of "$A^1$, $A^2$" of the tables, a case where the specific compound was a compound in which $A^1$ and $A^2$ in Formula (1) were the group represented by Formula (A-2) is indicated as "A", and a case other than the case is indicated as "B".

In the column of "$R^{N2} \neq R^{N3}$" of the tables, a case where $R^{N2}$ and $R^{N3}$ were groups different from each other is indicated as "A", and a case other than the case is indicated as "B".

In the column of "$X^1$, $X^2$ (-$CR^{a1}$=)" of the tables, a case where the specific compound was a compound in which $X^1$ and $X^2$ in Formula (1) were -$CR^{a1}$= is indicated as "A", and a case other than the case is indicated as "B".

In the column of "Requirements 1 to 4" of the tables, a case where the specific compound was a compound satisfying any one of the requirements 1 to 4 is indicated as "A", and a case other than the case is indicated as "B".

In the column of "Requirements 4 to 7" of the tables, a case where the specific compound was a compound satisfying any one of the requirements 4 to 7 is indicated as "A", and a case other than the case is indicated as "B".

In the column of "$X^1$, $X^2$ (-$CR^{a2}$=)" of the tables, a case where the specific compound was a compound in which at least one of $X^1$ or $X^2$ in Formula (1) was -$CR^{a2}$= is indicated as "A", and a case other than the case is indicated as "B".

[Table 1]

| | Compound | $X^3$ | $A^1$, $A^2$ | $R^{N2} \neq R^{N3}$ | $X^1$, $X^2$ (-$CR^{a1}$=) | Requirements 1 to 4 | Requirements 4 to 7 | $X^1$, $X^2$ (-$CR^{a2}$=) | p-type organic semiconductor |
|---|---|---|---|---|---|---|---|---|---|
| Example 1-1 | 1-1 | A | A | B | A | A | A | A | P-1 |
| Example 1-2 | 1-1 | A | A | B | A | A | A | A | P-2 |
| Example 1-3 | 1-2 | A | A | B | A | A | A | A | P-1 |
| Example 1-4 | 1-3 | A | A | B | A | A | A | A | P-1 |
| Example 1-5 | 1-4 | A | A | B | A | A | A | A | P-1 |
| Example 1-6 | 1-5 | A | A | A | A | A | A | A | P-1 |
| Example 1-7 | 1-6 | A | A | A | A | A | A | A | P-1 |
| Example 1-8 | 1-7 | A | A | B | A | A | A | A | P-1 |
| Example 1-9 | 1-8 | A | A | B | A | A | A | A | P-1 |
| Example 1-10 | 1-9 | A | A | B | A | A | A | A | P-1 |
| Example 1-11 | 1-10 | A | A | B | A | A | A | A | P-1 |
| Example 1-12 | 1-11 | A | A | B | A | A | A | A | P-1 |
| Example 1-13 | 1-12 | A | A | B | A | A | A | A | P-1 |
| Example 1-14 | 1-13 | A | A | B | A | A | B | A | P-1 |
| Example 1-15 | 1-14 | A | A | B | A | B | B | A | P-1 |
| Example 1-16 | 1-15 | A | A | A | A | B | B | A | P-1 |

(continued)

| | Compound | $X^3$ | $A^1$, $A^2$ | $R^{N2} \neq R^{N3}$ | $X^1$, $X^2$ (-$CR^{a1}$=) | Requirements 1 to 4 | Requirements 4 to 7 | $X^1$, $X^2$ (-$CR^{a2}$=) | p-type organic semiconductor |
|---|---|---|---|---|---|---|---|---|---|
| Example 1-17 | 1-16 | A | A | B | A | A | A | B | P-1 |
| Example 1-18 | 1-17 | A | A | A | A | A | A | B | P-1 |
| Example 1-19 | 1-18 | A | A | B | A | A | A | B | P-1 |
| Example 1-20 | 1-19 | A | B | B | A | A | A | B | P-1 |
| Example 1-21 | 1-20 | A | B | B | A | A | A | A | P-1 |
| Example 1-22 | 1-21 | B | A | B | A | A | A | B | P-1 |
| Example 1-23 | 1-22 | B | A | B | A | A | A | B | P-1 |
| Example 1-24 | 1-23 | B | A | B | A | A | A | A | P-1 |
| Example 1-25 | 1-24 | A | A | B | B | A | A | B | P-1 |
| Example 1-26 | 1-25 | A | A | B | A | B | B | A | P-1 |
| Example 1-27 | 1-26 | A | A | B | A | B | B | A | P-1 |
| Example 1-28 | 1-27 | A | A | B | A | B | B | A | P-1 |
| Example 1-29 | 1-28 | A | A | A | A | B | B | A | P-1 |
| Example 1-30 | 1-29 | A | A | B | A | B | B | A | P-1 |
| Example 1-31 | 1-30 | A | A | B | A | B | B | A | P-1 |
| Example 1-32 | 1-31 | A | A | A | A | B | B | A | P-1 |
| Example 1-33 | 1-32 | A | A | B | A | B | B | B | P-1 |
| Example 1-34 | 1-33 | A | A | A | A | B | B | B | P-1 |
| Example 1-35 | 1-34 | A | A | B | A | B | B | B | P-1 |
| Example 1-36 | 1-35 | A | A | B | A | B | B | B | P-1 |
| Example 1-37 | 1-36 | A | A | A | A | B | B | B | P-1 |
| Example 1-38 | 1-37 | B | A | B | A | B | B | B | P-1 |

[Table 2]

| | Quantum efficiency | Response speed | Electric field strength dependence of response speed | Manufacturing suitability | Electric field strength dependence of quantum efficiency |
|---|---|---|---|---|---|
| Example 1-1 | A | A | A | A | B |
| Example 1-2 | A | B | B | A | B |
| Example 1-3 | A | A | A | A | B |
| Example 1-4 | A | A | A | A | B |
| Example 1-5 | A | A | A | A | B |
| Example 1-6 | A | A | A | A | A |
| Example 1-7 | A | A | A | A | A |
| Example 1-8 | A | A | A | A | B |
| Example 1-9 | A | A | A | A | B |
| Example 1-10 | A | A | A | A | B |
| Example 1-11 | A | A | A | A | B |
| Example 1-12 | A | A | A | A | B |
| Example 1-13 | A | A | A | A | B |
| Example 1-14 | A | A | A | A | C |
| Example 1-15 | B | A | A | A | C |
| Example 1-16 | B | A | A | A | B |
| Example 1-17 | A | B | B | B | B |
| Example 1-18 | A | B | B | B | A |
| Example 1-19 | A | B | B | B | B |
| Example 1-20 | B | B | B | B | B |
| Example 1-21 | B | A | A | A | B |
| Example 1-22 | B | B | B | C | B |
| Example 1-23 | B | B | B | C | B |
| Example 1-24 | A | B | B | B | B |
| Example 1-25 | B | B | B | B | B |
| Example 1-26 | B | A | A | A | C |
| Example 1-27 | B | A | A | A | C |
| Example 1-28 | B | A | A | A | C |
| Example 1-29 | B | A | A | A | B |
| Example 1-30 | B | A | A | A | C |
| Example 1-31 | B | A | A | A | B |
| Example 1-32 | B | A | A | A | A |
| Example 1-33 | B | B | B | B | B |
| Example 1-34 | B | B | B | B | B |
| Example 1-35 | B | B | B | B | C |
| Example 1-36 | B | B | B | B | C |
| Example 1-37 | B | B | B | B | B |

(continued)

| | Quantum efficiency | Response speed | Electric field strength dependence of response speed | Manufacturing suitability | Electric field strength dependence of quantum efficiency |
|---|---|---|---|---|---|
| Example 1-38 | B | B | B | B | C |

[Table 3]

| | Compound | $X^3$ | $A^1$, $A^2$ | $R^{N2} \neq R^{N3}$ | $X^1$, $X^2$ (-$CR^{a1}$=) | Requirements 1 to 4 | Requirements 4 to 7 | $X^1$, $X^2$ (-$CR^{a2}$=) | p-type organic semiconductor |
|---|---|---|---|---|---|---|---|---|---|
| Example 1-39 | 1-38 | B | A | A | A | B | B | B | P-1 |
| Example 1-40 | 1-39 | B | A | A | A | B | B | B | P-1 |
| Example 1-41 | 1-40 | B | A | A | A | A | B | B | P-1 |
| Example 1-42 | 1-41 | A | A | A | A | A | A | B | P-1 |
| Example 1-43 | 1-42 | A | A | A | A | A | A | B | P-1 |
| Example 1-44 | 1-43 | A | A | A | A | A | A | B | P-1 |
| Example 1-45 | 1-44 | A | A | A | A | B | B | B | P-1 |
| Example 1-46 | 1-45 | A | A | A | A | B | B | B | P-1 |
| Example 1-47 | 1-46 | A | A | A | A | A | A | B | P-1 |
| Example 1-48 | 1-47 | A | A | B | A | A | A | B | P-1 |
| Example 1-49 | 1-48 | A | A | B | A | A | A | B | P-1 |
| Example 1-50 | 1-49 | A | A | A | A | A | A | B | P-1 |
| Example 1-51 | 1-50 | A | A | B | A | A | A | B | P-1 |
| Comparative Example 1-1 | C-1 | - | - | - | - | - | - | - | P-1 |
| Comparative Example 1-2 | C-2 | - | - | - | - | - | - | - | P-1 |
| Comparative Example 1-3 | C-3 | - | - | - | - | - | - | - | P-1 |
| Comparative Example 1-4 | C-4 | - | - | - | - | - | - | - | P-1 |

[Table 4]

| | Quantum efficiency | Response speed | Electric field strength dependence of response speed | Manufacturing suitability | Electric field strength dependence of quantum efficiency |
|---|---|---|---|---|---|
| Example 1-39 | A | B | B | C | B |
| Example 1-40 | A | B | B | C | B |
| Example 1-41 | A | B | B | C | A |
| Example 1-42 | A | B | B | B | A |
| Example 1-43 | A | B | B | B | A |
| Example 1-44 | A | B | B | B | A |
| Example 1-45 | B | B | B | B | B |
| Example 1-46 | B | B | B | B | B |
| Example 1-47 | A | B | B | B | B |
| Example 1-48 | A | B | B | B | B |
| Example 1-49 | A | B | B | B | B |
| Example 1-50 | A | B | B | B | A |
| Example 1-51 | A | B | B | B | B |
| Comparative Example 1-1 | D | C | C | D | D |
| Comparative Example 1-2 | D | D | D | C | D |
| Comparative Example 1-3 | D | D | D | D | D |
| Comparative Example 1-4 | D | D | D | D | D |

From the results shown in Table 1, it was found that the photoelectric conversion element according to the embodiment of the invention was excellent in quantum efficiency in a case of receiving red and green light. In addition, it was found that the photoelectric conversion element according to the embodiment of the present invention was also excellent in response speed in a case of receiving red and green light, electric field strength dependence of the response speed, and manufacturing suitability.

From the comparison between Example 1-24 and Examples 1-3 to 1-14, it was found that, in a case where $X^3$ in Formula (1) was $-C(R^{c1}R^{c2})-$, the response speed and the electric field strength dependence of the response speed in a case of receiving red and green light, and the manufacturing suitability were more excellent.

From the comparison between Example 1-21 and Examples 1-3 to 1-14, and the comparison between Example 1-20 and Examples 1-17 to 1-19, it was found that, in a case where $A^1$ and $A^2$ in Formula (1) were the group represented by Formula (A-2), the quantum efficiency in a case of receiving red and green light was more excellent.

From the comparison between Example 1-25 and Examples 1-17 to 1-19, it was found that, in a case where $X^1$ and $X^3$ were $-CR^{a1}=$, the quantum efficiency in a case of receiving red and green light was more excellent.

From the comparison between Examples 1-15 and 1-16, and Examples 1-26 to 1-32, and Examples 1-3 to 1-14, it was found that, in a case where the specific compound satisfied any one of the requirements 1 to 3, the quantum efficiency in a case of receiving red and green light was more excellent.

From the comparison between Examples 1-17 to 1-19 and Examples 1-3 to 1-14, and the comparison between Examples 1-33 to 1-38 and Examples 1-26 to 1-32, it was found that, in a case where at least one of $X^1$ or $X^2$ in Formula (1) was $-CR^{a2}=$, the response speed and the electric field strength dependence of the response speed in a case of receiving red and green light, and the manufacturing suitability were more excellent.

From the comparison between Examples 1-3 to 1-5, Examples 1-8 to 1-13, and Examples 1-6 and 1-7, it was found that, in a case where $R^{N2}$ and $R^{N3}$ were groups different from each other, the electric field strength dependence of the quantum efficiency was smaller.

From the comparison between Examples 1-39 and 1-40 and Example 1-41, it was found that, in a case where $X^3$ represented $-NR^{N11}-$, and $R^{N11}$ was the group represented by Formula (C-1) or the group represented by Formula (C-2), the electric field strength dependence of the quantum efficiency was smaller.

From the comparison between Example 1-14 and Example 1-15, it was found that, in a case where the specific compound satisfied any one of the requirements 4 to 7, the quantum efficiency was more excellent.

[Test Y]

The quantum efficiency, the response speed, the electric field strength dependence of the response speed, and the electric field strength dependence of the quantum efficiency of the photoelectric conversion element containing a coloring agent in a case of receiving red and green light (600 nm) or blue light (460 nm) were evaluated by the following methods.

<Production of photoelectric conversion element (C)>

A photoelectric conversion element (C) of each of Examples and Comparative Examples was produced by the same procedure as in <Production of photoelectric conversion element (A)> in [Test X], except that each specific compound or each comparative compound shown in Table 2, C60 as the n-type organic semiconductor, the p-type organic semiconductor shown in Table 2, and a coloring agent shown in Table 2 were subjected to co-vapor deposition by a vacuum vapor deposition method at a predetermined ratio (specific compound:coloring agent:p-type organic semiconductor:n-type organic semiconductor = 1:1:2:2; in terms of thickness) to form a film, thereby forming the photoelectric conversion film 12 having a thickness of 300 nm.

<Dark current>

The dark current of the obtained photoelectric conversion element (C) was measured according to the same procedure as in <Measurement of dark current> in [Test X].

As a result, it was confirmed that all of the photoelectric conversion elements (C) had a dark current of 50 $nA/cm^2$ or less, which indicates that all of the photoelectric conversion elements had a sufficiently low dark current.

<Quantum efficiency (external quantum efficiency)>

Quantum efficiency (external quantum efficiency) of each photoelectric conversion element (C) was evaluated by the following method.

A voltage was applied to each photoelectric conversion element (C) with an electric field strength of $2.0 \times 10^5$ V/cm. Thereafter, light was emitted from an upper electrode (transparent conductive film) side, and the quantum efficiency (external quantum efficiency) at a wavelength of 460 nm or at a wavelength of 600 nm was measured.

Using the obtained quantum efficiency of each photoelectric conversion element (C) at a wavelength of 460 nm or at a wavelength of 600 nm, a relative ratio of the quantum efficiency at each wavelength was calculated according to Expression (S5). From the obtained values, the quantum efficiency was evaluated according to the following evaluation standard.

Using the photoelectric conversion efficiency of each photoelectric conversion element (C), a relative ratio of the quantum efficiency at each wavelength was calculated according to Expression (S5). From the obtained values, the quantum efficiency was evaluated according to the following evaluation standard.

Expression (S5): Relative ratio of quantum efficiency = (Photoelectric conversion efficiency of each photoelectric conversion element (C))/(Photoelectric conversion efficiency of photoelectric conversion element (C) of Example 2-1)    Expression (S5):

In a case of obtaining the relative ratio of the quantum efficiency, the values of the photoelectric conversion efficiency at the same wavelength were used as the numerator and the denominator.

A: relative ratio of the quantum efficiency was 0.95 or more.
B: relative ratio of the quantum efficiency was 0.80 or more and less than 0.95.
C: relative ratio of the quantum efficiency was 0.60 or more and less than 0.80.
D: relative ratio of quantum efficiency was less than 0.60.

<Response speed>

A response speed of each photoelectric conversion element (C) was evaluated by the following method.

A voltage was applied to each photoelectric conversion element (C) with a strength of $2.0 \times 10^5$ V/cm. Thereafter, LED was turned on for an instant to emit light from the upper electrode (transparent conductive film) side, a photocurrent at a wavelength of 460 nm or at a wavelength of 600 nm was measured with an oscilloscope, and a rise time until the signal intensity rose from 0% to 97% was measured. Using the obtained rise time of each photoelectric conversion element (C) at each wavelength, a relative response speed at each wavelength was calculated according to Expression (S6). From the obtained values, the response speed was evaluated according to the following evaluation standard.

Expression (S6): Relative response speed = (Rise time of each photoelectric conversion element (C))/(Rise time of photoelectric conversion element (C) of Example 2-1) Expression (S6):

In a case of obtaining the relative response speed, the rise times at the same wavelength were used as the numerator and the denominator.

A: relative response speed was less than 1.1.
B: relative response speed was 1.1 or more and less than 1.5.
C: relative response speed was 1.5 or more and less than 2.0.
D: relative response speed was 2.0 or more.

<Electric field strength dependence of response speed>

For each photoelectric conversion element (C), electric field strength dependence of the response speed was evaluated by the following method.
The rise time of each photoelectric conversion element (C) at an applied voltage of $7.5 \times 10^4$ V/cm and an applied voltage of $2.0 \times 10^5$ V/cm was measured in the same procedure as in <Evaluation of electric field strength dependence of response speed> in [Test Y].
Using the rise time of each photoelectric conversion element (C) at a wavelength of 460 nm or at a wavelength of 600 nm at each applied voltage, a relative ratio of the rise time at each wavelength was calculated according to Expression (S7). From the obtained values, electric field strength dependence of the response speed was evaluated according to the following evaluation standard.

Expression (S7): Relative ratio of rise time = (Rise time of each photoelectric conversion element (C) at applied voltage of $7.5 \times 10^4$ V/cm)/(Rise time of each photoelectric conversion element (C) at applied voltage of $2.0 \times 10^5$ V/cm) Expression (S7):

In a case of obtaining the relative ratio of the rise time, the rise times at the same wavelength were used as the numerator and the denominator.

A: relative ratio of the rise time was less than 2.0.
B: relative ratio of the rise time was 2.0 or more and less than 3.0.
C: relative ratio of the rise time was 3.0 or more and less than 4.0.
D: relative ratio of the rise time was 4.0 or more and less than 5.0.
E: relative ratio of the rise time was 5.0 or more.

<Electric field strength dependence of quantum efficiency>

Electric field strength dependence of the quantum efficiency of each photoelectric conversion element was evaluated by the following method.
The quantum efficiency (external quantum efficiency) at an electric field strength of $7.0 \times 10^4$ V/cm was measured for each photoelectric conversion element (C) by the same procedure as in the evaluation of <Quantum efficiency> described above.
Using the obtained quantum efficiency of each photoelectric conversion element (C) at a wavelength of 460 nm or at a wavelength of 600 nm at each applied voltage, the electric field strength dependence of the quantum efficiency was calculated according to Expression (SX2), and the electric field strength dependence of the quantum efficiency was evaluated according to the following evaluation standard. In Expression (SX2), the numerator and the denominator are values measured for the same photoelectric conversion element of Examples of Comparative Examples. In addition, the numerator and the denominator were both the quantum efficiency with respect to light having the same wavelength measured at 460 nm or 600 nm. It is preferable that the electric field strength dependence of the quantum efficiency was

evaluated as C or more.

Expression (SX2): Electric field strength dependence of quantum efficiency = (Quantum efficiency of each photoelectric conversion element (C) at applied voltage of 7.0 × $10^4$ V/cm)/(Quantum efficiency of each photoelectric conversion element (C) at applied voltage of 2.0 × $10^5$ V/cm) Expression (SX2):

<Result>

Hereinafter, the configuration of each photoelectric conversion element and the evaluation results of each photoelectric conversion element are shown in the tables.

In the tables, the columns of "$X^3$", "$A^1$, $A^2$", "$R^{N2} \neq R^{N3}$", "$X^1$, $X^2$ (-$CR^{a1}$=)", "Requirements 1 to 4", "Requirements 4 to 7", and "$X^1$, $X^2$ (-$CR^{a2}$=)" are the same as those in Table 1.

[Table 5]

| | Compound | $X^3$ | $A^1$, $A^2$ | $R^{N2} \neq R^{N3}$ | $X^1$, $X^2$ (-$CR^{a1}$=) | Requirements 1 to 4 | Requirements 4 to 7 | $X^1$, $X^2$ (-$CR^{a2}$=) | p-type organic semiconductor | Coloring agent |
|---|---|---|---|---|---|---|---|---|---|---|
| Example 2-1 | 1-1 | A | A | B | A | A | A | A | P-1 | B-1 |
| Example 2-2 | 1-1 | A | A | B | A | A | A | A | P-2 | B-1 |
| Example 2-3 | 1-1 | A | A | B | A | A | A | A | P-1 | B-2 |
| Example 2-4 | 1-1 | A | A | B | A | A | A | A | P-1 | B-3 |
| Example 2-5 | 1-1 | A | A | B | A | A | A | A | P-1 | B-4 |
| Example 2-6 | 1-1 | A | A | B | A | A | A | A | P-1 | B-5 |
| Example 2-7 | 1-1 | A | A | B | A | A | A | A | P-1 | B-6 |
| Example 2-8 | 1-1 | A | A | B | A | A | A | A | P-1 | B-7 |
| Example 2-9 | 1-2 | A | A | B | A | A | A | A | P-1 | B-1 |
| Example 2-10 | 1-3 | A | A | B | A | A | A | A | P-1 | B-1 |
| Example 2-11 | 1-4 | A | A | B | A | A | A | A | P-1 | B-1 |
| Example 2-12 | 1-5 | A | A | A | A | A | A | A | P-1 | B-1 |
| Example 2-13 | 1-6 | A | A | A | A | A | A | A | P-1 | B-1 |
| Example 2-14 | 1-7 | A | A | B | A | A | A | A | P-1 | B-1 |
| Example 2-15 | 1-8 | A | A | B | A | A | A | A | P-1 | B-1 |
| Example 2-16 | 1-9 | A | A | B | A | A | A | A | P-1 | B-1 |
| Example 2-17 | 1-10 | A | A | B | A | A | A | A | P-1 | B-1 |
| Example 2-18 | 1-11 | A | A | B | A | A | A | A | P-1 | B-1 |
| Example 2-19 | 1-12 | A | A | B | A | A | A | A | P-1 | B-1 |
| Example 2-20 | 1-13 | A | A | B | A | A | B | A | P-1 | B-1 |
| Example 2-21 | 1-14 | A | A | B | A | B | B | A | P-1 | B-1 |
| Example 2-22 | 1-15 | A | A | A | A | B | B | A | P-1 | B-1 |
| Example 2-23 | 1-16 | A | A | B | A | A | A | B | P-1 | B-1 |
| Example 2-24 | 1-17 | A | A | A | A | A | A | B | P-1 | B-1 |
| Example 2-25 | 1-18 | A | A | B | A | A | A | B | P-1 | B-1 |
| Example 2-26 | 1-19 | A | B | B | A | A | A | B | P-1 | B-1 |

(continued)

| | Compound | $X^3$ | $A^1, A^2$ | $R^{N2} \neq R^{N3}$ | $X^1, X^2$ ($-CR^{a1}=$) | Requirements 1 to 4 | Requirements 4 to 7 | $X^1, X^2$ ($-CR^{a2}=$) | p-type organic semiconductor | Coloring agent |
|---|---|---|---|---|---|---|---|---|---|---|
| Example 2-27 | 1-20 | A | B | B | A | A | A | A | P-1 | B-1 |
| Example 2-28 | 1-21 | B | A | B | A | A | A | B | P-1 | B-1 |
| Example 2-29 | 1-22 | B | A | B | A | A | A | B | P-1 | B-1 |
| Example 2-30 | 1-23 | B | A | B | A | A | A | A | P-1 | B-1 |
| Example 2-31 | 1-24 | A | A | B | B | A | A | B | P-1 | B-1 |
| Example 2-32 | 1-25 | A | A | B | A | B | B | A | P-1 | B-1 |
| Example 2-33 | 1-26 | A | A | B | A | B | B | A | P-1 | B-1 |
| Example 2-34 | 1-27 | A | A | B | A | B | B | A | P-1 | B-1 |
| Example 2-35 | 1-28 | A | A | A | A | B | B | A | P-1 | B-1 |
| Example 2-36 | 1-29 | A | A | B | A | B | B | A | P-1 | B-1 |
| Example 2-37 | 1-30 | A | A | B | A | B | B | A | P-1 | B-1 |
| Example 2-38 | 1-31 | A | A | A | A | B | B | A | P-1 | B-1 |
| Example 2-39 | 1-32 | A | A | B | A | B | B | B | P-1 | B-1 |
| Example 2-40 | 1-33 | A | A | A | A | B | B | B | P-1 | B-1 |

[Table 6]

| | Quantum efficiency | | Response speed | | Electric field strength dependence of response speed | | Electric field strength dependence of quantum efficiency | |
|---|---|---|---|---|---|---|---|---|
| | 460 nm | 600 nm | 460 nm | 600 nm | 460 nm | 600 nm | 460 nm | 600 nm |
| Example 2-1 | A | A | A | A | A | A | B | B |
| Example 2-2 | A | A | B | B | B | B | B | B |
| Example 2-3 | A | A | A | A | A | A | B | B |
| Example 2-4 | A | A | A | A | A | A | B | B |
| Example 2-5 | A | A | A | A | A | A | B | B |
| Example 2-6 | A | A | A | A | A | A | B | B |
| Example 2-7 | A | A | A | A | A | A | B | B |
| Example 2-8 | A | A | A | A | A | A | B | B |
| Example 2-9 | A | A | A | A | A | A | B | B |
| Example 2-10 | A | A | A | A | A | A | B | B |
| Example 2-11 | A | A | A | A | A | A | B | B |
| Example 2-12 | A | A | A | A | A | A | A | A |
| Example 2-13 | A | A | A | A | A | A | A | A |
| Example 2-14 | A | A | A | A | A | A | B | B |
| Example 2-15 | A | A | A | A | A | A | B | B |
| Example 2-16 | A | A | A | A | A | A | B | B |
| Example 2-17 | A | A | A | A | A | A | B | B |
| Example 2-18 | A | A | A | A | A | A | B | B |
| Example 2-19 | A | A | A | A | A | A | B | B |
| Example 2-20 | A | A | A | A | A | A | C | C |
| Example 2-21 | A | B | A | A | A | A | C | C |
| Example 2-22 | A | B | A | A | A | A | B | B |
| Example 2-23 | A | A | B | B | B | B | B | B |
| Example 2-24 | A | A | B | B | B | B | A | A |
| Example 2-25 | A | A | B | B | B | B | B | B |
| Example 2-26 | A | B | B | B | B | B | B | B |
| Example 2-27 | A | B | A | A | A | A | B | B |
| Example 2-28 | A | B | B | B | B | B | B | B |
| Example 2-29 | A | B | B | B | B | B | B | B |
| Example 2-30 | A | A | B | B | B | B | B | B |
| Example 2-31 | A | B | B | B | B | B | B | B |
| Example 2-32 | A | B | A | A | A | A | C | C |
| Example 2-33 | A | B | A | A | A | A | C | C |
| Example 2-34 | A | B | A | A | A | A | C | C |
| Example 2-35 | A | B | A | A | A | A | B | B |
| Example 2-36 | A | B | A | A | A | A | C | C |

(continued)

| | Quantum efficiency | | Response speed | | Electric field strength dependence of response speed | | Electric field strength dependence of quantum efficiency | |
|---|---|---|---|---|---|---|---|---|
| | 460 nm | 600 nm | 460 nm | 600 nm | 460 nm | 600 nm | 460 nm | 600 nm |
| Example 2-37 | A | B | A | A | A | A | B | B |
| Example 2-38 | A | B | A | A | A | A | A | A |
| Example 2-39 | A | B | B | B | B | B | B | B |
| Example 2-40 | A | B | B | B | B | B | B | B |

[Table 7]

| | Compound | $X^3$ | $A^1$, $A^2$ | $R^{N2} \neq R^{N3}$ | $X^1$, $X^2$ ($-CR^{a1}=$) | Requirements 1 to 4 | Requirements 4 to 7 | $X^1$, $X^2$ ($-CR^{a2}=$) | p-type organic semiconductor | Coloring agent |
|---|---|---|---|---|---|---|---|---|---|---|
| Example 2-41 | 1-34 | A | A | B | A | B | B | B | P-1 | B-1 |
| Example 2-42 | 1-35 | A | A | B | A | B | B | B | P-1 | B-1 |
| Example 2-43 | 1-36 | A | A | A | A | B | B | B | P-1 | B-1 |
| Example 2-44 | 1-37 | A | A | B | A | B | B | B | P-1 | B-1 |
| Example 2-45 | 1-38 | B | A | A | A | B | B | B | P-1 | B-1 |
| Example 2-46 | 1-39 | B | A | A | A | B | B | B | P-1 | B-1 |
| Example 2-47 | 1-40 | B | A | A | A | A | B | B | P-1 | B-1 |
| Example 2-48 | 1-41 | A | A | A | A | A | A | B | P-1 | B-1 |
| Example 2-49 | 1-42 | A | A | A | A | A | A | B | P-1 | B-1 |
| Example 2-50 | 1-43 | A | A | A | A | A | A | B | P-1 | B-1 |
| Example 2-51 | 1-44 | A | A | A | A | B | B | B | P-1 | B-1 |
| Example 2-52 | 1-45 | A | A | A | A | B | B | B | P-1 | B-1 |
| Example 2-53 | 1-46 | A | A | A | A | A | A | B | P-1 | B-1 |
| Example 2-54 | 1-47 | A | A | B | A | A | A | B | P-1 | B-1 |
| Example 2-55 | 1-48 | A | A | B | A | A | A | B | P-1 | B-1 |
| Example 2-56 | 1-49 | A | A | A | A | A | A | B | P-1 | B-1 |
| Example 2-57 | 1-50 | A | A | B | A | A | A | B | P-1 | B-1 |
| Comparative Example 2-1 | C-1 | - | - | | - | - | | - | P-1 | B-1 |
| Comparative Example 2-2 | C-2 | - | - | | - | - | | - | P-1 | B-1 |
| Comparative Example 2-3 | C-3 | - | - | | - | - | | - | P-1 | B-1 |
| Comparative Example 2-4 | C-4 | - | - | | - | - | | - | P-1 | B-1 |

[Table 8]

| | Quantum efficiency | | Response speed | | Electric field strength dependence of response speed | | Electric field strength dependence of quantum efficiency | |
|---|---|---|---|---|---|---|---|---|
| | 460 nm | 600 nm | 460 nm | 600 nm | 460 nm | 600 nm | 460 nm | 600 nm |
| Example 2-41 | A | B | B | B | B | B | C | C |
| Example 2-42 | A | B | B | B | B | B | C | C |
| Example 2-43 | A | B | B | B | B | B | B | B |
| Example 2-44 | A | B | B | B | B | B | C | C |
| Example 2-45 | A | B | B | B | B | B | B | B |
| Example 2-46 | A | B | B | B | B | B | B | B |
| Example 2-47 | A | B | B | B | B | B | A | A |
| Example 2-48 | A | A | B | B | B | B | A | A |
| Example 2-49 | A | A | B | B | B | B | A | A |
| Example 2-50 | A | A | B | B | B | B | A | A |
| Example 2-51 | A | B | B | B | B | B | B | B |
| Example 2-52 | A | B | B | B | B | B | B | B |
| Example 2-53 | A | A | B | B | B | B | B | B |
| Example 2-54 | A | A | B | B | B | B | B | B |
| Example 2-55 | A | A | B | B | B | B | B | B |
| Example 2-56 | A | A | B | B | B | B | A | A |
| Example 2-57 | A | A | B | B | B | B | B | B |
| Comparative Example 2-1 | B | D | C | C | C | C | D | D |
| Comparative Example 2-2 | B | D | D | D | D | D | D | D |
| Comparative Example 2-3 | C | D | D | D | D | D | D | D |
| Comparative Example 2-4 | C | D | D | D | D | D | D | D |

From the results shown in the above tables, it was found that the photoelectric conversion element according to the embodiment of the present invention, containing a coloring agent, was excellent in quantum efficiency in a case of receiving red and green light and receiving blue light. In addition, it was found that the photoelectric conversion element according to the embodiment of the present invention was also excellent in response speed and electric field strength dependence of the response speed in a case of receiving red and green light or receiving blue light.

From the comparison between Example 2-30 and Examples 2-9 to 2-20, it was found that, in a case where $X^3$ in Formula (1) was -C($R^{c1}R^{c2}$)-, the response speed and the electric field strength dependence of the response speed in a case of receiving red and green light or receiving blue light were more excellent.

From the comparison between Example 2-27 and Examples 2-9 to 2-20, and the comparison between Example 2-26 and Examples 2-23 to 2-25, it was found that, in a case where $A^1$ and $A^2$ in Formula (1) were the group represented by Formula (A-2), the quantum efficiency in a case of receiving red and green light was more excellent.

From the comparison between Example 2-31 and Examples 2-23 to 2-25, it was found that, in a case where $X^1$ and $X^3$ in Formula (1) were -$CR^{a1}$=, the quantum efficiency in a case of receiving red and green light was more excellent.

From the comparison between Examples 2-21 and 2-22, Examples 2-32 to 2-38, and Examples 2-9 to 2-20, and the comparison between Examples 2-39 to 2-44 and Examples 2-23 to 2-25, it was found that, in a case where the specific compound satisfied any one of the requirements 1 to 3, the quantum efficiency in a case of receiving red and green light was more excellent.

From the comparison between Examples 2-23 to 2-25 and Examples 2-9 to 2-20, and the comparison between Examples 2-39 to 2-44 and Examples 2-32 to 2-38, it was found that, in a case where at least one of $X^1$ or $X^2$ in Formula (1) was -$CR^{a2}$=, the response speed and the electric field strength dependence of the response speed in a case of receiving

red and green light or receiving blue light were more excellent.

From the comparison between Examples 2-9 to 2-11, Examples 2-14 to 2-20, and Examples 2-12 and 2-13, it was found that, in a case where $R^{N2}$ and $R^{N3}$ were groups different from each other, the electric field strength dependence of the quantum efficiency in a case of receiving red and green light or receiving blue light was smaller.

From the comparison between Examples 2-45 and 2-46 and Example 2-47, it was found that, in a case where $X^3$ represented $-NR^{N11}-$, and $R^{N11}$ was the group represented by Formula (C-1) or the group represented by Formula (C-2), the electric field strength dependence of the quantum efficiency in a case of receiving red and green light or receiving blue light was smaller.

From the comparison between Example 2-20 and Example 2-21, it was found that, in a case where the specific compound satisfied any one of the requirements 4 to 7, the quantum efficiency in a case of receiving red and green light was more excellent.

Explanation of References

10a, 10b: photoelectric conversion element
11: conductive film (lower electrode)
12: photoelectric conversion film
15: transparent conductive film (upper electrode)
16A: electron blocking film
16B: hole blocking film

## Claims

**1.** A photoelectric conversion element comprising, in the following order:

a conductive film;
a photoelectric conversion film; and
a transparent conductive film,
wherein the photoelectric conversion film contains a compound represented by Formula (1),

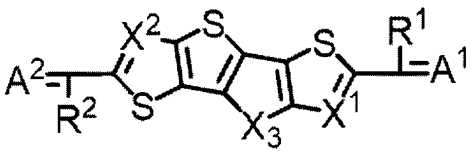

(1)

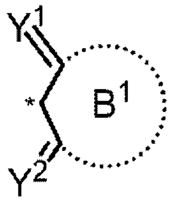

(A-1)

in Formula (1), $R^1$ and $R^2$ each independently represent a hydrogen atom or a substituent,
$X^1$ and $X^2$ each independently $-CR^{a1}=$ or a nitrogen atom, $R^{a1}$ represents a hydrogen atom or a substituent,
$X^3$ represents $-C(R^{c1}R^{c2})-$, $-O-C(R^{c3}R^{c4})-$, $-NR^{N1}-C(R^{c5}R^{c6})-$, or $-NR^{N11}-$, $R^{c1}$, $R^{c2}$, $R^{c3}$, $R^{c4}$, $R^{c5}$, $R^{c6}$, $R^{N1}$, and $R^{N11}$ each independently represent a hydrogen atom or a substituent, $R^{c1}$ and $R^{c2}$, $R^{c3}$ and $R^{c4}$, or $R^{c5}$ and $R^{c6}$ may be bonded to each other to form a ring which may have a substituent, provided that, in a case where the ring which may have a substituent has a plurality of the substituents, the substituents may be bonded to each other to form a ring which may have a substituent, and
$A^1$ and $A^2$ each independently represent Formula (A-1),
in Formula (A-1), $B^1$ represents a monocyclic ring or a polycyclic ring, which contains at least three or more carbon atoms and may have a substituent, provided that, in a case where $B^1$ represents the monocyclic ring and the monocyclic ring has two or more of the substituents, the number of aromatic ring groups among the substituents in the monocyclic ring is 1 or less,
$Y^1$ and $Y^2$ each independently represent an oxygen atom, a sulfur atom, $=NR^{Y1}$, or $=CR^{Y2}R^{Y3}$, $R^{Y1}$ represents a hydrogen atom, an aliphatic hydrocarbon group which may have a substituent, or an aromatic ring group which may have a substituent, $R^{Y2}$ and $R^{Y3}$ each independently represent a cyano group, $-COOR^{Y4}$, $-C(=O)R^{Y5}$, -S(=O)

$R^{Y6}$, or $-SO_2R^{Y7}$, $R^{Y4}$, $R^{Y5}$, $R^{Y6}$, and $R^{Y7}$ each independently represent an aliphatic hydrocarbon group which may have a substituent, an aromatic ring group which may have a substituent, or an aliphatic heterocyclic group which may have a substituent, and

* represents a bonding position,

provided that at least one of $A^1$ or $A^2$ represents the group represented by Formula (A-1), in which $B^1$ is represented by the monocyclic ring.

**2.** The photoelectric conversion element according to claim 1,

wherein $X^3$ represents $-C(R^{c1}R^{c2})-$,

where $R^{c1}$ and $R^{c2}$ may be bonded to each other to form a ring which may have a substituent, and in a case where the ring which may have a substituent has a plurality of the substituents, the substituents may be bonded to each other to form a ring which may have a substituent.

**3.** The photoelectric conversion element according to claim 1,

wherein the group represented by Formula (A-1) is a group represented by Formula (A-2),

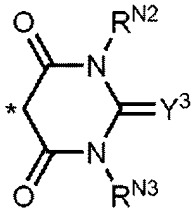

(A-2)

in Formula (A-2), $R^{N2}$ and $R^{N3}$ each independently represent a hydrogen atom or a substituent, provided that one of $R^{N2}$ or $R^{N3}$ represents a hydrogen atom or a substituent other than an aromatic ring group,

$Y^3$ represents an oxygen atom or a sulfur atom, and

* represents a bonding position.

**4.** The photoelectric conversion element according to claim 3, wherein, in Formula (A-2), $R^{N2}$ and $R^{N3}$ are groups different from each other.

**5.** The photoelectric conversion element according to claim 1, wherein $X^1$ and $X^2$ each independently represent $-CR^{a1}=$.

**6.** The photoelectric conversion element according to claim 1,

wherein any one of the following requirements 1 to 4 is satisfied,

the requirement 1: $X^3$ represents $-C(R^{c1}R^{c2})-$, and $R^{c1}$ and $R^{c2}$ are groups different from each other, or $R^{c1}$ and $R^{c2}$ are bonded to each other to form a monocyclic ring having a substituent, and in a case where the monocyclic ring having a substituent has a plurality of the substituents, the substituents may be bonded to each other to form a ring which may have a substituent,

the requirement 2: $X^3$ represents $-O-C(R^{c3}R^{c4})-$, and $R^{c3}$ and $R^{c4}$ are groups different from each other, or $R^{c3}$ and $R^{c4}$ are bonded to each other to form a monocyclic ring having a substituent, and in a case where the monocyclic ring having a substituent has a plurality of the substituents, the substituents may be bonded to each other to form a ring which may have a substituent,

the requirement 3: $X^3$ represents $-NR^{N1}-C(R^{c5}R^{c6})-$, and $R^{c5}$ and $R^{c6}$ are groups different from each other, or $R^{c5}$ and $R^{c6}$ are bonded to each other to form a monocyclic ring having a substituent, and in a case where the monocyclic ring having a substituent has a plurality of the substituents, the substituents may be bonded to each other to form a ring which may have a substituent,

the requirement 4: $X^3$ represents $-NR^{N11}-$, and $R^{N11}$ is a group represented by Formula (C-1) or a group represented by Formula (C-2),

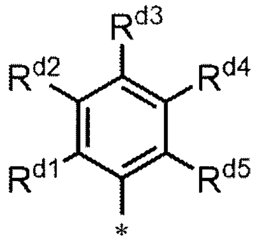

(C-1)

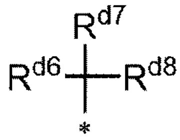

(C-2)

in Formula (C-1), $R^{d1}$ to $R^{d5}$ each independently represent a hydrogen atom or a substituent, and at least one of the following requirement C1 or the following requirement C2 is satisfied,
the requirement C1: $R^{d1}$ and $R^{d5}$ are groups different from each other,
the requirement C2: $R^{d2}$ and $R^{d4}$ are groups different from each other,
in Formula (C-2), $R^{d6}$ to $R^{d8}$ each independently represent a hydrogen atom or a substituent, and $R^{d6}$ to $R^{d8}$ are groups different from each other.

**7.** The photoelectric conversion element according to claim 6,

wherein any one of the following requirements 5 to 7 or the requirement 4 is satisfied,
the requirement 5: $X^3$ represents $-C(R^{c1}R^{c2})-$, and $R^{c1}$ and $R^{c2}$ are groups different from each other,
the requirement 6: $X^3$ represents $-O-C(R^{c3}R^{c4})-$, and $R^{c3}$ and $R^{c4}$ are groups different from each other,
the requirement 7: $X^3$ represents $-NR^{N1}-C(R^{c5}R^{c6})-$, and $R^{c5}$ and $R^{c6}$ are groups different from each other.

**8.** The photoelectric conversion element according to any one of claims 1 to 7,

wherein at least one of $X^1$ or $X^2$ represents $-CR^{a2}=$,
$R^{a2}$'s each independently represent an aliphatic hydrocarbon group which may have a substituent, an aromatic ring group which may have a substituent, a halogen atom, an alkoxy group which may have a substituent, an acyl group which may have a substituent, an aliphatic heterocyclic group which may have a substituent, a cyano group, a nitro group, an amino group, or $-Si(R^{Sil}R^{Si2}R^{Si3})$, and $R^{Si1}$, $R^{Si2}$, and $R^{Si3}$ each independently represent an aliphatic hydrocarbon group which may have a substituent or an aromatic ring group which may have a substituent.

**9.** The photoelectric conversion element according to any one of claims 1 to 7,

wherein the photoelectric conversion film further contains an n-type organic semiconductor, and
the photoelectric conversion film has a bulk hetero structure formed in a state in which the compound represented by Formula (1) and the n-type organic semiconductor are mixed with each other.

**10.** The photoelectric conversion element according to claim 9,
wherein the n-type organic semiconductor includes fullerenes selected from the group consisting of a fullerene and derivatives of the fullerene.

**11.** The photoelectric conversion element according to any one of claims 1 to 7,
wherein the photoelectric conversion film further contains a p-type organic semiconductor.

**12.** The photoelectric conversion element according to any one of claims 1 to 7,
wherein the photoelectric conversion film further contains a coloring agent.

**13.** The photoelectric conversion element according to any one of claims 1 to 7, further comprising:
one or more interlayers between the conductive film and the transparent conductive film, in addition to the photoelectric conversion film.

**14.** An imaging element comprising:
the photoelectric conversion element according to any one of claims 1 to 7.

**15.** An optical sensor comprising:
the photoelectric conversion element according to any one of claims 1 to 7.

**16.** A method for manufacturing an imaging element, comprising:
a step of manufacturing the photoelectric conversion element according to any one of claims 1 to 7.

**17.** A compound represented by Formula (1),

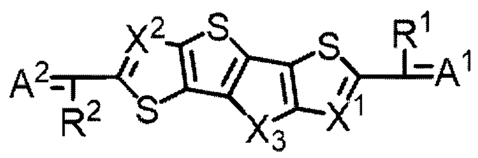

(1)

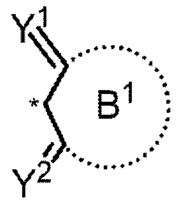

(A-1)

in Formula (1), $R^1$ and $R^2$ each independently represent a hydrogen atom or a substituent,
$X^1$ and $X^2$ each independently $-CR^{a1}=$ or a nitrogen atom, $R^{a1}$ represents a hydrogen atom or a substituent,
$X^3$ represents $-C(R^{c1}R^{c2})-$, $-O-C(R^{c3}R^{c4})-$, $-NR^{N1}-C(R^{c5}R^{c6})-$, or $-NR^{N11}-$, $R^{c1}$, $R^{c2}$, $R^{c3}$, $R^{c4}$, $R^{c5}$, $R^{c6}$, $R^{N1}$, and $R^{N11}$ each independently represent a hydrogen atom or a substituent, $R^{c1}$ and $R^{c2}$, $R^{c3}$ and $R^{c4}$, or $R^{c5}$ and $R^{c6}$ may be bonded to each other to form a ring which may have a substituent, provided that, in a case where the ring which may have a substituent has a plurality of the substituents, the substituents may be bonded to each other to form a ring which may have a substituent, and
$A^1$ and $A^2$ each independently represent Formula (A-1),
in Formula (A-1), $B^1$ represents a monocyclic ring or a polycyclic ring, which contains at least three or more carbon atoms and may have a substituent, provided that, in a case where $B^1$ represents the monocyclic ring and the monocyclic ring has two or more of the substituents, the number of aromatic ring groups among the substituents in the monocyclic ring is 1 or less,
$Y^1$ and $Y^2$ each independently represent an oxygen atom, a sulfur atom, $=NR^{Y1}$, or $=CR^{Y2}R^{Y3}$, $R^{Y1}$ represents a hydrogen atom, an aliphatic hydrocarbon group which may have a substituent, or an aromatic ring group which may have a substituent, $R^{Y2}$ and $R^{Y3}$ each independently represent a cyano group, $-COOR^{Y4}$, $-C(=O)R^{Y5}$, $-S(=O)R^{Y6}$, or $-SO_2R^{Y7}$, $R^{Y4}$, $R^{Y5}$, $R^{Y6}$, and $R^{Y7}$ each independently represent an aliphatic hydrocarbon group which may have a substituent, an aromatic ring group which may have a substituent, or an aliphatic heterocyclic group which may have a substituent, and
* represents a bonding position.
provided that at least one of $A^1$ or $A^2$ represents the group represented by Formula (A-1), in which $B^1$ is represented by the monocyclic ring.
* represents a bonding position.

**18.** The compound according to claim 17,

wherein $X^3$ represents $-C(R^{c1}R^{c2})-$,
where $R^{c1}$ and $R^{c2}$ may be bonded to each other to form a ring which may have a substituent, and in a case where the ring which may have a substituent has a plurality of the substituents, the substituents may be bonded to each other to form a ring which may have a substituent.

**19.** The compound according to claim 17,

wherein the group represented by Formula (A-1) is a group represented by Formula (A-2),

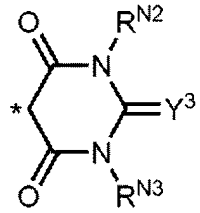

(A-2)

in Formula (A-2), $R^{N2}$ and $R^{N3}$ each independently represent a hydrogen atom or a substituent, provided that one of $R^{N2}$ or $R^{N3}$ represents a hydrogen atom or a substituent other than an aromatic ring group,
$Y^3$ represents an oxygen atom or a sulfur atom, and
* represents a bonding position.

**20.** The compound according to claim 19,
wherein, in Formula (A-2), $R^{N2}$ and $R^{N3}$ are groups different from each other.

**21.** The compound according to claim 17,
wherein $X^1$ and $X^2$ each independently represent $-CR^{a1}=$.

**22.** The compound according to claim 17,

wherein any one of the following requirements 1 to 4 is satisfied,
the requirement 1: $X^3$ represents $-C(R^{c1}R^{c2})-$, and $R^{c1}$ and $R^{c2}$ are groups different from each other, or $R^{c1}$ and $R^{c2}$ are bonded to each other to form a monocyclic ring having a substituent, and in a case where the monocyclic ring having a substituent has a plurality of the substituents, the substituents may be bonded to each other to form a ring which may have a substituent,
the requirement 2: $X^3$ represents $-O-C(R^{c3}R^{c4})-$, and $R^{c3}$ and $R^{c4}$ are groups different from each other, or $R^{c3}$ and $R^{c4}$ are bonded to each other to form a monocyclic ring having a substituent, and in a case where the monocyclic ring having a substituent has a plurality of the substituents, the substituents may be bonded to each other to form a ring which may have a substituent,
the requirement 3: $X^3$ represents $-NR^{N1}-C(R^{c5}R^{c6})-$, and $R^{c5}$ and $R^{c6}$ are groups different from each other, or $R^{c5}$ and $R^{c6}$ are bonded to each other to form a monocyclic ring having a substituent, and in a case where the monocyclic ring having a substituent has a plurality of the substituents, the substituents may be bonded to each other to form a ring which may have a substituent,
the requirement 4: $X^3$ represents $-NR^{N11}-$, and $R^{N11}$ is a group represented by Formula (C-1) or a group represented by Formula (C-2),

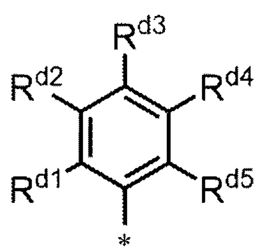

(C-1)

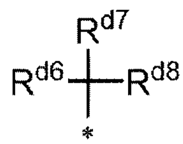

(C-2)

in Formula (C-1), $R^{d1}$ to $R^{d5}$ each independently represent a hydrogen atom or a substituent, and at least one of the following requirement C1 or the following requirement C2 is satisfied,
the requirement C1: $R^{d1}$ and $R^{d5}$ are groups different from each other,
the requirement C2: $R^{d2}$ and $R^{d4}$ are groups different from each other,
in Formula (C-2), $R^{d6}$ to $R^{d8}$ each independently represent a hydrogen atom or a substituent, and $R^{d6}$ to $R^{d8}$ are groups different from each other.

**23.** The compound according to claim 22,

wherein any one of the following requirements 5 to 7 or the requirement 4 is satisfied,
the requirement 5: $X^3$ represents -C($R^{c1}R^{c2}$)-, and $R^{c1}$ and $R^{c2}$ are groups different from each other,
the requirement 6: $X^3$ represents -O-C($R^{c3}R^{c4}$)-, and $R^{c3}$ and $R^{c4}$ are groups different from each other,
the requirement 7: $X^3$ represents -$NR^{N1}$-C($R^{c5}R^{c6}$)-, and $R^{c5}$ and $R^{c6}$ are groups different from each other.

**24.** The compound according to any one of claims 17 to 23,

wherein at least one of $X^1$ or $X^2$ represents -$CR^{a2}$=,
$R^{a2}$'s each independently represent an aliphatic hydrocarbon group which may have a substituent, an aromatic ring group which may have a substituent, a halogen atom, an alkoxy group which may have a substituent, an acyl group which may have a substituent, an aliphatic heterocyclic group which may have a substituent, a cyano group, a nitro group, an amino group, or -Si($R^{Si1}R^{Si2}R^{Si3}$), and $R^{Si1}$, $R^{Si2}$, and $R^{Si3}$ each independently represent an aliphatic hydrocarbon group which may have a substituent or an aromatic ring group which may have a substituent.

FIG. 1

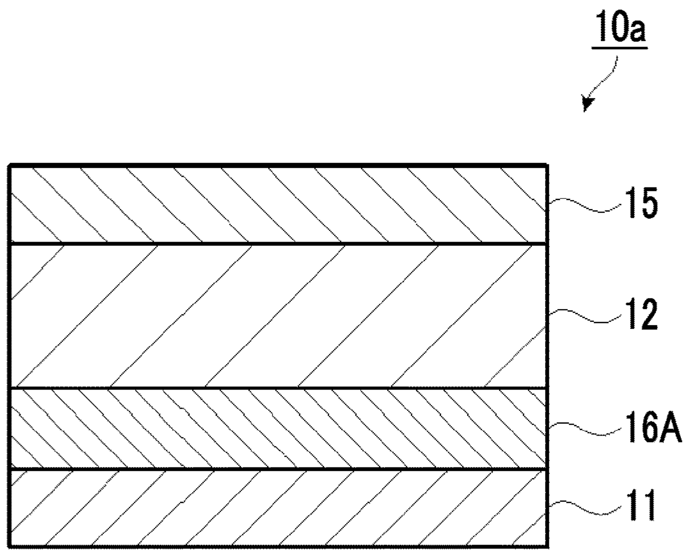


FIG. 2

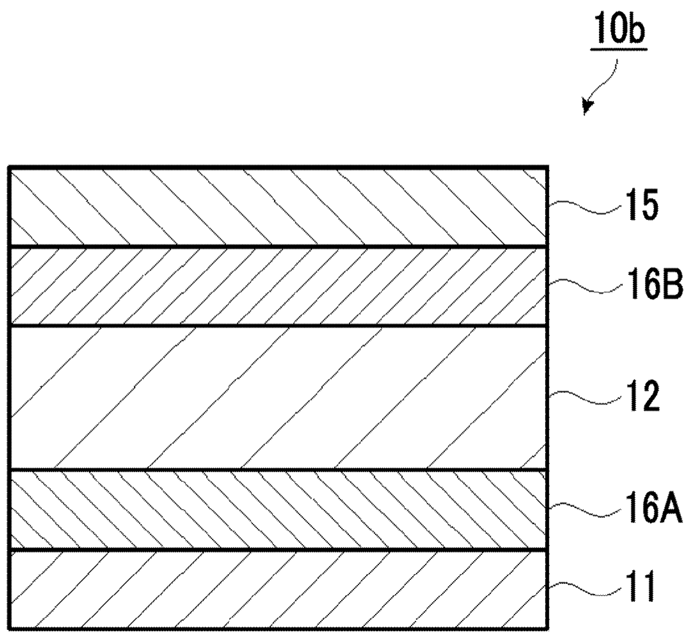

## INTERNATIONAL SEARCH REPORT

International application No.
**PCT/JP2024/008059**

**A. CLASSIFICATION OF SUBJECT MATTER**

***H10K 30/60***(2023.01)i; ***C07D 495/14***(2006.01)i; ***H10K 30/30***(2023.01)i; ***H10K 39/32***(2023.01)i; ***H10K 85/40***(2023.01)i; ***H10K 85/60***(2023.01)i
FI: H10K30/60; C07D495/14; H10K30/30; H10K39/32; H10K85/40; H10K85/60

According to International Patent Classification (IPC) or to both national classification and IPC

**B. FIELDS SEARCHED**

Minimum documentation searched (classification system followed by classification symbols)

H10K10/00-99/00; C07D201/00-521/00

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

CAplus/REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| A | WO 2023/012366 A1 (CAMBRIDGE DISPLAY TECHNOLOGY LTD.) 09 February 2023 (2023-02-09) | 1-24 |
| A | Indandione-Terminated Quinoidal Compounds for Low-Bandgap Small Molecules with Strong Near-Infrared Absorption: Effect of Conjugation Length on the Properties. Sun, Lei et al.. Chemistry-A European Journal, 19 October 2021, vol. 27, pp. 17437-17443 | 1-24 |
| P, X | WO 2023/218933 A1 (FUJIFILM CORPORATION) 16 November 2023 (2023-11-16) paragraphs [0136], [0189]-[0209] | 1-3, 5, 9-19, 21 |
| P, A | | 4, 6-8, 20, 22-24 |

☐ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **22 March 2024** | **02 April 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (July 2022)

**INTERNATIONAL SEARCH REPORT**
**Information on patent family members**

International application No.
**PCT/JP2024/008059**

| Patent document cited in search report | Publication date (day/month/year) | Patent family member(s) | Publication date (day/month/year) |
|---|---|---|---|
| WO 2023/012366 A1 | 09 February 2023 | TW 202321264 A | |
| WO 2023/218933 A1 | 16 November 2023 | (Family: none) | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20220135587 A
- JP 2006100767 A
- JP 2007123707 A
- JP 2005303266 A
- JP 2011228614 A
- JP 2011176259 A
- JP 2011225544 A
- JP 2015153910 A
- JP 2012094660 A
- JP 2018014474 A
- WO 2016194630 A
- WO 2017159684 A
- JP 2017076766 A
- WO 2018207722 A
- JP 2019054228 A
- WO 2019058995 A
- WO 2019081416 A
- JP 2019080052 A
- WO 2019054125 A
- WO 2019093188 A
- JP 2019050398 A
- JP 2018206878 A
- JP 2018190755 A
- JP 2018026559 A
- JP 2018170487 A
- JP 2018078270 A
- JP 2018166200 A
- JP 2018113425 A
- JP 2018085430 A
- JP 2018056546 A
- JP 2018046267 A
- WO 2018016465 A
- JP 2020010024 A
- JP 2022123944 A
- JP 2022122839 A
- JP 2022120323 A
- JP 2022120273 A
- JP 2022115832 A
- JP 2022108268 A
- JP 2023005703 A
- JP 2022100258 A
- JP 2022181226 A
- JP 2022027575 A
- JP 2021163968 A
- WO 2020013246 A
- WO 2022168856 A
- JP 2023010305 A
- JP 2023010299 A
- JP 2011082508 A